# EUROPÄISCHE PATENTANMELDUNG

(11) **EP 2 106 833 A1**
(43) Veröffentlichungstag der Anmeldung: **07.10.2009**
(21) Anmeldenummer: 08153931.4
(22) Anmeldetag: 02.04.2008
(51) Int. Cl.: B01D 1/18, A61K 8/87, C08J 3/12, C08L 75/02, C08G 18/08

(54) **Partikel, erhältlich durch Trocknung einer wässrigen Nanoharnstoff-Dispersion**

(71) Anmelder: Bayer MaterialScience AG, 51368 Leverkusen (DE)
(72) Erfinder: Die Erfindernennung liegt noch nicht vor

(57) **Zusammenfassung**

Die Erfindung betrifft Partikel hergestellt durch Trocknung wässriger Dispersionen von Nanoharnstoffen, ein Verfahren zu deren Herstellung und deren Verwendung.

## Beschreibung

Die vorliegende Erfindung betrifft Partikel, welche durch Trocknung wässriger Dispersionen von Nanoharnstoffen, herstellbar sind sowie ein entsprechendes Verfahren zu deren Herstellung. Weiterer Gegenstand der vorliegenden Erfindung ist deren Verwendung. Ferner betrifft die vorliegende Erfindung die Verwendung von wässrigen Dispersionen von quervernetzten Polyharnstoffen zur Herstellung von Kosmetik.

Die Sprühtrocknung von Lösungen aus Polyharnstoffe in organischen Lösemitteln wird bereits in EP 1 630 191 A beschrieben. In dieser Veröffentlichung wird jedoch weder die Trocknung von wässrigen Dispersionen offenbart, noch wird Wasser als Lösemittel eingesetzt. Ferner ist diese Veröffentlichung auf zu trocknenden Polyharnstoffe fokussiert, die linear ausgebaut sind. Querverzweigten Nanopartikel werden nicht angesprochen. Die in der EP 1 630 191 A beschriebenen Polyharnstoffe werden durch Polyaddition von Aminen an Isocyanaten hergestellt, wobei eine Aufbaureaktion durch Kettenverlängerung mittels Hydrolyse nicht beschrieben ist.

Die Herstellung von wässrigen Dispersionen, enthaltend quervernetze, nanoskalige Polyharnstoffpartikel, wird in der WO 2005/063873 A beschrieben. Dabei werden hydrophile Isocyanate in Gegenwart eines Katalysators in Wasser gegeben, wodurch eine Quervernetzung innerhalb der dispergierten Partikel durch Harnstoffbindungen ausgebildet wird. Auch DE die 10 2006 008 69 A beschreibt die Hersteilung wässriger Dispersionen, enthaltend quervernetzte Harnstoffpartikel. Die Teilchengrößen der Partikel liegen in Bereichen von 10 bis 300 nm (gemessen über Laser-Korrelationsspektroskopie). Diese Partikel werden als Additive für Kontaktklebstoffe auf Basis von Polychloropren-Dispersionen eingesetzt.

Da viele Anwendungen wie beispielsweise Kosmetika, Beschichtungsmittel, Dichtstoffe oder Klebstoffe, pulverförmige Füllstoffe mit Partikeldurchmessern im Bereich von im Allgemeinen 1 bis 50 µm erfordern, besteht ein Bedarf für die Bereitstellung entsprechender Pulver.

Eine Aufgabe der vorliegenden Erfindung ist somit die Bereitstellung von Polyharnstoffenthaltenden Pulver in Partikelform, wobei die Partikel vorzugsweise einen mittleren Durchmessern von 1 bis 50 µm aufweisen sollen.

Erfindungsgemäß wurde nun gefunden, dass durch Trocknung wässrige Dispersionen quervernetzter Nanoharnstoff-Partikei Pulver erhalten werden können.

Gegenstand der vorliegenden Erfindung ist somit ein Nanoharnstoffpulver, welches ausgehend von einer wässrigen Dispersion quervernetzter Nanoharnstoffe durch Trocknung erhalten wird. Dabei war es nicht vorherzusehen, dass durch die Trocknung einer entsprechenden Nanoharnstoffdispersion ein Pulverprodukt erhalten wird, sondern vielmehr eine Masse mit klebriger Konsistenz.

Weiterer Gegenstand der vorliegenden Erfindung ist ein Verfahren zur Herstellung von Nanoharnstoffpulvern durch Trocknung wässriger Dispersionen von Polyharnstoffen.

Ebenfalls Gegenstand der vorliegenden Erfindung ist die Verwendung der Partikel, die durch Trocknung wässriger Dispersionen von Nanoharnstoffen hergestellt werden, in Kosmetika, Beschichtungsmittel, Dichtstoffe oder Klebstoffe.

Ebenfalls Gegenstand der vorliegenden Erfindung ist die Verwendung der Partikel, die durch Trocknung wässriger Dispersionen von Nanoharnstoffen hergestellt wurden, als Füllstoff, Additiv, Hilfs- und/oder Zusatzstoff.

Ebenfalls Gegenstand der vorliegenden Erfindung sind Kosmetika, Gegenstände, Beschichtungsmittel, Dichtstoffe und Klebstoffe, welcher unter Verwendung der erfindungsgemäßen Partikel erhalten werden.

Im Rahmen der vorliegenden Erfindung werden unter Nanoharnstoffen querverzweigte Polyharnstoffpartikel mit einem Durchmesser im nanosklaigen Bereich verstanden.

Im Rahmen der vorliegenden Erfindung ist in einer Ausführungsform ein Nanoharnstoffpulver ausgeschlossen, welches durch Gefriertrocknung einer wässrigen Nanoharnstoff-Dispersion erhalten wird.

Im Rahmen der vorliegenden Erfindung ist in einer weiteren Ausführungsform ein Nanoharnstoff-pulver ausgeschlossen, welches durch Gefriertrocknung einer wässrigen Nanoharnstoff-Dispersion erhalten wird, die so erhalten wird, dass zu einer Lösung von 20,72 g Triethylamin in 4952 g entionisiertem Wasser bei 30 °C unter heftigem Rühren 820,20 g Bayhydur^{®} VP LS 2336 und anschließend 0,32 g Isofoam^{®} 16 gegeben und weiter gerührt wird, nach 3, 6 und 9 Stunden jeweils weitere 820,20 g Bayhydur^{®} VP LS 2336 und je anschließend 0,32 g Isofoam^{®} 16 zugesetzt werden und anschließend bei 30°C weitere 4 Stunden nachgerührt wird und bei 200 mbar Vakuum und 30 °C weiter 3 Stunden gerührt wird und die entstandene Dispersion abgefüllt wird.

Bei den erfindungsgemäßen Polyharnstoffpulvern handelt es sich vorzugsweise um Pulver, welche ausgehend von wässrigen Dispersionen von quervernetzten Nanoharnstoffpartikeln erhalten werden.

Die mittleren Teilchendurchmesser der erfindungsgemäßen Partikel (bestimmt durch Vermessung mittels optischer Spektroskopie durch Abgleich der gefundenen Partikeldurchmesser mit einer kalibrierten Längenskala) weisen Größen von im Allgemeinen 0,5 bis 1000 µm, bevorzugt von 1 bis 200 µm, besonders bevorzugt von 1 bis 50 µm, auf.

Der Restwassergehalt der erfindungsgemäßen Partikel, welche durch Trocknung der Nanoharnstoff-Dispersion erhalten werden und welcher bestimmt wird durch gravimetrische Analyse bei weiterer Trocknung einer Probe von ca. 1 g des Pulvers im Umluftofen bei 120 °C bis zur Gewichtskonstanz, liegt im Allgemeinen unter 10 Gew.-%, bevorzugt von 0,001 bis 5 Gew.-%, besonders bevorzugt von 0,1 bis 3 Gew.-%.

Der Gehalt an erfindungsgemäßen Nanoharnstoff-Partikeln in Dispersionen in erfindungsgemäßen Gegenständen, insbesondere Kosmetika, Beschichtungsmitteln, Dichtstoffen oder Klebstoffen, beträgt im Allgemeinen von 0,01 bis 50 Gew.-%, bevorzugt von 0,1 bis 20 Gew.-%.

Die in der erfindungsgemäßen Partikel werden durch Trocknung wässriger Nanoharnstoff-Dispersion erhalten. Dabei sind die Nanoharnstoff-Partikel in der wässrigen Dispersion intrapartikulär im Wesentlichen durch Harnstoffbindungen quervernetzt. Unter dem Begriff "im Wesentlichen durch Harnstoffbindungen quervernetzt" wird erfindungsgemäß verstanden, wenn vorzugsweise mindestens 50 mol-% der Verzweigungsstellen, bevorzugt mindestens 90 mol-%, jeweils ausgehend von dem Isocyanathaltigen hydrophilen Ausgangsbaustein, quervernetzt sind.

Entsprechende Nanoharnstoff-Dispersionen sind beispielsweise gemäß der WO 2005/063873 A1 erhältlich, dessen diesbezügliche Offenbarung in die vorliegende Erfindung durch Bezugnahme eingeschlossen wird.

Die unvernetzten oder vorvernetzen Partikel bilden sich durch Dispergierung von hydrophilierten Polyisocyanaten i) in Wasser. Anschließend wird ein Teil der vorhandenen Isocyanatgruppen durch eine Isocyanat-Wasser-Reaktion zum primären oder sekundären Amin abgebaut. Diese Aminogruppen bilden dann durch Abreaktion mit weiteren Isocyanatgruppen Harnstoffgruppen und vernetzten dadurch zu Nanoharnstoffpartikel, die in wässriger Nanoharnstoff-Dispersion vorliegen. Ein Teil der Isocyanatgruppen kann dabei auch vor oder während der Reaktion mit Wasser oder mit anderen Isocyanat-reaktiven Spezies, wie z.B. primären oder sekundären Aminen und/oder Alkoholen, umgesetzt werden.

Als hydrophilierte Polyisocyanate i) können an sich alle dem Fachmann bekannten NCOgruppenhaltigen Verbindungen eingesetzt werden, die nichtionisch oder potentiell ionisch hydrophiliert sind. Werden Mischungen verschiedener Polyisocyanate i) eingesetzt, ist es bevorzugt, dass mindestens ein Polyisocyanat eine nichtionisch hydrophilierende Struktureinheit aufweist. Besonders bevorzugt werden ausschließlich Polyisocyanate i) mit nichtionisch hydrophilierende Gruppen eingesetzt.

Unter ionisch bzw. potentiell ionisch hydrophilierende Verbindungen werden sämtliche Verbindungen verstanden, die mindestens eine isocyanatreaktive Gruppe sowie mindestens eine Funktionalität, wie z.B. -COOY, -SO₃Y, -PO(OY)₂ (Y beispielsweise = H, NH₄⁺, Metallkation), -NR₂, -NR₃⁺ (R = H, Alkyl, Aryl), aufweisen, die bei Wechselwirkung mit wässrigen Medien ein pH-Wertabhängiges Dissoziationsgleichgewicht eingeht und auf diese Weise negativ, positiv oder neutral geladen sein kann. Bevorzugte isocyanatreaktive Gruppen sind Hydroxyl- oder Aminogruppen.

Geeignete ionisch oder potentiell ionisch hydrophilierende Verbindungen sind z.B. Mono- und Dihydroxycarbonsäuren, Mono- und Diaminocarbonsäuren, Mono- und Dihydroxysulfonsäuren, Mono-und Diaminosulfonsäuren sowie Mono- und Dihydroxyphosphonsäuren oder Mono- und Diaminophosphonsäuren und ihre Salze wie Dimethylolpropionsäure, Dimethylolbuttersäure, Hydroxypivalinsäure, N-(2-Aminoethyl)-β-alanin, 2-(2-Amino-ethylamino)-ethansulfonsäure, Ethylendiaminpropyl- oder -butylsulfonsäure, 1,2- oder 1,3-Propylendiamin-β-ethylsulfonsäure, Äpfelsäure, Zitronensäure, Glykolsäure, Milchsäure, Glycin, Alanin, Taurin, Lysin, 3,5-Diaminobenzoesäure, ein Additionsprodukt von IPDI und Acrylsäure (EP-A 0 916 647, Beispiel 1) und dessen Alkali- und/oder Ammoniumsalze; das Addukt von Natriumbisulfit an Buten-2-diol-1,4, Polyethersulfonat, das propoxylierte Addukt aus 2-Butendiol und NaHSO₃, z.B. beschrieben in der DE-A 2 446 440 (Seite 5-9, Formel I-III) sowie Verbindungen, die in kationische Gruppen überführbare, z.B. Amin-basierende, Bausteine wie N-Methyl-diethanolamin als hydrophile Aufbaukomponenten enthalten. Weiterhin kann Cyclohexylaminopropansulfonsäure (CAPS) wie z.B. in der WO 01/88006 A als Verbindung verwendet werden.

Bevorzugte ionische oder potentielle ionische Verbindungen sind solche, die über Carboxy- oder Carboxylat- und/oder Sulfonatgruppen und/oder Ammoniumgruppen verfügen. Besonders bevorzugte ionische Verbindungen sind solche, die Carboxyl- und/oder Sulfonatgruppen als ionische oder potentiell ionische Gruppen enthalten, wie die Salze von N-(2-Aminoethyl)-β-alanin, der 2-(2-Aminoethylamino-)ethansulfonsäure oder des Additionsproduktes von IPDI und Acrylsäure (EP 0 916 647 A, Beispiel 1) sowie der Dimethylolpropionsäure.

Geeignete nichtionisch hydrophilierende Verbindungen sind z.B. Polyoxyalkylenether, die mindestens eine Hydroxy- oder Aminogruppe enthalten. Diese Polyether enthalten einen Anteil von 30 Gew.-% bis 100 Gew.-% an Bausteinen, die vom Ethylenoxid abgeleitet sind.

Hydrophile Aufbaukomponenten zum Einbau endständiger hydrophiler Ethylenoxideinheiten aufweisender Ketten sind bevorzugt Verbindungen der Formel (I),

H-Y'-X-Y-R (I)

in welcher
- R: für einen einwertigen Kohlenwasserstoffrest mit 1 bis 12 Kohlenstoffatomen, bevorzugt einen unsubstituierten Alkylrest mit 1 bis 4 Kohlenstoffatomen, steht;
- X: für eine Polyalkylenoxidkette mit 5 bis 90, bevorzugt 20 bis 70 Kettengliedern, welche zumindest zu 40 %, bevorzugt zumindest zu 65 %, aus Ethylenoxid-Einheiten bestehen und die neben Ethylenoxid-Einheiten aus Propoylenoxid-, Butylenoxid- oder Styroloxid-Einheiten bestehen können, wobei unter den letztgenannten Einheiten Propylenoxid-Einheiten bevorzugt sind, steht und
- Y'/Y: für Sauerstoff oder für -NR'- steht, wobei R' bezüglich seiner Definition R oder Wasserstoff entspricht.

Besonders bevorzugt sind die Mischpolymerisate des Ethylenoxids mit Propylenoxid mit einem Ethylenoxidmassenanteil größer 50 %, besonders bevorzugt von 55 bis 89 %. In einer bevorzugten Ausführungsform werden Verbindungen mit einem Molekulargewicht von mindestens 400 g/mol, bevorzugt von mindestens 500 g/mol und besonders bevorzugt von 1200 bis 4500 g/mol eingesetzt.

Besonders bevorzugt sind nichtionisch hydrophilierte Polyisocyanate i), die im statistischen Mittel 5 bis 70, bevorzugt 7 bis 55 Oxyethylengruppen, bevorzugt Ethylengruppen pro Molekül aufweisen.

Die hydrophilierten Polyisocyanate i) basieren auf die dem Fachmann an sich bekannten aliphatischen, cycloaliphatischen, araliphatischen und aromatischen Polyisocyanaten mit mehr als einer NCO-Gruppe pro Molekül und einem Isocyanatgehalt von 0,5 bis 50 Gew.-%, bevorzugt 3. bis 30 Gew.-%, besonders bevorzugt 5 bis 25 Gew.-%, oder deren Gemische.

Beispiele geeigneter Polyisocyanate sind Butylendiisocyanat, Tetramethylendiisocyanat, Cyclohexan-1,3- und 1,4-diisocyanat, Hexamethylendiisocyanat (HDI), 1-Isocanato-3,3,5-trimethyl-5-isocyanato-methylcyclohexan (Isophorondiisocyanat, IPDI), 2,4,4-Trimethylhexamethylendiisocyanat, Isocyanatomethyl-1,8-octandiisocyanat, Methylen-bis-(4-isocyanatocyclohexan), Tetramethylxylylendiisocyanat (TMXDI) oder Triisocyanatononan (TiN, 4-Isocyanatomethyl-1,8-octandiisocyanat) sowie deren Mischungen. Prinzipiell geeignet sind auch aromatische Polyisocyanate wie 1,4-Phenylendiisocyanat, 2,4- und/oder 2;6-Toluylendiisocyanat (TDI), Diphenylmethan-2,4'-und/oder 4,4'-diisocyanat (MDI), Triphenylmethan-4,4'-diisocyanat oder Naphthylen-1,5-diisocyanat.

Neben den vorstehend genannten Polyisocyanaten können auch höhermolekulare Folgeprodukte mit Uretdion-, Isocyanurat-, Urethan-, Allophanat-, Biuret-, Iminooxadiazindion- und/oder Oxadiazintrionstruktur eingesetzt werden. Solche Folgeprodukte sind in an sich bekannter Weise aus den monomeren Diisocyanaten durch die im Stand der Technik beschriebene Modifizierungsreaktionen bekannt.
Bevorzugt liegen den hydrophilierten Polyisocyanaten i) Polyisocyanate oder Polyisocyanatgemische der vorstehend genannten Art mit ausschließlich aliphatisch oder cycloaliphatisch gebundenen Isocyanatgruppen oder deren beliebige Mischungen zugrunde.

Besonders bevorzugt basieren die hydrophilierten Polyisocyanate auf Hexamethylendiisocyanat, Isophorondiisocyanat oder die isomeren Bis-(4,4'-isocyanatocyclohexyl)methane sowie Mischungen der vorgenannten Diisocyanate. Bevorzugt enthalten die Polyisocyanate i) mindestens 50 Gew.-% Polyisocyanate auf Basis von Hexamethylendiisocyanat.

Die Dispergierung der Polyisocyanate a) in Wasser und Reaktion mit Wasser zur Herstellung der wässrigen Dispersion erfolgt bevorzugt unter Durchmischung durch ein Rührwerk oder andere Arten der Durchmischung wie Umpumpen, Statikmischer, Stachelmischer, Düsenstrahldispergator, Rotor und Stator oder unter Einfluss von Ultraschall.

Grundsätzlich kann während oder nach der Dispergierung noch eine Modifizierung von NCO-Gruppen mit isocyanatreaktiven Verbindungen wie primären oder sekundären Aminen oder (Poly)-Alkoholen erfolgen. Beispiele hierfür sind Ethylendiamin, 1,3-Propylendiamin, 1,6-Hexamethylendiamin, Isophorondiamin, 4,4'-Diaminodicyclohexylmethan, Hydrazin, 1,4-Butandiol, 1,4-Cyclohexandimethanol, 1,6-Hexandiol, Trimethylolethan, Trimethylolpropan, Glycerin, N-Methylethanol- und N-Methylisopropanolamin, 1-Amino-propanol oder Diethanolamin.

Bevorzugt beträgt das molekulare Verhältnis von NCO-Gruppen des hydrophilierten Polyisocyanats i) zu Wasser 1 zu 100 bis 1 zu 5, besonders bevorzugt 1 zu 30 bis 1 zu 10. Die Einhaltung dieser Verhältnisses ist vorteilhaft, um eine stabile Dispersion zu erhalten und die Reaktionsenthalpie abführen zu können.

Grundsätzlich ist es möglich das hydrophilierte Polyisocyanat i) in einer Portion in das Wasser einzudispergieren. Ebenfalls ist eine kontinuierliche Zugabe des hydrophilierten Polyisocyanates, beispielsweise über einen Zeitraum von 30 Minuten bis 20 Stunden, möglich. Bevorzugt ist eine portionsweise Zugabe, wobei die Anzahl der Portionen 2 bis 50, bevorzugt 3 bis 20, besonders bevorzugt 4 bis 10 beträgt, und die Portionen gleich oder auch unterschiedlich groß sein können.

Die Wartezeit zwischen den einzelnen Portionen beträgt typischerweise 5 Minuten bis 12 Stunden, bevorzugt 10 Minuten bis 8 Stunden, besonders bevorzugt 30 Minuten bis 5 Stunden.

Ebenfalls möglich ist eine über einen Zeitraum von 1 Stunde bis 24 Stunden, bevorzugt 2 Stunden bis 15 Stunden verteilte kontinuierliche Zugabe des hydrophilierten Polyisocyanats i).

Bei der Harnstoffpartikelherstellung beträgt die Temperatur im Kessel 10 bis 80 °C, bevorzugt 20 bis 70 °C und besonders bevorzugt 25 bis 50 °C.
Bevorzugt wird im Anschluss an die Umsetzung des hydrophilierten Polyisocyanats i) mit Wasser der Reaktor bei Innentemperaturen von 0 bis 80 °C, bevorzugt 20 bis 60 °C, besonders bevorzugt 25 bis 50 °C evakuiert. Die Evakuierung erfolgt bis zu einen Innendruck von 1 bis 900 mbar, bevorzugt 10 bis 800 mbar, besonders bevorzugt 100 bis 400 mbar. Die Dauer dieses sich an die eigentliche Reaktion anschließenden Entgasung beträgt 1 Minute bis 24 Stunden, bevorzugt 10 Minuten bis 8 Stunden. Eine Entgasung ist auch durch Temperaturerhöhung ohne Evakuierung möglich.

Bevorzugt wird gleichzeitig mit der Evakuierung die Nanoharnstoff-Dispersion A') durchmischt, z.B. durch Rühren.

Die Herstellung der wässrigen Dispersionen A') erfolgt bevorzugt in Anwesenheit von Katalysatoren.

Die zur Herstellung der Nanoharnstoff-Dispersionen A') eingesetzten Katalysatoren sind beispielsweise tertiäre Amine, Zinn-, Zink- oder Wismuthverbindungen oder basische Salze.

Geeignete Katalysatoren sind z.B. Eisen(II)-chlorid, Zinkchlorid, Zinnsalze, Tetraalkylammoniumhydroxide, Alkalihydroxide, Alkalialkoholate, Alkalisalze von langkettigen Fettsäuren mit 10 bis 20 Kohlenstoffatomen und gegebenenfalls seitenständigen OH-Gruppen, Bleioctoat oder tertiäre Amine wie Triethylamin, Tributylamin, Dimethylbenzylamin, Dicyclohexylmethylamin, Dimethylcyclohexylamin, N,N,N',N'-Tetramethyl-diamino-diethylether, Bis-(dimethylaminopropyl)harnstoff, N-Methyl- bzw. N-Ethylmorpholin, N,N'-Dimorpholinodiethylether (DMDEE), N-Cyclohexylmorpholin, N,N,N',N'-Tetramethylethylendiamin, N,N,N',N'-Tetramethylbutandiamin, N,N,N',N'-Tetramethylhexandiamin-1,6, Pentamethyldiethylentriamin, Dimethylpiperazin, N-Dimethylaminoethylpiperidin, 1,2-Dimethylimidazol, N-Hydroxypropylimidazol, 1-Azabicyclo-(2, 2,0)-octan, 1,4-Diazabicyclo-(2,2,2)-octan (Dabco) oder Alkanolaminverbindungen, wie Triethanolamin, Triisopropanolamin, N-Methyl- und N-Ethyldiethanolamin, Dimethyl-aminoethanol, 2-(N,N-Dimethylaminoethoxy)ethanol oder N-Tris-(dialkylaminoalkyl)hexahydrotriazine, z.B. N,N',N-Tris-(dimethylaminopropyl)-s-hexahydrotriazin.

Bevorzugt sind tertiäre Amine wie Tributylamin, Triethylamin, Ethyldiisopropylamin oder 1,4-Diazabicyclo-[2,2,2]-octan. Bevorzute Zinnverbindungen sind Zinndioctoat, Zinndiethylhexoat, Dibuthylzinndilaurat oder Dibutyldilaurylzinnmercaptid. Des Weiteren sind bevorzugt 2,3- Dimethyl-3,4,5,6-tetrahydropyrimidin, Tetramethylammoniumhydroxid, Natriumhydroxid, Natriummethylat oder Kaliumisopropylat.

Besonders bevorzugte Katalysatoren sind Natriumhydroxid, Triethylamin, Ethyldiisopropylamin oder 1,4-Diazabicyclo-[2,2,2]-octan.

Die Katalysatoren werden in Mengen von vorzugsweise 0 bis 8 Gew.-%, bevorzugt von 0,05 bis 5 Gew.-%, besonders bevorzugt von 0,1 bis 3 Gew.-%, jeweils bezogen auf den gesamten Festkörpergehalt der resultierenden Dispersion, eingesetzt.

Der Katalysator kann mit dem hydrophilierten Polyisocyanate i) oder mit dem Dispergierwasser gemischt werden oder nach der Dispergierung der Polyisocyanate i) in Wasser zugefügt werden. Bevorzugt ist es, den Katalysator dem Dispergierwasser vor Zusatz des Polyisocyanates i) beizumischen. Es ist auch möglich den Katalysator in Portionen aufzuteilen und zu unterschiedlichen Zeit punkten des Reaktionsverlaufes hinzuzufügen.

Es ist ebenfalls möglich, Lösemittel wie beispielsweise N-Methylpyrrolidon, N-Ethylpyrrolidon, Methoxypropyalcetat, Dimethylsulfoxid, Methyoxypropyacetat, Aceton und/oder Methylethylketon dem hydrophilierten Polyisocyanat i) vor der Dispergierung zuzugeben. Nach Abschluss der Reaktion und Dispergierung können flüchtige Lösemittel wie Aceton und/oder Methylethylketon durch Destillation entfernt werden. Bevorzugt ist die Herstellung ohne Lösemittel oder die Verwendung von Aceton oder Methylethylketon, besonders bevorzugt ist die Herstellung ohne organisches Lösemittel.

Prinzipiell ist die Entfernung des Wassers bei Atmosphärendruck, Unterdruck oder Überdruck möglich. In einer bevorzugten Verfahrensvariante wird das Wasser destillativ entfernt, wobei unter Druckverminderung und/oder unter Temperaturerhöhung gearbeitet werden kann.

Auch andere Techniken zur Abtrennung von Wasser sind möglich, wie beispielsweise die Entwässerung durch Membranverfahren oder der Einsatz von wasserziehenden Trockenmitteln, wie z. B. Silicagel oder Zeolithe. Auch die Kombination verschiedener Entwässerungstechniken, gleichzeitig oder nacheinander, ist möglich. Auch die Abtrennung des Wassers unter Zuhilfenahme von Additiven ist möglich, beispielsweise die Zumischung von Schleppmitteln zur vereinfachten destillativen Entfernung von Wasser.

Besonders bevorzugt ist die Trocknung mittels Gefriertrocknungsverfahren oder Sprühtrocknung, ganz besonders bevorzugt ist die Sprühtrocknung.

Die Sprühtrocknung kann mit Hilfe üblicher Verfahren durchgeführt werden. Beispielsweise kann mit Hilfe eines Rotationszerstäubers, eines Druckzerstäubers oder mittels pneumatischer Zerstäubung gearbeitet werden. Bevorzugt wird aber unter Verwendung eines Druckzerstäubers gearbeitet.

Für die Sprühtrocknung kommt zum Trocknen insbesondere ein erwärmtes Gas, insbesondere ein erwärmtes Inertgas, bevorzugt erwärmte Luft oder erwärmter Stickstoff zum Einsatz. Das Gas wird bevorzugt auf eine solche Temperatur erwärmt, dass bei der jeweiligen Ausflussgeschwindigkeit durch die Düse die Temperatur am Sprühkorn so hoch ist, dass einerseits eine gute Verdampfung des Wassers bzw. Lösemittels gewährleistet ist und andererseits die Temperatur am Sprühkorn maximal so hoch ist, dass keine Verklebungen/Vernetzungen o.ä. auftreten. Besonders bevorzugt sollte die Temperatur am Sprühkorn maximal 80 °C, ganz besonders bevorzugt 30 bis 70 °C, betragen. Besonders bevorzugt kommt Gas, das auf eine Temperatur von 50 bis 170 °C erwärmt wurde, zum Einsatz. Die zur Verdampfung des Wassers erforderliche Wärmemenge kann aber selbstverständlich auch teilweise oder vollständig durch andere Methoden, beispielsweise Strahlungsheizung, zugeführt werden.

Die für die Sprühtrocknung eingesetzte, zu versprühende Dispersion weist bevorzugt einen Festkörpergehalt von 5 bis 60 Gew.-%, besonders bevorzugt von 20 bis 50 Gew.-% auf.

Das Versprühen der Teilchen kann insbesondere mittels eines Rotationszerstäubers, mittels eines Druckzerstäubers oder mittels eines pneumatischen Zerstäubers, bevorzugt mittels eines Rotationszerstäubers, erfolgen. Die Verwendung eines Rotationszerstäubers weist dabei den Vorteil auf, dass sehr feinteilige Pulver mit einer engen Korngrößenverteilung erhalten werden. Außerdem sind insbesondere im Falle der Sprühtrocknung unter Verwendung eines Rotationszerstäubers die erhaltenen größeren Pulverteilchen hauptsächlich Aggregate kleinerer Teilchen, so dass leicht möglich ist.

Gegebenenfalls kann an die Sprühtrocknung noch ein Sichtvorgang (insbesondere mittels Windsichter) angeschlossen werden.

Im Falle der Verwendung eines Rotationszerstäubers beträgt der Flüssigkeitsdruck im Allgemeinen 2 bis 3 bar. Im Falle der Verwendung eines pneumatischen Zerstäubers oder eines Druckzerstäubers beträgt der Flüssigkeitsdruck bevorzugt 20 bis 50 bar.

Im Falle der Rotationszerstäuber liegt der Durchmesser der Anlage bevorzugt zwischen 2,5 und 3,5 m und die Länge beträgt bevorzugt 4,5 bis 5,5 m. Die Temperatur des zum Trocknen eingesetzten Gases liegt innerhalb der oben genannten Grenzen, bevorzugt bei 100 bis 200 °C. Die Menge an Trocknungsgas, bevorzugt Trocknungsluft, liegt bevorzugt bei 1000 bis 5500 Normkubikmetern pro Stunde.

Im Falle der Druckzerstäubung und der pneumatischen Zerstäubung wird ebenfalls mit üblichen Anlagen unter Verwendung von inerten Gasen oder Luft gearbeitet. Im Falle der pneumatischen Zerstäubung beträgt die verwendete Menge an Zerstäubungsluft bzw. Gas pro Tonne zu zerstäubendem Material im Allgemeinen etwa 1 Tonne und es wird vorzugsweise unter Einsatz feiner Düsen gearbeitet.

Die Durchflussgeschwindigkeit durch die Düse wird bei der Sprühtrocknung bevorzugt so eingestellt, dass beispielsweise auf einer Anlage der Firma Niro Atomizer pro Minute 50 ml bis 800 ml versprüht werden.

Die mittels der Sprühtrocknung hergestellten Pulver weisen im allgemeinen eine mittlere Teilchengröße zwischen 5 und 50 µm auf. Werden die Pulver unter Verwendung eines Rotationszerstäubers sprühgetrocknet, so liegt die mittlere Teilchengröße im Allgemeinen unter 15 µm. Bei Verwendung eines pneumatischen Zerstäubers liegt die mittlere Teilchengröße im Allgemeinen unter 50 µm und bei Verwendung eines Druckzerstäubers im Allgemeinen bei 20 bis 80 µm. Die mittlere Teilchengröße kann beispielsweise beeinflusst werden durch die Konzentration der eingesetzten Dispersion, die Düsendurchmesser, die Düsengeometrie, die Durchflussgeschwindigkeit durch die Düse oder Temperatur und Flussgeschwindigeit des Trocknungsgases, wobei der Fachmann durch Routineversuche unter Variation der vorstehenden Parameter die Teilchengröße einstellen kann.

Falls eine enge Korngrößenverteilung der Pulver gewünscht ist, kann an die Sprühtrocknung noch ein Sichten angeschlossen werden, insbesondere mittels eines Windsichters. Hierdurch kann die mittlere Teilchengröße beispielsweise auf 5 bis 25 µm, bevorzugt 8 bis 20 µm, eingestellt werden. Auch die Abtrennung möglicherweise unerwünschter grober oder feiner Anteile ist möglich. Im Anschluss an die Trocknung kann das erhaltene Pulver weiter gemahlen werden.

Beispiele für verwendbare Sprühtrockner sind Geräte der Firmen Niro (Dänemark), Anhydro (Dänemark), Nubilosa, Caldyn, Büchi, APV, Trema etc.

Bei der Herstellung der erfindungsgemäßen Partikel können auch Colöser, Entschäumer, oberflächenaktive Detergentien und andere Hilfsmittel und Additive verwendet werden. Auch die Beimischung anderer dispergierter Nanopartikel wie beispielsweise wässrige kolloiddisperse Lösung von Siliciumdioxid. Werden flüchtige Colösemittel eingesetzt, können diese wieder aus der erfindungsgemäßen Nanoharnstoff-Dispersion A) entfernt werden, beispielsweise zusammen mit der Entfernung des Wassers. Bevorzugt ist es, frei von Colöser zu arbeiten.

Auch die Zugabe von Trennmitteln wie beispielsweise Talkum ist möglich. Der Zusatz kann vor, während oder nach dem Trocknen der Nanoharnstoff-Dispersion erfolgen.

Es ist auch möglich, Farbstoffe, Aromastoffe, Pigmente und Wirkstoffe zuzufügen.

Weitere Additive, welche der Ausgangsdispersion zugesetzt werden können, sind beispielsweise Katalysatoren, filmbildende Polymere, Stabilisatoren, Lichtschutzmittel, Oxidationsschutzmittel, Biozide, Pigmente und/oder Füllstoffe. Der Zusatz kann vor, während oder nach der Herstellung der Nanoharnstoff-Dispersion erfolgen.

Die erfindungsgemäßen Partikel können als solche beispielsweise als Additiv, Bindemittel oder Hilfs- oder Zusatzstoff verwendet werden, beispielsweise für Beschichtungsmittel, Lacke, Anstrichstoffe, Klebstoffe, Kaschiermaterialien, Dichtstoffe, Druckfarben, Tinten, Färbungsmittel, Farbstoffe, Beizen, Korrosions- und Rostschutzmittel, Imprägniermittel, Schmier-, Gleit-, Trenn- oder Kühlmittel, Weichmacher, Verlaufsmittel, Reaktivverdünner, Additive; in Kosmetika bzw. als Kosmetikrohstoff, zur Herstellung pharmazeutischer Formulierungen, in Ölen, in Sonnenschutzmitteln, in bzw. als Verdickungs-, Reinigungs- bzw. Vorbehandlungsmittel und in Lebensmitteln aller Art.

Die Einbringung der erfindungsgemäßen Partikel kann beispielsweise erfolgen durch Einarbeitung mit einem Rührwerk oder andere Arten der Einbringung wie Umpumpen, Statikmischer, Stachelmischer, Düsenstrahldispergator, Rotor und Stator, Einarbeiten im Extruder, im Dreiwalzenwerk oder unter Einfluss von Ultraschall.

Bevorzugte Beispiele zum Einsatz der erfindungsgemäßen Partikel ist die Einbringung in Kosmetika, Beschichtungsmittel, Dichtstoffe oder Klebstoffe

Im folgenden wird eine besonders bevorzugte Anwendung der erfindungsgemäßen NanoharnstoffPulver beschrieben. Diese Anwendung betrifft deren Verwendung in der Kosmetik.

Bei der Auswahl eines kosmetischen Produktes achten die Verbrauchen besonders auf das Hautgefühl der kosmetischen Zusammensetzung bei dessen Auftrag und nach Einziehen des Produktes. Zahlreiche kosmetische Produkte hinterlassen beim Auftrag auf Haut und Haar ein fettiges klebriges Hautgefühl. Insbesondere führt im Fall von hautkosmetischen Produkten der Einsatz von hydrophilen Feuchthaltmittel, wie Glycerin und Lipiden als Schutzbarrieresubtanzen (wie zum Beispiel Mineralöle oder polare Öle) gegen den Wasserverlust, zur Bildung einen fettigen und teilweise klebrigen Film.

Der vorliegenden Erfindung möchte daher auch eine kosmetische Zusammensetzung bereitstellen, die bei der Verteilung auf die Haut oder das Haar glatte Eigenschaften aufweist. Unter "glatten Eigenschaften" wird im Rahmen der vorliegenden Erfindung im Wesentlichen verstanden, dass beim Auftragen auf die Haut oder das Haar die kosmetische Zusammensetzung ein nicht-fettiges, pudriges Hautgefühl, bevorzugt ein nicht-klebriges Hautgefühl, aufweist.

Dekorative kosmetische Zusammensetzungen bestehen, abhängig von der Formulierungsform, aus bis zum 80 Gew.-% Farbstoffe, insbesondere Pigmente, bezogen auf das Gesamtgewicht der Zusammensetzung. Der hohe Anteil an Farbstoffen bzw. Pigmenten hat den Nachteil, beim Auftrag ein raues Hautgefühl zurückzulassen. Zusätzlich führen zahlreiche dekorative kosmetische Zusammensetzungen aus dem Stand der Technik wegen der schweren Verteilbarkeit der Zusammensetzung bei Verteilung auf der Haut zu einer farblich inhomogenen Schminke mit sichtbaren Farbspuren.
Daher besteht im Allgemeinen Verbesserungsbedarf für dekorative kosmetische Zusammensetzungen. Gewünscht ist die Entwicklung einer dekorativen kosmetischen Zusammensetzung, welche beim Auftrag auf der Haut eine einfach verteilbare, farblich homogene Schminke ohne sichtbare Farbspuren bildet. Die anderen wichtigen Eigenschaften von dekorativen kosmetischen Produkten wie zum Beispiel long lasting sollen dadurch gleichzeitig nicht vernachlässigt werden.

Erfindungsgemäß wird diese Aufgabe durch den Einsatz des erfindungsgemäßen Polyharnstoffpulvers, welches zuvor beschrieben wurde, und/oder einer nicht-filmbildenden Polyharnstoffdispersion, umfassend quervernetzte Nanoharnstoffe, gelöst. Als die nicht-filmbildende Polyharnstoffdispersion kann diejenige Polyharnstoffdispersion verwendet werden, aus weicher durch Trocknung die erfindungsgemäßen, zuvor beschriebenen Polyharnstoffpulver erzeugt werden.

### Kosmetische Zusammensetzung

Die erfindungsgemäße kosmetische Zusammensetzung, enthaltend das erfindungsgemäße Nanoharnstoffpulver und/oder die nicht-filmbildende Polyharnstoffdispersion, liegt als wässrige oder wässrige-alkoholische Löungen, Öl-in-Wasser-, Silikon-in-Wasser, Wasser-in-Öl oder Wasser-in-Silkonemulsion und Mischform, Multiple Emulsion wie zum Beispiel Öl-in-Wasser-in-Öl-, Wasser-in-Öl-in-Wasser- Emulsion, Polymer stabilisierte Emulsion (so genannte Hydrodispersion), feststoffstabilisierte Emulsion (auch Pickering-Emulsion genannt), PIT-Formulierung und Pulver in Form von Cremen, Lotionen, Schäumen, Sprays (Pump-Spray oder Aerosol), Gelen, Gelsprays, Ölen, Ölgelen, Mousse, "loose Pulver", Kompaktpulver oder Stiftformulierungen für Anwendung auf Haar und/oder Haut vor.

Die erfindungsgemäßen kosmetischen Zusammensetzungen enthalten die erfindungsgemäßen Nanoharnstoffpulver und/oder die nicht-filmbildende Polyharnstoffdispersion sowie gegebenenfalls in der Kosmetik übliche Wirkstoffe und Hilfsmittel, die aus der Gruppe, bestehend aus Emulgatoren; Tensiden; Konservierungsmitteln; Parfümölen; kosmetischen Wirkstoffen, wie Phytantriol, Vitamin A, E und C, Retinol, Bisabolol, Panthenol; organischen oder inoragnischen Lichtschutzmitteln; Bleichmitteln, Färbemitteln; Tönungsmitteln; Bräunungsmitteln; Stabilisatoren; pH-Wert-Regulatoren; Farbstoffen; Salzen; Verdickern; Gelbildnern; Konsistenzgebern; Silikonen; Feuchthaltemitteln; Konditioniermitteln; Filmbildnern, Rückfettern und weiteren üblichen Additiven, ausgewählt werden.

Die erfindungsgemäßen kosmetischen Zusammensetzungen können unter anderem als
(1) hautpflegende Zusammensetzung oder
(2) haarkosmetische Zusammensetzung

ausgebildet sein, wobei die vorliegende Erfindung nicht auf diese speziellen Arten der kosmetischen Zusammensetzungen beschränkt sind.

### Hautpflegende Zusammensetzung

Im Sinne der Erfindung kann die kosmetische Zusammensetzung eine hautkosmetische Zusammensetzung sein. Eine hautkosmetische Zusammensetzung ist definiert als ein kosmetisches Mittel zur Reinigung, Pflege und zum Schutz der Haut. Im Sinne der vorliegenden Erfindung sind hautkosmetische Zusammensetzungen Hautpflegeprodukt, Sonnenschutzmittel, After-sun-Präparate, Selbstbräuner, dekorative Kosmetik, Wasch-, Dusch- und Badepräparate zur Anwendung auf der Haut, Gesichtwasser, Gesichtmasken, Insect-Repellent-Präparate, Fußpflegemittel, Rasiermittel, Haarentfernungsmittel, Intimpflegemittel, Babypflegemittel, Deodorantien und Antitranspirantien.

Bevorzugte hautkosmetische Zusammensetzung im Sinne der vorliegenden Erfindung sind Hautpflegeprodukte, Sonnenschutzmittel, Selbstbräunungsmittel und dekorative Kosmetik.

Bei einem Hautpflegeprodukt handelt es ich um eine kosmetische Zusammensetzung zum Auftragen auf die Haut, das Gesicht oder/und den Körper zum Schutz gegen Hautveränderungen, beispielweise Hautalterung, Austrocknung usw.

Entsprechend ihrem Aufbau können die erfindungsgemäßen Zusammensetzungen beispielweise als Gesichtscreme, Tages- oder Nachtcreme, Augencreme, Antifaltencreme, Whitening-Produkte, Körperlotion, Tränkungsmedium, After-sun-Präparate usw. verwendet werden. Es ist gegebenenfalls möglich, dass die erfindungsgemäßen Zusammensetzungen als pharmazeutisches Produkt verwendet werden.

Insect-Repellent-Präparat sind im Rahmen der vorliegenden Erfindung Präparate, die zur Abwehr und Vertreibung von Insekten, insbesondere von Mücken, Zecken und Milben, äußerlich verwendet werden. In solchen Formulierungen werden Wirkstoffe eingesetzt, welche die Insekten aufgrund der Bildung eines Duftmantels über der Haut von der Haut entfernt halten.

Bei einem Sonnenschutzmittel handelt es sich um eine Zusammensetzung zum Schutz der Haut gegenüber kurzwelliger und längerwelliger Sonnenstrahlung. Ein Sonnenschutzmittel enthält mindestens eine Lichtschutzfiltersubtanz (UVA-, UVB- und/oder Breitbandfilter).

Die erfindungsgemäßen Zusammensetzungen können als Hautpflegeprodukte, enthaltend das erfindungsgemäße Nanoharnstoff-Pulver und/oder die nicht-filmbildende Polyharnstoffdispersion, Feuchthaltmittel und gegebenenfalls weitere kosmetische und/oder dermatologische Wirk-, Hilfs- und Zusatzstoffe, ausgebildet sein.

Die erfindungsgemäßen Zusammensetzungen können als Insect-Reppelent-Präparate, enthaltend mindestens ein erfindungsgemäßes Nanoharnstoffpulver und/oder die nicht-filmbildende Polyharnstoffdispersion, Insect-Repellent-Wirkstoffe und gegebenenfalls weitere kosmetische und/oder dermatologische Wirk-, Hilfs- und Zusatzstoffe, ausgebildet sein.

Vorteilhaft werden als Insect-Repellent-Wirkstoffe 3-(N-n-Butyl-N-acetyl-amino)-propionsäureethylester (unter der Handelsbezeichnung Repellent 3535 erhältlich), N,N-Ditethyl-m-toluamid (so genannt DEET) und 2-(2-Hydroxyethyl)-piperidin-1-carbonsäure-2-butylester (unter der Handelsbezeichnung Bayrepel^{®} erhältlich) eingesetzt.

Die erfindungsgemäßen Zusammensetzungen könne auch als Sonnenschutzmittel, enthaltend mindestens ein erfindungsgemäßes Nanoharnstoffpulver und/oder die nicht-filmbildende Polyharnstoffdispersion, mindestens eine oder mehrere Lichtschutzfiltersubtanzen und gegebenenfalls weitere kosmetische und/oder dermatologische Wirk-, Hilfs- und Zusatzstoffe, ausgebildet sein.

Lichtschutzfiltersubtanzen könne aus der Gruppe, bestehend aus den UVA-, UVB-, Sreitbandfiltern und deren Mischungen, ausgewählt werden.

Die erfindungsgemäßen Zusammensetzungen könne als Selbstbräunungsmittel, enthaltend mindestens ein erfindungsgemäßes Nanoharnstoffpulver und/oder die nicht-filmbildende Polyharnstoffdispersion, mindestens ein oder mehrere selbtsbräunende Subtanzen und gegebenenfalls weitere kosmetische und/oder dermatologische Wirk-, Hilfs- und Zusatzstoffe, ausgebildet sein.

Selbstbräunungsmittel enthalten mindestens ein oder mehrere selbtsbräunende Subtanzen, die bevorzugt ausgewählt werden aus der Gruppe, bestehend aus Glycerolaldehyd, Hydroxymethylglyoxal, γ-Dialdehyd, Erythrulose, 5-Hydroxy-1,4-naphtochionon, 2-Hydroxy-1,4-naphtochinon, 1-, 3-Dihydorxyaceton (DHA), 6-Aldo-D-Fructose und Ninhydrin.

Bei einer dekorativen kosmetischen Formulierung handelt es sich um eine kosmetische Zusammensetzung zur farblichen Gestaltung der menschlichen Haut, Schleimhaut, Semi-Schleimhaut, des Haar und des Nagels. Die erfindungsgemäße dekorative Formulierung kann ein Gesichts-Make-up (Foundation), eine getönte (Tages-)Creme, ein Blush, ein Rouge, eine Wimperntusche, ein Eyeliner, ein Kajal, ein Lidschatten, ein Lippenstift, ein Lip-Gloss zur farblichen Veränderung oder zum Schminken des Körpers gegen Augenringe, inhomogenen Teint oder weitere Unvollkommenheiten der Haut wie Rötungen, Flecken, Falten oder Pickel sein. Die Liste von dekorativen Produkten ist im Rahmen der vorliegenden Erfindung selbstverständlich nicht limitierend zu verstehen.

Die erfindungsgemäßen Zusammensetzungen können als dekorative kosmetische Zusammensetzungen, enthaltend mindestens ein erfindungsgemäßes Nanoharnstoffpulver und/oder die nicht-filmbildende Polyharnstoffdispersion, mindestens einem Farbstoff und gegebenenfalls weitere kosmetische und/oder dermatologische Wirk-, Hilfs- und Zusatzstoffe, ausgebildet sein. Die Farbstoffe könne ausgewählt werden aus der Gruppe, bestehend aus löslichen Farbstoffen; anorganischen Pigmenten, wie zum Beispiel Eisenoxiden und Chromoxiden; Ultramarin; Manganviolett; organischen Pigmenten und Perlmutt.

Die erfindungsgemäßen hautkosmetischen Zusammensetzungen können fest (Stick), flüssig (Lotion, Pflegeöl) oder halbfest (Creme, Salben oder gelartige Produkte) sein. Die Zusammensetzungen können beispielweise in Form einer Öl-in-Wasser-, Silikon-in-Wasser- Wasser-in-Öl-, Wasser-in-Silikon-, Öl-in-Wasser-in-Öl- oder Wasser-in-Öl-in-Wasser-Emulsion vorliegen. Die Zusammensetzung können ferner mit einem Treibgas aufgeschäumt werden (so genanntes Mousse). Die oben erwähnten Emulsionen können durch O/W-, W/O- oder W/Si-Emulgator, Verdicker (wie beispielweise im Fall einer Hydrodispersion) oder Feststoffe (wie beispielweise Pickeringemulsion) stabilisiert sein. Die erfindungsgemäße Formulierung kann in Form von "loose Pulver" oder Kompaktpulver vorliegen.

### Haarkosmetische Zusammensetzung

Eine bevorzugte Verwendung in Sinne der vorliegenden Erfindung ist die Verwendung der erfindungsgemäßen Pulver und/oder der nicht-filmbildende Polyharnstoffdispersion in einer haarkosmetische Zusammensetzung, ausgewählt aus der Gruppe, bestehend aus Egalisierungsmittel für Dauerwellen, Curl Relaxer, Styling Wrap Lotion, Haarfestiger, Haarverformungsmittel, Haarfärbemittel, Haarkure und Shampoo.

Die erfindungsgemäßen haarkosmetische Zusammensetzungen enthalten mindestens ein erfindungsgemäßes Pulver und/oder die nicht-filmbildende Polyharnstoffdispersion, mindestens ein Komponente, die ausgewählt ist aus der Gruppe, bestehend aus Konditioniermitteln, Filmbildnern und Tensiden, und gegebenenfalls weiteren kosmetischen und/oder dermatologischen Wirk-, Hilfs- und Zusatzstoffen.

### Inhaltstoffe für die erfindungsgemäßen kosmetischen Zusammensetzungen

### Öle, Fette, Wachse

Die erfindungsgemäßen haut- und haarkosmetischen Zusammensetzungen enthalten bevorzugt weitere nicht flüchtige und/oder flüchtige Öle, Fette und/oder Wachse.

Nicht flüchtige Öle und Fette werden vorteilhaft ausgewählt aus der Gruppe, bestehend aus Ölen und Fetten aus mineralischer, tierischer, pflanzlicher oder synthetischer Herkunft; polaren oder unpolaren Ölen und deren Gemischen. Nicht flüchtige Öle und Fette der erfindungsgemäßen Zusammensetzungen können vorteilhaft ausgewählt werden aus folgender Substanzgruppe: Mineralöle, polare Öle, wie Triglyceride der Caprin- oder der Caprylsäure, ferner natürliche Öle wie z.B. Rizinusöl;

Fette, natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;

Alkylbenzoate; Silikonöle wie Dimethylpolysiloxane, Diethylpolysiloxane, Diphenylpolysiloxane sowie Mischformen daraus.

Die polaren Öle werden vorteilhaft ausgewählt aus der Gruppe, bestehend aus:
a) Estern aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen.
b) Estern aus aromatischen Carbonsäuren und gesättigten und/oder ungesättigten, verzweigten und/oder unverzweigten Alkoholen einer Kettenlänge von 3 bis 30 C-Atomen.
   Solche Esteröle können dann vorteilhaft ausgewählt werden aus der Gruppe, bestehend aus:
   Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, lsotridecylisononanoat, 2-Ethylhexylpalmitat, 2-Ethylhexyllaurat, 2-Ethylhexylisostearat, 2-Hexyldecylstearat, 2-Octyldodecylpalmitat, 2-Ethylhexylcocoat, Oleyloleat, Oleylerucat, Erucyloleat, Erucylerucat Dicaprypyp Carbonat (Cetiol CC) und Cocoglyceride (Myritol 331) sowie synthetische, halbsynthetische und natürliche Gemische solcher Ester, z.B. Jojobaöl;
c) Alkylbenzoate (C12-15-Alkylbenzoate (Finsolv^{®} TN von Finetex)) oder 2-Phenylethylbenzoate (X-Tend 226 von ISP).
d) Lecithine und den Fettsäuretriglyceride, insbesondere Triglycerinester gesättigter und/oder ungesättigter, verzweigter und/oder unverzweigter Alkancarbonsäuren einer Kettenlänge von 8 bis 24, insbesondere 12 bis 18 C-Atomen.
   Beispielweise können die Fettsäuretriglyceride ausgewählt werden aus der Gruppe, bestehend aus Cocoglycerid, Olivenöl, Sonnenblumenöl, Sojaöl, Erdnussöl, Rapsöl, Mandelöl, Palmöl, Kokosöl, Rizinusöl, Weizenkeimöl, Traubenkernöl, Distelöl, Nachtkerzenöl, Macadamianussöl, Aprikosenkernöl, Avocadoöl und dergleichen.
e) Dialkylether und Dialkylcarbonate, wobei z.B. Dicaprylylether (Cetiol^{®} OE der Firma Cognis) und/oder Dicaprylylcarbonat (beispielsweise Cetiol^{®} CC der Firma Cognis) bevorzugt sind.
f) gesättigte oder ungesättigte, verzweigte oder unverzweigte Alkohole, wie zum Beispiel Octyldodecanol.

Nicht flüchtige Öle können ebenfalls vorteilhaft auch unpolare Öle sein, welche ausgewählt werden aus der Gruppe, bestehend aus den verzweigten und unverzweigten Kohlenwasserstoffen, insbesondere Mineralöl, Vaselineöl, Paraffinöl, Squalan und Squalen; Polyolefinen, beispielweise Polydecene, hydrogenierte Polyisobutene, C13-16 Isoparaffin und lsohexadecan.

Unpolare nicht flüchtige Öle können aus den nicht flüchtigen Silikonölen ausgewählt werden.

Von den nicht flüchtigen Silikonölen können die Polydimethylsiloxane (PDMS), die gegebenenfalls phenyliert sind, wie Phenyltrimethicon, oder gegebenenfalls substituiert sind mit aliphatischen und/oder aromatischen Gruppen oder mit funktionellen Gruppen, beispielsweise Hydroxygruppen, Thiolgruppen und/oder Aminogruppen; mit Fettsäuren, Fettalkoholen oder Polyoxyalkylenen modifizierte Polysiloxane und deren Gemische angegeben werden.

Besondere vorteilhafte Öle sind 2-Ethylhexylisostearat, Octyldodecanol, Isotridecylisononanoat, Isoeicosan, 2-Ethylhexylcocoat, C12-15 Alkylbenzoat, Caprylic/Capric Triglycerid, Dicaprylylether, Mineral Öl, Dicaprylylcarbonate, Cocoglyceride, Butylenglykol Dicarprylate/Dicaprate, Hydrogenated Polyisobutene, Cetaryl Isononanoate, Isodecyl Neopentanoate, Squalan und C13-16 Isoparaffin.

Die erfindungsgemäßen Zusammensetzungen können ferner ein Wachs enthalten.

Im Sinne der vorliegenden Schrift ist ein Wachs als lipophile Fettsubstanz definiert, die bei Raumtemperatur (25°C) fest ist und bei einer Schmelztemperatur zwischen 30°C und 200°C eine reversible Zustandsänderung fest/flüssig zeigt. Oberhalb des Schmelzpunktes wird das Wachs niedrigviskos und mischbar mit Ölen.

Das Wachs wird vorteilhaft gewählt aus der Gruppen von natürlichen Wachsen wie beispielweise Baumwollwachs, Carnaubawachs, Candelillawachs, Espartoawachs, Japanwachs, Montanwachs, Zuckerrohrwachs, Bienenwachs, Wollwachs, Schellack, Mikrowachse, Ceresin, Ozokerit, Ouricuriwachs, Korkfaserwachs, Lignitwachse, Berrenwachs, Sheabutter oder synthetische Wachse wie Paraffinwachse, Polyethylenwachse, durch Fischer-Tropsch-Synthese hergestellte Wachse, hydrierte Öle, Fettsäureester und Glyceride, die bei 25 °C fest sind, Silikonwachse und Derivaten (Alkylderivate, Alkoxyderivate und/oder Ester von Polymethylsiloxan) und deren Gemischen. Die Wachse können in Form von stabilen Dispersionen von kolloidalen Wachspartikeln vorliegen, die nach bekannten Verfahren hergestellt werden können, beispielsweise gemäß "Microemulsions Theory and Practice", L.M. Prince Ed., Academic Press (1977), Seiten 21-32.

Die erfindungsgemäßen Zusammensetzungen können ferner ein flüchtiges Öl enthalten, das aus der Gruppe von flüchtigen Kohlenwasserstoffölen, silikonierten Ölen oder fluorierten Ölen ausgewählt wird.

Im Sinne der vorliegenden Erfindung ist ein flüchtiges Öl ein Öl, das im Kontakt mit der Haut bei Raumtemperatur und Atmosphärendruck in weniger als einer Stunde verdampft. Das flüchtige Öl ist bei Raumtemperatur flüssig und weist bei Raumtemperatur und Atmosphärendruck einen Dampfdruck von vorzugsweise 0,13 bis 40 000 Pa (10⁻³ bis 300 mg Hg), insbesondere 1,3 bis 13 000 Pa (0,01 bis 100 mm Hg), besonders bevorzugt 1,3 bis 13 00 Pa (0,01 bis 10 mm Hg), und einen Siedepunkt von vorzugsweise von 150 bis 260 °C, besonders bevorzugt 170 bis 250 °C, auf.

Unter einem Kohlenwasserstofföl wird ein Öl verstanden, das im Wesentlichen aus Kohlenstoffatomen und Wasserstoffatomen und gegebenenfalls Sauerstoffatomen oder Stickstoffatomen gebildet wird und keine Siliziumatome oder Fluoratome enthält, wobei es auch aus Kohlenstoffatomen und Wasserstoffatomen bestehen kann; es kann mindestens eine Estergruppe, Ethergruppe, Aminogruppe und/oder Amidgruppe enthalten.

Unter einem silikonierten Öl wird ein Öl verstanden, das mindestens ein Siliziumatom und insbesondere Si-O-Gruppen enthält.

Unter einem fluorierten Öl ist ein Öl zu verstehen, das mindestens ein Fluoratom enthält.

Das flüchtige Kohlenwasserstofföl kann unter den Kohlenwasserstoffölen mit einem Flammpunkt von im Allgemeinen 40 bis 102 °C, vorzugsweise 40 bis 55 °C, besonders bevorzugt 40 bis 50 °C, ausgewählt werden.

Beispielweise sind die flüchtigen Kohlenwasserstoffölen flüchtige Kohlenwasserstofföle mit 8 bis 16 Kohlenstoffatomen und deren Gemische, insbesondere verzweigte C₈₋₁₆-Alkane, wie die Isoalkane (die auch als Isoparaffine bezeichnet werden) mit 8 bis 16 Kohlenstoffatomen, insbesondere lsododecan, Isodecan und Isohexadecan, sowie beispielsweise die Öle, die unter den Handelsnamen Isopars^{®} oder Permetyls^{®} angeboten werden; und die verzweigten C₈₋₁₆-Ester, wie Isohexylneopentanoat und deren Gemische.

Besonders vorteilhaft sind die flüchtigen Kohlenwasserstofföle wie Isododecan, Isodecan und lsohexadecan.

Das flüchtige silikonierte Öl kann unter den silikonierten Ölen mit einem Flammpunkt von im Allgemeinen 40 bis 102 °C, vorzugsweise einem Flammpunkt über 55 °C und höchstens 95 °C, besonders bevorzugt im Bereich von 65 bis 95 °C, ausgewählt werden.

Beispielweise können für die flüchtigen silikonierten Öle die geradkettigen oder cyclischen Silikonöle mit 2 bis 7 Siliziumatomen genannt werden, wobei diese Silikone gegebenenfalls Alkyl- oder Alkoxygruppen mit 1 bis 10 Kohlenstoffatomen enthalten.

Besonders vorteilhaft sind die flüchtigen silikonierten Öle wie Octamethylcyclotetrasiloxan, Decamethylcyclopentasiloxan, Dodecamethylcyclohexasiloxan, Heptamethylhexyltrisiloxan, Heptamethyloctyltrisiloxan, Hexamethyldisiloxan, Octamethyltrisiloxan, Decamethyltetrasiloxan, Dodecamethylpentasiloxan und deren Gemische.

Das flüchtige fluorierte Öl besitzt im Allgemeinen keinen Flammpunkt.

Beispielweise sind die flüchtigen fluorierten Öle Nonafluorethoxybutan, Nonafluormethoxybutan, Decafluorpentan, Tetradecafluorhexan, Dodecafluorpentan und Mischungen davon.

Falls vorhanden kann die Fettphase der erfindungsgemäßen Zusammensetzungen ein nicht flüchtiges Öl und/oder flüchtiges Öl, Fette und Wachse enthalten. Die O/W- Zusammensetzung enthält vorzugsweise 0,01 bis 45 Gew.-% Öle, besonders bevorzugt 0,01 bis 20 Gew-% Öle, jeweils bezogen auf das Gesamtgewicht der Zusammensetzung. Die W/O- oder W/Si-Zusammensetzung enthält vorzugsweise mindestens 20 Gew.-% Öle, bezogen auf das Gesamtgewicht der Zusammensetzung.

### Verdickungsmittel

Die erfindungsgemäßen Zusammensetzungen können, sofern eine wässrige Phase enthalten ist, vorteilhaft Verdicker (der Wasserphase) enthalten. Vorteilhafte Verdicker sind:
- Homo- oder Copolymere von Vernetzter oder nichtvernetzter Acrylsäure oder Methacrylsäure. Hierzu gehören vernetzte Hompolymere von Methacrylsäure oder Acrylsäure, Copolymerisate aus Acrylsäure und/oder Methacrylsäure und Monomeren, die von anderen Acryl- oder Vinylmonomeren abgeleitet sind, wie C10-30 Alkylacrylate, C10-30-Alkylmethacrylate, Vinylacetat und Vinylpyrrolidone;
- Verdickende Polymere natürlicher Herkunft beispielweise auf Basis von Cellulose, Guargummi, Xanthan, Scleroglucan, Gellangummi, Rhamsan und Karayagummi, Alginate, Maltodextrin, Stärke und ihre Derivate, Johannisbrotkernmehl, Hyaluronsäure, Carrageenan;
- Nichtionische, anionische, kationische oder amphotere assoziative Polymere z.B. auf Basis von Polyethylenglycole und ihre Derivaten, oder Polyurethanen; und
- Vernetzte oder nichtvernetzte Homopolymere oder Copolymere auf Basis von Acrylamid oder Methacrylamid, wie Homopolymere von 2-Acrylamido-2-methylpropansulfonsäure, Copolymere von Acrylamid oder Methacrylamid und Methacryloyloxyethyltrimethylammoniumchlorid oder Copolymere von Acrylamid und 2-Acrylamido-2-methylpropansulfonsäure.

Besondere vorteilhafte Verdicker sind verdickende Polymere natürlicher Herkunft, Homo- oder Copolymere von vernetzter Acrylsäure oder Methacrylsäurer und vernetzte Copolymere von 2-Acrylamido-2-methylpropansulfonsäure.

Ganz besondere vorteilhafte Verdicker sind Xanthangummi, wie die unter den Bezeichnungen Keltrol^{®} und Kelza^{®} von der Firma CP Kelco angebotenen Produkte oder die Produkte der Firma RHODIA mit der Bezeichnung Rhodopol^{®}, und Guargummi, wie die unter der Bezeichnung Jaguar^{®} HP105 von der Firma RHODIA erhältlichen Produkte.

Ganz besondere vorteilhafte Verdicker sind vernetzte Homopolymere von Methacrylsäure oder Acrylsäure, die von der Firma Lubrizol unter den Bezeichnungen Carbopol^{®} 940, Carbopol^{®} 941, Carbopol^{®} 980, Carbopol^{®} 981, Carbopol^{®} ETD 2001, Carbopol^{®} EDT 2050, Carbopol^{®} 2984, Carbopol^{®} 5984 und Carbopol^{®} Ultrez 10; und von der Firma 3V unter den Bezeichnungen Synthalen^{®} K, Synthalen^{®} L und Synthalen^{®} MS im Handel erhältlich sind.

Ganz besondere vorteilhafte Verdicker sind vernetzte Copolymer von Acrylsäure oder Methacrylsäure und einem C₁₀₋₃₀-Alkylacrylat oder C₁₀₋₃₀-Alkylmethacrylat und Coplymere von Acrylsäure oder Methacrylsäure und Vinylpyrrolidone. Solche Copolymere sind beispielsweise von der Firma Lubrizol unter den Bezeichnungen Carbopol^{®} 1342, Carbopol^{®} 1382, Pemulen^{®} TR1 oder Pemulen^{®} TR2 und von der Firma ISP unter den Bezeichnungen Ultrathix^{®} P-100 (INCI: Acrylic Acid/VP Crosspolymer) im Handel erhältlich.

Ganz besondere vorteilhafte Verdicker sind vernetzte Copolymere von 2-Acrylamido-2-methylpropansulfonsäure. Solche Copolymere sind beispielsweise von der Firma Clariant unter den Bezeichnungen Aristoflex^{®} AVC (INCl: Ammonium Acryloyldimethyltaurate/VP Copolymer) erhältlich.

Diese Verdicker sind im Allgemeinen in einer Konzentration von etwa 0 % bis 2 Gew.-%, vorzugsweise 0 % bis 1 Gew.-%, jeweils bezogen auf das Gesamtgewicht der Zusammensetzung, vorhanden.

Zur Stabilisierung der erfindungsgemäßen W/O Emulsionen gegen Sedimentierung oder Flockung der Wassertröpfchen kann ein Ölverdicker eingesetzt werden. Ölverdicker können auch als Konsistenzgeber in Öle-haltigen Zusammensetzungen eingesetzt werden.

Besonders vorteilhaft Ölverdicker sind organomodifizierte Tonen wie organomodifizierte Bentonite (Bentone^{®} 34 der Firma Rheox) organomodifizierte Hectorite (Bentone^{®} 27 und Bentone^{®} 38 der Firma Rheox) oder organomodifizierte Montmorillonit, hydrophobe pyrogene Kiesselsäure, wobei die Silanolgruppen mit Trimethylsiloxygruppen subtituiert sind (AEROSIL^{®} R812 der Firma Degussa) oder mit Dimethylsiloxygruppen oder Polydimethylsiloxan (AEROSIL^{®} R972, AEROSIL^{®} R974 von Degussa, CAB-O-SIL^{®} TS-610, "CAB-O-SIL^{®} TS-720 von Cabot), Magnesium- oder Aluminiumstearat, oder Styrol-Copolymere wie zum Beispiel Styrol-Butadiene-Styrol, Styrol-Isopropen-Styrol, Styrol-Ethylen/Buten-Styrol oder Styrol-Ethylen/Propen-Styrol.

Das Verdickungsmittel für die Fettphase kann in einer Menge von im Allgemeinen 0,1 bis 5 Gew.- %, vorzugsweise 0,4 bis 3 Gew.-%, jeweils bezogen auf das Gesamtgewicht der Zusammensetzung enthalten sein.

### Emulgatoren

Die erfindungsgemäßen Zusammensetzungen in Form einer Emulsionen wie beispielweise Öl-in-Wasser-, Silikon-in-Wasser-, Wasser-in-Öl-, Wasser-in-Silikon-, Öl-in-Wasser-in-Öl-, Wasser-in-Öl-in-Wasser- Emulsion können einen emulgatoren enthalten.

Von dem Fachmann bekannt, hängt der Auswahl des Emulgators von der Darreichungsform der erfindungsgemäßen Zusammensetzungen ab. So enthalten erfindungsgemäße Öl-in-Wasser-Emulsionen (O/W) bevorzugt mindestens einen Emulgator mit einem HLB-Wert > 7 und gegebenenfalls einen Coemulgator. Die Wasser-in-Öl- (W/O) oder Wasser-in-Silikon-Emulsionen (W/Si) enthalten bevorzugt die ein oder mehrere Silikonemulgatoren (W/S) mit einem HLB-Wert ≤ 8 oder ein oder mehrere W/O-Emulgatoren mit einem HLB-Wert < 7 und gegebenenfalls ein oder mehrere O/W-Emulgatoren mit einem HLB-Wert > 10 enthalten.

O/W Emulgatoren können vorteilhaft ausgewählt werden aus der Gruppe der nichtionischen, anionischen, kationischen oder amphoteren Emulgatoren.

Unter den nichtionischen Emulgatoren befinden sich:
a) Partialfettsäureester und Fettsäureester mehrwertiger Alkohole und deren ethoxylierte Derivate;
b) ethoxylierte Fettalkohole und Fettsäuren;
c) ethoxylierte Fettamine, Fettsäureamide, Fettsäurealkanolamide;
d) Alkylphenolpolyglycolether (z.B. Triton X); und
e) ethoxylierte Fettalkoholether

Besonders vorteilhafte nichtionische O/W-Emulgatoren sind ethoxylierte Fettalkohole oder Fettsäuren, bevorzugt PEG-100-Stearat, PEG-40-Stearat, PEG-50 stearate , Ceteareth-20, Ceteth-20, Steareth-20, Ceteareth-12, Ceteth-12, Steareth-12, Ester aus Mono-, Oligo- oder Polysacchariden mit Fettsäuren, bevorzugt Cetearylglucosid, Methylglucosedistearat, Glycerylmonostearate (selbstemulgierend),Sorbitanester wie zum Beispiel Sorbitanstearate (Tween^{®} 20 und Tween^{®} 60 der Firma Uniqema), Sorbitanpalmitate (span 40, Uniqema), Glycerylstearylcitrate, Sucroseester wie zum beispiel Sucrosestearate, PEG-20 methylglucose sequistearate) Dicarbonsäureester von Fettalkohol (Dimyristyltartrate).

Vorteilhafte anionische Emulgatoren sind Seifen (z. B. Natrium- oder Triethanolamin-Salze der Stearin- oder Palmitinsaeure), Ester der Zitronensäure wie Glycerylstearatcitrat, Fettalkoholsulfate sowie Mono-, Di- und Trialkylphosphosäureester und deren Ethoxylate.

Unter den kationischen Emulgatoren befinden sich quaternäre Ammoniumverbindungen mit einem langkettigen aliphatischen Rest z.B. Distearyldimonium Chloride.

Unter den amphoteren Emulgatoren befinden sich:
a) Alkylamininoalkancarbonsäuren;
b) Betaine, Sulfobetaine; und
c) Imidazolinderivate

Weiterhin gibt es natürlich vorkommende Emulgatoren, zu denen Bienenwachs, Wollwachs, Lecithin und Sterole gehören.

Die Silikonemulgatoren können vorteilhaft aus der Gruppe enthaltend Alkyldimethiconcopolyole wie z. B. Cetyl PEG/PPG 10/1 Dimethiconcopolyol (ABIL^{®} EM 90 der Fa. Evonik) oder Lauryl PEG/PPG-18/18 Dimethicone (Dow Corning^{®} 5200 Formulation Aid der Fa. Dow Corning Ltd.) und Dimethiconecopolyole wie z. B. PEG-10 Dimethicone (KF-6017 der Fa. Shin Etsu), PEG/PPG-18/18 Dimethicone (Dow Corning Formulation aid 5225C der Fa. Dow Corning Ltd.), PEG/PPG-19/19 Dimethicone (Dow Corning BY-11 030 der Fa. Dow Corning Ltd.) oder Trimethylsilylamodimethicone gewählt werden.

Die W/O-Emulgatoren mit einem HLB-Wert < 7 können vorteilhaft aus der Gruppe Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 bis 18 C-Atomen, Propylenglycolester gesättigter und/oder ungesättigter, verzweigter und/oder unverzweigter Alkancarbonsäuren einer Kettenlänge von 8 bis 24, insbesondere 12 bis 18 C-Atomen sowie Sorbitanester gesättigter und/oder ungesättigter, verzweigter und/oder unverzweigter Alkancarbonsäuren einer Kettenlänge von 8 bis 24, insbesondere 12 bis 18 C-Atomen.

Besonders vorteilhafte *W*/*O-Emulgatoren* sind :Glycerylmonostearat" Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol,Chimylalkohol, Polyethylenglycol(2)stearylether (Steareth-2), Glycerylmonolaurat, Glycerylmonocaprinat und Glycerylmonocaprylat.

Weitere mögliche W/O-Emulgatoren warden gewählt aus der Gruppe der Verbindungen Polyglyceryl-2-dipolyhydroxystearat, PEG-30 Dipolyhydroxystearat, Cetyl Dimethicon Copolyol, Polyglyceryl-3 Diisostearat enthält.

Die O/W-Emulgatoren mit einem HLB-Wert > 10 können vorteilhaft aus der Gruppe enthaltend Lecithin, Trilaureth-4-Phosphat, Polysorbate-20, Polysorbate-60, PEG-22-Dodecylglycol Copolymer, Sucrosestearat und Sucroselaurat gewählt werden.

Als geeignete Coemulgatoren für die erfindungsgemässen O/W-Emulsionen können Fettalkohole mit 8 bis 30 Kohlenstoffatomen, Monoglycerinester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, Propylenglycolester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, sowie Sorbitanester gesättigter oder ungesättigter, verzweigter oder unverzweigter Alkancarbonsäuren mit einer Kettenlänge von 8 bis 24 Kohlenstoffatomen, insbesondere 12 bis 18 Kohlenstoffatomen, eingesetzt werden.
Besonders vorteilhafte Coemulgatoren sind Glycerylmonostearat, Glycerylmonooleat, Diglycerylmonostearat, Sorbitanmonoisostearat, Saccharosedistearat, Cetylalkohol, Stearylalkohol, Behenylalkohol, Isobehenylalkohol und Polyethylenglycol(2)stearylether (Steareth-2).

### UV- Filter

Die erfindungsgemäßen Zusammensetzungen können Sonnenschutzfilter enthalten, wobei die Gesamtmenge der Sonnenschutzfilter 0 Gew.-% bis 30 Gew.-%, vorteilhaft 0 Gew.-% bis 20 Gew.- %, besonders vorteilhaft 0 Gew.-% bis 10 Gew.-%, jeweils bezogen auf das Gesamtgewicht der erfindungsgemäßen Zusammensetzung, beträgt. Die Sonnenschutzfilter (oder UV-Filter) können unter den organischen Filtern, den physikalischen Filtern und deren Gemischen ausgewählt werden.

Die erfindungsgemäßen Zusammensetzungen können UV-A-, UV-B-Filter oder Breitbandfilter enthalten. Die eingesetzten organischen UV-Filter können öllöslich oder wasserlöslich sein. Die untere beigefügte Liste der genannten UV-Filter ist selbstverständlich nicht limitierend.

Von den UV-B-Filtern sind beispielsweise zu nennen:
(1) Salicylsäurederivate, besonders Homomenthylsalicylat, Octylsalicylat und Salicylsäure(4-isopropylbenzyl)ester;
(2) Zimtsäurederivate, insbesondere 2-Ethylhexyl-p-methoxycinnamat, das von der Firma Givaudan unter der Bezeichnung Parsol MCX^{®} erhältlich ist und 4-Methoxyzimtsäureisopentylester;
(3) flüssige β,β'-Diphenylacrylatderivate, insbesondere 2-Ethylhexyl-α,β'-diphenylacrylat oder Octocrylen, das von der Firma BASF unter der Bezeichnung UVINUL N539^{®} erhältlich ist;
(4) p-Aminobenzoesäurederivate, insbesondere 4-(dimethylamino)-benzoesäure (2-ethylhexyl)-ester, 4-(Dimethylamino)benzoesäureamylester;
(5) 3-Benzylidencampher-Derivate, insbesondere 3-(4-Methylbenzyliden)campher der von der Firma Merck unter der Bezeichnung EUSOLEX 6300^{®} im Handel ist, 3-Benzylidencampher, Benzylidencamphersulfonsäure und Polyacrylamidomethyl-Benzylidencampher;
(6) 2-Phenylbenzimidazol-5-sulfonsaeure, die unter der Bezeichnung EUSOLEX 232^{®} von Merck erhältlich ist;
(7) 1,3,5-Triazinderivate, insbesondere:
   - 2,4,6-Tris[p-(2'-ethylhexyl-1'-oxycarbonyl)anilino]-1,3,5-triazin, das von der Firma BASF unter der Bezeichnung UVINUL T150^{®} angeboten wird, und
   - Dioctylbutamidotriazon, das von der Firma Sigma 3V unter der Bezeichnung UVASORB HEB^{®} angeboten wird;
(8) Ester der Benzalmalonsäure, insbesondere 4-Methoxybenzaimalonsäuredi-(2-ethylhexyl)ester und 3-(4-(2,2-Bisethoxycarbonylvinyl)-phenoxy)propenyl)-methoxysiloxan/ dimethylsiloxan-Copolymer, das von der Firma Roche Vitamines unter der Bezeichnung Parsol^{®} SLX erhältlich ist; und
(9) die Gemische dieser Filter.

Als UV-A-Filter sind beispielsweise zu nennen:
(1) Dibenzoylmethanderivate, besonders das 4-(t-Butyl)-4'-methoxydibenzoylmethan, das von der Firma Givaudan unter der Bezeichnung PARSOL 1789^{®} angeboten wird, und 1-Phenyl-3-(4'-isopropylphenyl)propan-1,3-dion;
(2) Benzol-1,4-[di(3-methylidencampher-10-sulfonsaeure)], gegebenenfalls ganz oder teilweise neutralisiert, welches unter der Bezeichnung MEXORYL SX^{®} von Chimex im Handel ist;
(3) 2-(4'-Diethylamino-2'-hydroxybenzoyl)-benzoesäurehexylester (auch Aminobenzophenon);
(4) Silanderivate oder Polyorganosiloxane mit Benzophenongruppen;
(5) Anthranilate, besonders Menthylanthranilat, das von der Firma Symrise unter der Bezeichnung NEO HELIOPAN MA^{®} angeboten wird;
(6) Verbindungen, die pro Molekül mindestens zwei Benzoazolylgruppen oder mindestens eine Benzodiazolylgruppe enthalten, insbesondere 1,4-Bis-benzimidazolylphenylen-3,3',5,5'-tetrasulfonsaeure sowie ihre Salze, die von der Firma Symrise im Handel sind;
(7) Siliciumderivate von Benzimidazolylbenzazolen, die N-substituiert sind, oder von Benzofuranylbenzazolen, insbesondere:
   - 2-[1-[3-[1,3,3,3-Tetramethyl-I-[(trimethylsilyl)oxy]disiloxanyl]-propyl]-1H-benzimidazol-2-yl]-benzoxazol;
   - 2-[1-[3-[1,3,3,3-Tetramethyl-I-[(trimethylsilyl)oxy]disiloxanyl]-propyl]-1H-benzimidazol-2-yl]-benzothiazol;
   - 2-[1-(3-Trimethylsilanylpropyl)-1H-benzimidazol-2-yl]-benzoxazol;
   - 6-Methoxy-1,1'-bis(3-trimethylsilanylpropyl)1H,1'H-[2,2']dibenzimidazolylbenzoxazol;
   - 2-[1-(3-Trimethylsilanylpropyl)-1H-benzimidazol-2-yl]-benzothiazol; die in der Patentanmeldung EP-A-1 028 120 beschrieben sind;
(8) Triazinderivate, insbesondere 2,4-bis-[5-1 (Dimethylpropyl)benzoxazol-2-yl-(4-phenyl)-imino]-6-(2-ethylhexyl)-imino-1,3,5-triazin, das von der Firma 3V unter der Bezeichnung Uvasorb^{®}K2A angeboten wird; und
(9) deren Gemische.

Als Breitbandfilter sind beispielweise zu nennen :
(1) Benzophenonderivate, beispielsweise
   - 2,4-Dihydroxybenzophenon (Benzophenon-1);
   - 2,2',4,4'-Tetrahydroxybenzophenon (Benzophenon-2);
   - 2-Hydroxy-4-methoxybenzophenon (Benzophenon-3), welches von der Firma BASF unter der Bezeichnung UNIVNUL M40^{®} erhältlich;
   - 2-Hydroxy-4-methoxybenzophenon-5-sulfonsäure (Benzophenon-4), sowie ihre Sulfonatform (Benzonphenon-5), welche von der Firma BASF unter der Bezeichnung UVINUL MS40^{®} erhältlich sind;
   - 2,2'-Dihydroxy-4,4'-dimethoxybenzophenon (Benzophenon-6);
   - 5-Chlor-2-hydroxybenzophenon (Benzophenon-7);
   - 2,2'-Dihydroxy-4-methoxybenzophenon (Benzophenon-8);
   - das Dinatriumsalz von 2,2'-Dihydroxy-4,4'-dimethoxybenzophenon-5,5'-disulfonsäure (Benzophenon-9);
   - 2-Hydroxy-4-methoxy-4'-methylbenzophenon (Benzophenon-10);
   - Bis-(2,4-dihydroxyphenyl)methanon (Benzophenon-11); und
   - 2-Hydroxy-4-(octyloxy)-benzophenon (Benzophenon-12).
(2) Triazinderivate, insbesondere das 2,4-Bis{[4-2-ethylhexyloxy)-2-hydroxy]-phenyl}-6-(4-methoxyphenyl)-1,3,5-triazin, das von der Firma Ciba Geigy unter der Bezeichnung TINOSORB S^{®} angeboten wird, und das 2,2'-Methylen-bis[6-(2H-benzotriazol-2-yl)4-(1,1,3,3-tetramethylbutyl)phenol], das von der Firma Ciba Geigy unter der Bezeichnung TINOSORB M^{®} erhältlich ist; und
(3) 2-(1H-benzotriazol-2-yl)-4-methyl-6-[2-methyl-3-[1,3,3,3-tetramethyl-1-[(trimethylsilyl)oxy]disiloxanyl]propyl]-phenol mit der INCI-Bezeichnung Dromotrizole Trisiloxane.

Es können auch ein Gemisch von mehreren Filtern und ein Gemisch von UV-B-Filtern, UV-A-Filtern und Breitbandfilter sowie Gemische mit physikalischen Filtern verwendet werden.

Von den physikalischen Filtern können die Sulfat des Bariums, Oxide von Titan (Titandioxid, amorph oder kristallin in Form von Rutil und/oder Anatas), von Zink, von Eisen, von Zirconium, von Cer, Silicium, Mangan oder deren Gemische angegeben werden. Die Metalloxide können in Partikelform mit einer Grösse im Mikrometerbereich oder Nanometerbereich (Nanopigmente) vorliegen.

Die mittleren Partikelgrössen betragen für die Nanopigmente beispielsweise 5 bis 100 nm.

### Farbstoffe

Gegebenenfalls enthalten die erfindungsgemäßen Zusammensetzungen einen Farbstoff, der aus der Gruppe von lipophilen Farbstoffen, hydrophilen Farbstoffen, Pigmenten und Perlmutt ausgewählt wird. Erfindungsgemäß besonders vorteilhaft ist die Konzentration von Farbstoffen 0 bis 40 Gewichts-%, besonders vorteilhaft 0 bis 30 Gewichts-%, ganz besonders vorteilhaft von 0 bis 25 Gewichts-%, jeweils bezogen auf das Gesamtgewicht der Zusammensetzung.
Beispielweise können die lipophilen Farbstoffe Sudan I (Gelb), Sudan II (Orange), Sudan III (Rot), Sudan IV (Scharlachrot), DC Red 17, DC Green 6, β-Carotin, Sojaöl, DC Yellow 11, DC Violet 2, DC Orange 5 und DC Yellow 10.

Die Pigmente können anorganische oder organische Pigmente sein, die in kosmetische oder dermatologische Zusammensetzung verwendet werden können. Die erfindungsgemäßen verwendeten Pigmente können weiß oder farbig sein, umgehüllt mit einem hydrophoben Behandlungsmittel oder nicht sein.

Vorteilhaft werden die Pigmente gewählt aus der Gruppe der Metalloxide, wie die Oxide von Eisen (insbesondere die Oxide von gelber, roter, brauner, schwarzer Farbe), Titandioxid, Zinkoxid, Ceroxid, Zirconiumoxid, Chromoxid; Manganviolett, Ultramarinblau, Preussisch Blau, Ultramarin und Eisenblau, Bismutoxidchlorid, Perlmutt, mit Titan oder Bismutoxidchlorid ueberzogene Glimmer-Pigmente, farbige Perlglanzpigmente, beispielsweise Titan-Glimmer-Pigmente mit Eisenoxiden, Titan-Glimmer-Pigmente insbesondere mit Eisenblau oder Chromoxid, Titan-Glimmer-Pigmente mit einem organischen Pigment vom vorgenannten Typ, sowie Perlglanzpigmente auf der Basis von Bismutoxidchlorid, Russ, die Pigmente vom Typ D & C und die Lacke auf der Basis von Cochenillerot, Barium, Strontium, Calcium und Aluminium und deren Gemische.

Besonders vorteilhaft werden die Pigmente von Eisenoxiden oder Titandioxid verwendet.

Für eine bessere Benetzbarkeit der Pigmente durch die Öle der Fettphase kann die Oberfläche der Pigmente mit einem hydrophoben Behandlungsmittel behandelt werden. Das hydrophobe Behandlungsmittel wird ausgewählt aus der Gruppe der Siliconen, wie Methiconen, Dimethiconen, Perfluoralkylsilanen; Fettsäuren wie Stearinsäure; Metallseifen, wie Aluminiumdimyristat, dem Aluminiumsalz von hydriertem Talgglutamat, Perfluoralkylphosphaten, Perfluoralkylsilanen, Perfluoralkylsilazanen, Hexafluorpropylenpolyoxiden, Polyorganosiloxanen, die Perfluoralkylperfluorpolyethergruppen enthalten, Aminosäuren; N-acylierten Aminosäuren oder deren Salzen; Lecithin, Isopropyltriisostearyltitanat und deren Gemischen ausgewählt sein. Die N-acylierten Aminosaeuren können eine Acylgruppe mit 8 bis 22 Kohlenstoffatomen enthalten, beispielsweise 2-Ethylhexanoyl, Caproyl, Lauroyl, Myristoyl, Palmitoyl, Stearoyl oder Cocoyl. Die Salze dieser Verbindungen können Aluminiumsalze, Magnesiumsalze, Calciumsalze, Zirkoniumsalze, Zinnsalze, Natriumsalze oder Kaliumsalze sein. Bei der Aminosäure kann es sich beispielsweise um Lysin, Glutaminsäure oder Alanin handeln.

### Konditionierungsmittel

Gegebenfalls enthalten die erfindungsgemäßen Zusammensetzungen eine Konditionierungsmittel. Erfindungsgemäß bevorzugte Konditionierungsmittel sind beispielsweise alle Verbindungen, welche im International Cosmetic Ingredient Dictionary and Handbook (Volume 4, Herausgeber: R. C. Pepe, J.A. Wenninger, G. N. McEwen, The Cosmetic, Toiletry, and Fragrance Association, 9. Auflage, 2002) unter Section 4 unter den Stichworten Hair Conditioning Agents, Humectants, SkinConditioning Agents, SkinConditioning Agents-Emollient, Skin-Conditioning Agents-Humectant, SkinConditioning Agents-Miscellaneous, Skin-Conditioning Agents-Occlusive und Skin Protectans aufgeführt sind sowie alle in der EP-A 934 956 (S.11-13) unter "water soluble conditioning agent" und "oil soluble conditioning agent" aufgeführten Verbindungen.

Besondere vorteilhafte Konditionierungsstoffe stellen beispielsweise die nach INCl als Polyquaternium bezeichneten Verbindungen dar (insbesondere Polyquaternium-1 bis Polyquaternium-56).

Zu den geeigneten Konditionierungsmitteln zählen beispielsweise auch polymere quaternäre Ammoniumverbindungen, kationische Cellulosederivate, Chitosanderivate Guar Gum Derivate und Polysaccharide, insbesondere Guar Hydroxypropylammoniumchlorid (z.B. Jaguar^{®} Excel, Jaguar^{®}162 der Firma Rhodia).

Weitere erfindungsgemäß vorteilhafte Konditionierungsmittel stellen nichtionische Poly-N-vinylpyrrolidon/Polyvinylacetat-Copolymere (z.B. Luviskol^{®}VA 64 der Firma BASF AG), anionische Acrylat-Copolymere (z.B. Luviflex^{®}Soft der Firma BASF AG), und/oder amphotere Amid/Acrylat/Methacrylat Copolymere (z.B. Amphomer^{®} der Firma National Starch dar. Weitere mögliche Konditioniermittel sind quatemisierte Silikone.

### Tenside

Die erfindungsgemäßen Zusammensetzungen können auch Tenside enthalten, die aus der Gruppe von anionischen, kationischen, nichtionischen und/oder amphoteren Tensiden ausgewählt werden.

Vorteilhafte anionische Tenside im Sinne der vorliegenden Erfindung sind:
- Acylaminosäuren und deren Salze, wie Acylglutamate, insbesondere Natriumacylglutamat und Sarcosinate, beispielsweise Myristoyl Sarcosin, TEA-Lauroyl Sarcosinat, Natriumlauroylsarcosinat und Natriumcocoylsarkosinat;
- Sulfonsäuren und deren Salze, wie Acylisethionate, beispielsweise Natriumoder Ammoniumcocoylisethionat, Sulfosuccinate, beispielsweise Dioctylnatriumsulfosuccinat, Dinatriumlaurethsulfosuccinat, Dinatriumlaurylsulfosuccinat und Dinatriumundecylenamido MEA- Sulfosuccinat, Dinatrium PEG-5 Laurylcitratsulfosuccinat und Derivate;
- Schwefelsäureester, wie Alkylethersulfat, beispielsweise Natrium-, Ammonium-, Magnesium-, MIPA-, TIPALaurethsulfat, Natriummyrethsulfat und Natrium C12-13 Parethsulfat, und Alkylsulfate, beispielsweise Natrium-, Ammoniumund TEA-Laurylsulfat;
- Taurate, beispielsweise Natriumlauroyltaurat und Natriummethylcocoyltaurat,
- Ether-Carbonsäuren, beispielsweise Natriumlaureth-13 Carboxylat und Natrium PEG-6 Cocamide Carboxylat, Natrium PEG-7-Olivenöl-Carboxylat;
- Phosphorsäureester und Salze, wie beispielsweise DEA-Oleth-10 Phosphat und Dilaureth-4 Phosphat;
- Alkylsulfonate, beispielsweise Natriumcocosmonoglyceridsulfat, Natrium C12 bis C14- Olefinsulfonat, Natriumlaurylsulfoacetat und Magnesium PEG-3 Cocamidsulfat;
- Acylgiutamate wie Di-TEA-palmitoylaspartat und Natrium Caprylic/Capric Glutamat,
- Acylpeptide, beispielsweise Palmitoyl hydrolysiertes Milchprotein, Natrium Cocoyl hydrolysiertes Sojaprotein und NatriunlKalium Cocoyl hydrolysiertes Kollagen;
- Carbonsäuren und Derivate, wie beispielsweise Laurinsäure, Aluminiumstearat, Magnesiumalkanolat und Zinkundecylenat, Ester-Carbonsäuren, beispielsweise Calciumstearoyilactylat, Laureth-6 Citrat und Natrium PEG-4 Lauramidcarboxylat; und
- Alkylarylsulfonate.

Vorteilhafte kationische Tenside im Sinne der vorliegenden Erfindung sind quatemäre Tenside. Quaternäre Tenside enthalten mindestens ein N-Atom, das mit 4 Alkyloder Arylgruppen kovalent verbunden ist. Vorteilhaft sind beispielsweise Alkylbetain, Alkylamidopropylbetain und Alkylamidopropylhydroxysultain.
Weitere vorteilhafte kationische Tenside im Sinne der vorliegenden Erfindung sind ferner Alkylamine, Alkylimidazole und ethoxylierte Amine und insbesondere deren Salze.

Vorteilhafte amphotere Tenside im Sinne der vorliegenden Erfindung sind Acyl-/dialkylethylendiamine, beispielsweise Natriumacylamphoacetat, Dinatriumacylamphodipropionat, Dinatriumalkylamphodiacetat, Natriumacylamphohydroxypropylsulfonat, Dinatriumacylamphodiacetat, Natriumacylamphopropionat, und N-KokosfettsäureamidoethylN-hydroxyethylglycinat Natriumsalze.

Weitere vorteilhafte amphotere Tenside sind N-Alkylaminosäuren, beispielsweise Aminopropylalkylglutamid, Alkylaminopropionsäure, Natriumalkylimidodipropionat und Lauroamphocarboxyglycinat.

Vorteilhafte aktive nicht-ionische Tenside im Sinne der vorliegenden Erfindung sind Alkanolamide, wie Cocamide MEA/DEA/MIPA, Ester, die durch Veresterung von Carbonsäuren mit Ethylenoxid, Glycerin, Sorbitan oder anderen Alkoholen entstehen, Ether, beispielsweise ethoxylierte Alkohole, ethoxyliertes Lanolin, ethoxylierte Polysiloxane, propoxylierte POE Ether, Alkylpolyglycoside wie Laurylglucosid, Decylglycosid und Cocoglycosid, Glycoside mit einem HLB-Wert von wenigstens 20 (z.B. Beisil^{®}SPG 128V der Firma Wacker).

Weitere vorteilhafte nicht-ionische Tenside sind Alkohole und Aminoxide, wie Cocoamidopropylamin-Oxid.

Unter den Alkylethersulfaten sind insbesondere Natriumalkylethersulfate auf Basis von zweioder dreifach ethoxyliertem Laurylund Myristylalkohol bevorzugt. Sie übertreffen die Alkylsulfate deutlich bezüglich der Unempfindlichkeit gegen Wasserhärte, der Verdickbarkeit, der Kältelöslichkeit und insbesondere der Hautund Schleimhautverträglichkeit. Laurylethersulfat weist bessere Schaumeigenschaften als Myristylethersulfat auf, ist diesem aber in der Milde unterlegen.

Alkylethercarboxylate mit mittlerem und besonders mit höherem gehören zu den mildesten Tensiden überhaupt, zeigen aber ein schlechtes Schaumund Viskositätsverhalten. Sie werden oft in Kombination mit Alkylethersulfaten und amphoteren Tensiden eingesetzt.

Sulfobemsteinsäureester (Sulfosuccinate) sind mild und gut schäumende Tenside werden aber wegen ihrer schlechten Verdickbarkeit bevorzugt nur zusammen mit anderen anionischen und amphoteren Tensiden und wegen ihrer geringen Hydrolysestabilität bevorzugt nur in neutralen bzw. gut gepufferten Produkten eingesetzt.

Amidopropylbetaine weisen eine ausgezeichnete Hautund Augenschleimhautverträglichkeit auf. In Kombination mit anionischen Tensiden lässt sich deren Milde synergistisch verbessern. Bevorzugt ist die Verwendung von Cocamidopropylbetain.
Arnphoacetate/Amphodiacetate besitzen als amphotere Tenside eine sehr gute Hautund Schleimhautverträglichkeit und können konditionierend wirken bzw. die Pflegewirkung von Zusatzstoffen erhöhen. Sie werden ähnlich wie die Betaine zur Optimierung von Alkylethersulfat-Formulierungen eingesetzt. Am meisten bevorzugt sind Natriumcocoamphoacetat und Dinatriumcocoamphodiacetat.

Alkylpolyglykoside sind mild, haben gute Universaleigenschaften, schäumen jedoch schwach. Aus diesem Grund werden sie bevorzugt in Kombinationen mit anionischen Tensiden verwendet.

### Filmbildner

Vorteilhaft werden der oder die Filmbildner gewählt aus der Gruppe der wasserlöslichen oder wasserdispergierbaren Polyurethane, der Polyharnstoffe, Silikonharze und/oder Polyester sowie der nichtionischen, anionischen, amphoteren und/oder kationischen Polymere und ihren Mischungen.

Vorteilhafte nichtionische Polymere, die in erfindungsgemäßen Zusammensetzungen alleine oder im Gemisch, vorzugsweise auch mit anionischen und/oder amphoteren und/oder zwitterionischen Polymeren enthalten sein können, sind ausgewählt aus:
- Polyalkyloxazoline;
- Vinylacetat Homo- oder Copolymerisate. Hierzu gehören beispielweise Copolymerisate aus Vinylacetat und Acrylsäureester, Copolymerisate aus Vinylacetat und Ethylen, Copolymerisate aus Vinylacetat und Maleinsäureester;
- Acrylsäureester Copolymerisate wie z. B. die Copolymerisate aus Alkyl-Acrylat und Alkyl-Methacrylat, Copolymerisate aus Alkyl-Acrylat und Urethanen;
- Copolymerisate aus Acrylnitril und nichtionischem monomer ausgewählt aus Butadien und (Meth)-Acrylat;
- Styrol Homo- und Copolymerisate. Hierzu gehören beispielweise Homopolystyrol, Copolymerisate aus Styrol und Alkyl-(Meth)Acrylat, Coplymerisate aus Styrol, Alkyl-Methacrylat und Alkyl-Acrylat, Copolymerisate aus Styrol und Butadien, Copolymerisate aus Styrol, Butadien und Vinylpyridin;
- Polyamide;
- Vinyllactame Homo- oder Copolymere, wie Vinylpyrrolidon-Homo- oder Copolymerisate; hierzu gehören beispielweise Polyvinylpyrrolidon, Polyvinylcaprolactam, Copolymerisate aus N-Vinylpyrrolidon und Vinylacetat und/oder Vinylpropionat in verschiedenen Konzentrationsverhältnissen, Polyvinylcaprolactam, Polyvinylamide und deren Salze sowie Copolymere aus Vinylpyrrolidon und Dimethylaminoethyl-methacrylat, Terpolymere aus Vinylcaprolactam, Vinylpyrrolidon und Dimethylaminoethylmethacrylat;
- Polysiloxane;
- Homopolymere des N-Vinylformamids z. B. PVF von National Starch.

Besondere bevorzugte nichtionische Polymere sind Acrylsäureester Copolymerisate, Homopolymere d und Copolymere es Vinylpyrrolidons und Polyvinylcaprolactam.
Ganz besondere bevorzugte nichtionische Polymere sind Homopolymere des Vinylpyrrolidons z. B. Luviskol® K von der Firma BASF, Copolymerisate aus Vinylpyrrolidon und Vinylacetat z. B. Luviskol® VA Typen der Firma BASF oder PVPVA® S630L der Firma ISP, Terpolymere aus Vinylpyrrolidon, Vinylacetat und Propionat wie z. B. Luviskol® VAP von BASF und Polyvinylcaprolactame z.B. Luviskol® PLUS der Firma BASF.

Vorteilhafte anionische Polymere sind Homo-oder Copolymere mit Säuregruppen enthaltenden Monomereinheiten, welche gegebenenfalls mit Comonomeren, die keine Säuregruppen enthalten copolymerisiert sind. Geeignete Monomere sind ungesättigte, radikalisch polymerisierbare-Verbindungen, welche mindestens eine Säuregruppe besitzen, insbesondere Carbonsäure, Sulfonsäure oder Phosphonsäure.

Vorteilhafte anionische Polymere enthaltend Carbonsäuregruppe sind:
- Acrylsäure oder Methacrylsäure Homo- oder Copolymer oder die Salze davon. Hierzu gehören beispielweise die Copolymerisate aus Acrylsäure und Acrylamide und/oder deren Natriumsalze, Copolymerisate aus Acrylsäure und/oder Methacrylsäure und einem ungesättigten Monomer ausgewählt aus Ethylen, Styrol, Vinylester, Acrylsäureester, Methacrylsäureester, gegebenenfalls ethoxylierten Verbindungen, Copolymerisate aus Vinylpyrrolidone, Acrylsäure und C1-C20 Alkyl Methacrylate z.B. Acrylidone^{®} LM der Firma ISP, Copolymerisate aus Methacrylsäure, Ethylacrylate und Tert-butyl Acrylate z.B. Luvimer^{®} 100 P der Firma BASF;
- Crotonsäurederivat-Homo- oder Copolymer oder die Salze davon. Hierzu gehören beispielweise VinylacetatlCrotonsäure-, Vinylacetat/Acrylat- und/oder Vinylacetat/Vinyineodecanoat/Crotonsäure-Copolymere und Natriumacrylat/Vinylalkohol-Copolymere;
- ungesättigte C4-C8 Carbonsäurederivate oder Carbonsäureanhydrid Copolymer ausgewählt aus Copolymerisate aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus Vinylester, Vinylether, Vinylhalogenderivate, Phenylvinylderivate, Acrylsäure, Acrylsäureester oder Copolymerisate aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus Allylester, Methallylester und ggf. Acrylamide, Methacrylamide, alpha-Olefin, Acrylsäureester, Methacrylsäureester, Vinylpyrrolidone. Weitere bevorzugte Polymere sind Methylvinylether/Maleinsäure-Copolymere, die durch Hydrolyse von Vinylether/Maleinsäureanhydrid-Copolymeren entstehen. Diese Polymere können auch teilverestert (Ethyl, Isopropyl- bzw. Butylester) oder teilamidiert sein.
- in Wasser lösliche oder dispergierbare anionische Polyurethane, z. B. Luviset^{®} PUR von BASF, die von den erfindungsgemäßen Polyurethanen verschieden sind,
wobei diese Aufstellung selbstverständlich nicht limitierend sein soll.

Vorteilhafte anionische Polymere enthaltend Sulfonsäuregruppe sind Salze von Polyvinylsulfonsäure, Salze von Polystyrolsulfonsäure wie z. B. Natriumpolystyrolsulfonat oder Salze von Polyacrylamidesulfonsäure.

Besonders vorteilhafte anionische Polymere sind Acrylsäure Copolymere, Crotonsäurederivat-Copolymer, Copolymerisate aus Maleinsäure bzw. Maleinsäureanhydrid oder Fumarsäure bzw. Fumarsäureanhydrid oder Itaconsäure bzw. Itaconsäureanhydrid und mindestens einem Monomer ausgewählt aus Vinylester, Vinylether, Vinylhalogenderivate, Phenylvinylderivate, Acrylsäure, Acrylsäureester und Salze von Polystyrolsulfonsäure.

Ganz besondere vorteilhafte anionische Polymere sind Acrylat-Copolymere, z.B. Luvimer von BASF, Ethylacrylatt/N-tert.-Butylacrylamid/Acrylsäure-Copolymere ULTRAHOLD® STRONG der Firma BASF, VA/Crotonat/Vinylneodecanoat-Copolymer z. B. Resyn 28-2930 von National Starch, Copolymerisate wie. Z. Copolymerisate aus Methylvinylether und Maleinsäureanhydrid teilverestert z.B. GANTREZ® der Firma ISP und Natrium-Polystyrol-Sulfonate z. B. Flexan 130 von National Starch.

Vorteilhafte amphotere Polymere können unter den Polymeren ausgewählt werden, die statistisch in der Polymerkette verteilte Einheiten A und B enthalten, wobei A eine Einheit bedeutet, die von einem Monomer mit mindestens einem basischen Stickstoffatom abgeleitet ist, und B eine Einheit ist, die von einem sauren Monomer stammt, das eine oder mehrere Carboxygruppen oder Sulfonsäuregruppen aufweist, oder A und B können Gruppen bedeuten, die von zwitterionischen Carboxybetainmonomeren oder Sulfobetainmonomeren abgeleitet sind; A und B können auch eine kationische Polymerkette bedeuten, die primäre, sekundäre, tertiäre oder quartanäre Gruppen enthält,
worin mindestens eine Aminogruppe eine Carboxygruppe oder Sulfonsäuregruppe trägt, die über eine Kohlenwasserstoffgruppe gebunden ist, oder B und C sind Teil einer Polymerkette mit Ethylenα,β-dicarbonsäureeinheit, bei der die Carbonsäuregruppen mit einem Polyamin umgesetzt wurden, das eine oder mehrere primäre oder sekundäre Aminogruppen enthält.

Besondere vorteilhafte amphotore Polymere sind:
- Polymere, die bei der Copolymerisation eines von einer Vinylverbindung mit Carboxygruppe abgeleiteten Monomers, wie insbesondere Acrylsäure, Methacrylsäure, Maleinsäure, α-Chloracrylsäure, und einem basischen Monomer gebildet werden, das von einer Vinylverbindung abgeleitet ist, die substituiert ist und mindestens ein basisches Atom enthält, wie insbesondere Dialkylaminoalkylmethacrylat und -acrylat, Dialkylaminoalkylmethacrylamid und -acrylamid. Solche Verbindungen sind in dem amerikanischen Patent Nr. 3 836 537 beschrieben worden.
- Polymere mit Einheiten, die abgeleitet sind von: a) mindestens einem Monomer, das unter den Acrylamiden oder Methacrylamiden ausgewählt ist, die am Stickstoffatom mit einer Alkylgruppe substituiert sind, b) mindestens einem sauren Comonomer, das eine oder mehrere reaktive Carboxygruppen enthält, und c) mindestens einem basischen Comonomer, wie Estern von Acrylsäure und Methacrylsäure mit primären, sekundären, tertiären und quartären Aminosubstituenten und dem Quaternisierungsprodukt von Dimethylaminoethylmethacrylat mit Dimethylsulfat oder Diethylsulfat.
   Erfindungsgemäß besonders bevorzugte N-substituierte Acrylamide oder Methacrylamide sind Verbindungen, deren Alkylgruppen 2 bis 12 Kohlenstoffatome enthalten, besonders N-Ethylacrylamid, N-t-Butylacrylamid, N-t-Octylacrylamid, N-Octylacrylamid, N-Decylacrylamid, N-Dodecylacrylamid sowie die entsprechenden Methacrylamide.
   Die sauren Comonomere sind insbesondere unter Acrylsäure, Methacrylsäure, Crotonsäure, Itaconsäure, Maleinsäure, Fumarsäure sowie den Alkylmonoestern mit 1 bis 4 Kohlenstoffatomen von Maleinsäure, Maleinsäureanhydrid, Fumarsäure oder Fumarsäureanhydrid ausgewählt.
   Bevorzugte basische Comonomere sind Aminoethylmethacrylat, Butylaminoethylmethacrylat, N,N-Dimethylaminoethylmethacrylat, N-t-Butylaminoethylmethacrylat.
- Vernetzte und ganz oder teilweise acylierte Polyaminoamide, die von Polyaminoamiden der folgenden allgemeinen Formel abgeleitet sind:

   -[CO-R-CO-Z]-

   worin R eine zweiwertige Gruppe bedeutet, die von einer gesättigten Dicarbonsäure, einer aliphatischen Mono- oder Dicarbonsäure mit ethylenischer Doppelbindung, einem Ester dieser Säuren mit einem niederen Alkanol mit 1 bis 6 Kohlenstoffatomen oder einer Gruppe abgeleitet ist, die bei der Addition einer dieser Säuren an ein bisprimäres oder bissekundäres Amin entsteht, und Z eine Gruppe bedeutet, die von einem bis-primären, mono- oder bis-sekundären Polyalkylenpolyamin abgeleitet ist, und vorzugsweise: a) in Mengenanteilen von 60 bis 100 Mol-% die Gruppen -NH-[(CH₂)ₓ-NH-]ₚ- mit x = 2 und p = 2 oder 3 oder x = 3 und p = 2, wobei diese Gruppe, von Diethylentriamin, Triethylentetramin oder Dipropylentriamin abgeleitet ist; b) in Mengenanteilen von 0 bis 40 Mol-% die Gruppe -NH-[(CH₂)ₓ-NH-]ₚ-, worin x = 2 und p = 1, die von Ethylendiamin abgeleitet ist, oder die Gruppe, die von Piperazin stammt: 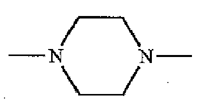 c) in Mengenanteilen von 0 bis 20 Mol-%, die Gruppe -H-(CH₂)₆-NH-, die von Hexamethylendiamin abgeleitet ist, wobei diese Polyaminoamide durch Addition eines bifunktionellen Vernetzungsmittels, das unter den Epihalohydrinen, Diepoxiden, Dianhydriden und bisungesättigten Derivaten ausgewählt ist, in einer Menge von 0,025 bis 0,35 mol Vernetzungsmittel pro Aminogruppe des Polyaminoamids vernetzt und mit Acrylsäure, Chloressigsäure oder einem Alkansulfon oder deren Salzen acyliert ist.
   Die gesättigten Carbonsäuren sind vorzugsweise unter den Säuren mit 6 bis 10 Kohlenstoffatomen ausgewählt, wie Adipinsäure, 2,2,4-Trimethyladipinsäure und 2,4,4,-Trimethyfadipinsäure, Terephthalsäure; Säuren mit ethylenischer Doppelbindung, wie beispielsweise Acrylsäure, Methacrylsäure und Itaconsäure.
   Die bei der Acylierung verwendeten Alkansultone sind vorzugsweise Propansulton oder Butansulton, die Salze der Acylierungsmittel sind vorzugsweise die Natriumsalze oder Kaliumsalze.

- Polymeren mit zwitterionischen Einheiten der folgenden Formel: 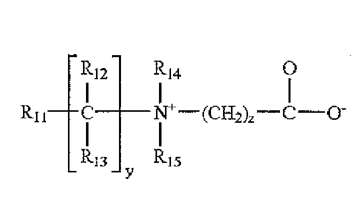 worin R₁₁ eine polymerisierbare ungesättigte Gruppe bedeutet, wie Acrylat, Methacrylat, Acrylamid oder Methacrylamid, y und z ganze Zahlen von 1 bis 3 bedeuten, R₁₂ und R₁₃ ein Wasserstoffatom, Methyl, Ethyl oder Propyl bedeuten, R₁₄ und R₁₅ ein Wasserstoffatom oder eine Alkylgruppe bedeuten, die so gewählt ist, dass die Summe der Kohlenstoffatome R₁₄ und R₁₅ 10 nicht übersteigt.
   Polymere, die solche Einheiten enthalten, können auch Einheiten aufweisen, die von nicht zwitterionischen Monomeren stammen, wie Dimethyl- und Diethylaminoethylacrylat oder Dimethyl- und Diethylaminoethylmethacrylat oder Alkylacrylate oder Alkylmethacrylate, Acrylamide oder Methacrylamide oder Vinylacetat.
- Polymere, die von Chitosan abgeleitet sind und Monomereinheiten enthalten, die den folgenden Formeln entsprechen: 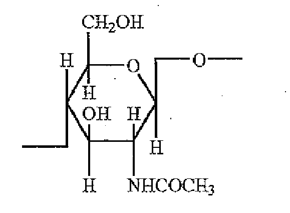 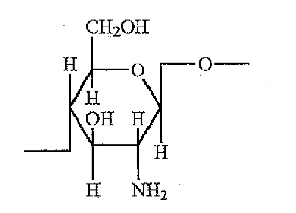 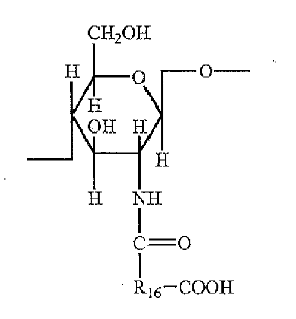 wobei die erste Einheit in Mengenanteilen von 0 bis 30 %, die zweite Einheit in Mengenanteilen von 5 bis 50 % und die dritte Einheit in Mengenanteilen von 30 bis 90 % enthalten ist, mit der Maßgabe, dass in der dritten Einheit R₁₆ eine Gruppe der folgenden Formel bedeutet: 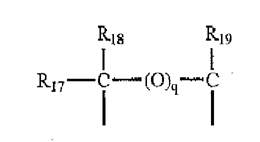 worin bedeuten: falls q = 0, die Gruppen R₁₇, R₁₈ und R₁₉, die gleich oder verschieden sind, jeweils ein Wasserstoffatom, Methyl, Hydroxy, Acetoxy oder Amino, einen Monoalkylaminrest oder einen Dialkylaminrest, die gegebenenfalls durch ein oder mehrere Stickstoffatome unterbrochen und/oder gegebenenfalls mit einer oder mehreren Gruppen Amino, Hydroxy, Carboxy, Alkylthio, Sulfonsäure, Alkylthio, dessen Alkylgruppe einen Aminorest trägt, wobei mindestens eine der Gruppen R₁₇, R₁₈ und R₁₉ in diesem Fall ein Wasserstoffatom bedeutet; oder falls q = 1, die Gruppen R₁₇, R₁₈ und R₁₉ jeweils ein Wasserstoffatom, sowie die Salze, die diese Verbindungen mit Basen oder Säuren bildern.
- Polymere, die der folgenden allgemeinen Formel entsprechen und die beispielsweise in dem französischen Patent 1 400 366 beschrieben sind: 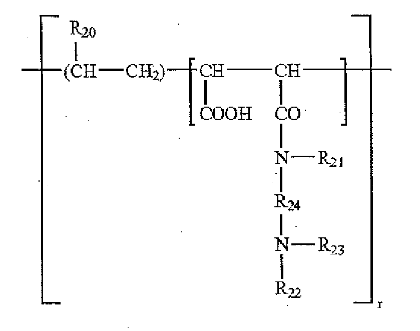 worin R₂₀ ein Wasserstoffatom, CH₃O, CH₃CH₂O oder Phenyl bedeutet, R₂₁ ein Wasserstoffatom oder eine niedere Alkylgruppe, wie Methyl oder Ethyl ist, R₂₂ ein Wasserstoffatom oder eine niedere C₁₋₆-Alkylgruppe bedeutet, wie Methyl oder Ethyl, R₂₃ eine niedere C₁₋₆-Alkylgruppe ist, wie Methyl oder Ethyl oder eine Gruppe der Formel: -R₂₄-N(R₂₂)₂, wobei R₂₄ eine Gruppe -CH₂-CH₂, -CH₂-CH₂-CH₂- oder -CH₂-CH(CH₃)- bedeutet und wobei R₂₂ die oben angegebenen Bedeutungen aufweist.
- Polymere, die bei der N-Carboxyalkylierung von Chitosan gebildet werden können, wie N-Carboxymethylchitosan oder N-Carboxybutylchitosan.
- Amphotere Polymere vom Typ -D-X-D-X, die ausgewählt sind unter:
   a) Polymere, die durch Einwirkung von Chloressigsäure oder Natriumchloracetat auf Verbindungen mit mindestens einer Einheit der folgenden Formel gebildet werden:
      worin D die Gruppe bedeutet 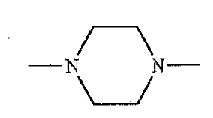 und X die Symbole E oder E' bedeutet, wobei E oder E', die gleich oder verschieden sind, eine zweiwertige Gruppe bedeuten, bei der es sich um eine geradkettige oder verzweigte Alkylengruppe mit bis zu 7 Kohlenstoffatomen in der Hauptkette handelt, die unsubstituiert vorliegt oder mit Hydroxygruppen substituiert ist und ein oder mehrere Sauerstoffatome, Stickstoffatome oder Schwefelatome und 1 bis 3 aromatische und/oder heterocyclische Ringe enthalten kann; wobei die Sauerstoffatome, Stickstoffatome und Schwefelatome in Form der folgenden Gruppen vorliegen: Ether, Thioether, Sulfoxid, Sulfon, Sulfonium, Alkylamin, Alkenylamin, Hydroxy, Benzylamin, Aminoxid, quartäres Ammonium, Amid, Imid, Alkohol, Ester und/oder Urethan.
   b) Polymere der Formel worin D die Gruppe bedeutet 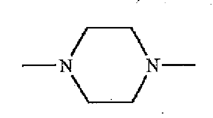 und X das Symbol E oder E' und mindestens einmal E' bedeutet; wobei E die oben angegebenen Bedeutungen aufweist und E' eine zweiwertige Gruppe ist, bei der es sich um eine geradkettige oder verzweigte Alkylengruppe mit bis zu 7 Kohlenstoffatomen in der Hauptkette handelt, die unsubstituiert vorliegt oder mit einer oder mehreren Hydroxygruppen substituiert ist und ein oder mehrere Stickstoffatome enthält, wobei das Stickstoffatom mit einer Alkylgruppe substituiert ist, die gegebenenfalls durch ein Sauerstoffatom unterbrochen ist und zwingend eine oder mehrere Carboxyfunktionen oder eine oder mehrere Hydroxyfunktionen enthält und durch Umsetzung mit Chloressigsäure oder Natriumchloracetat betainisiert ist.
- Alkyl(C₁₋₅)vinylether/Maleinsäureanhydrid-Copolymere, die teilweise durch Semiamidierung mit einem N,N-Dialkylaminoalkylamin wie N,N-Dimethylaminopropylamin oder einem N,N-Dialkylaminoalkohol teilweise modifiziert sind. Diese Polymere können auch weitere Comonomere enthalten, wie Vinylcaprolactam.

Ganz besondere vorteilhafte amphotere Polymere sind z.B. die Copolymere Octylacrylamid/Acrylate/Butylamino-Ethylmethacrylat-Copolymer, die unter den Bezeichnungen AMPHOMER^{®}, AMPHOMER^{®} LV 71 oder BALANCE^{®} 47 der Firma NATIONAL STARCH im Handel sind, und Methylmethacrylat/Methyl-dimethylcarboxymethylammoniumethylmethacrylat-Copolymere.

### Lösungsmittel

Das kosmetische akzeptable Medium der erfindungsgemäßen Zusammensetzungen kann Wasser und gegebenenfalls ein kosmetisch im Wasser mischbares geeignetes organisches Lösungsmittel sein.

Das verwendete Wasser in den erfindungsgemäßen Zusammensetzungen kann ein Blütenwasser, reines demineralisiertes Wasser, Mineralwasser, Thermalwasser und/oder Meerwasser sein. Die bevorzugten Lösungsmittel sind beispielsweise die aliphatischen Alkohole mit C1-4 Kohlenstoffatomen wie Ethanol und Isopropanol; Polyol und deren Derivate wie Propylenglykol, Dipropylenglykol, Butylen-1,3-glykol, Polypropylenglykol, Glykolether wie Alkyl (C1-4)ether von Mono-, Di- oder Tripropylenglykol oder Mono-; Di- oder Triethylenglykol, und deren-Gemischen ausgewählt.

### Treibgase

Gegebenfalls enthalten die erfindungsgemäßen Treibgase. Die erfindungsgemäßen bevorzugten Treibgase sind Kohlenwasserstoffe wie Propan, Isobutan und n-Butan sowie deren Mischungen. Aber auch Druckluft, Kohlendioxid, Stickstoff, Stickstoffdioxid und Dimethylether sowie Mischungen all dieser Gase sind erfindungsgemäß vorteilhaft zu verwenden.

Natürlich weiß der Fachmann, dass es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW) wie z.B. 1,2-difluoroethan (Treibmittel 152 A).

### Wirkstoffe

Die erfindungsgemäßen kosmetischen Zusammensetzungen enthalten zweckmäßig einen oder mehrere kosmetisch wirksame, gegebenenfalls auch pharmazeutisch wirksamen Inhaltsstoffe. Beispiele kosmetisch gegebenenfalls auch therapeutisch wirksamer Inhaltsstoffe schließen ein: Antiaknemittel, antimikrobielle Mittel, Antitranspirationsmittel, astringierende Mittel, desodorierende Mittel, Konditionierungsmittel für die Haut, hautglättende Mittel, Mittel zur Steigerung der Hauthydratation wie z. B. Glycerin oder Harnstoff (so genannte Feuchhaltmittel), Keratolytika, Radikalfänger für freie Radikale, antiseptische Wirkstoffe, Wirkstoffe gegen Hautalterung und/oder Mittel, welche die Differenzierung und/oder Proliferation und/oder Pigmentierung der Haut modulieren, Vitamine wie Vitamin C, Wirkstoffe mit reizender Nebenwirkung, wie alpha-Hydroxysäuren, β-Hydroxysäuren, alpha-Ketosäuren, β-Ketosäuren, Retinoide (Retinol, Retinal, Retin-Säure) Anthralinen (Dioxyanthranol), Anthranoide, Peroxide (insbesondere Benzoylperoxid), Minoxidil, Lithiumsalze, Antimetabolite, Vitamin D und seine Derivate; Katechine, Flavonoide, Ceramide, Fettsubstanzen, synthetische Öle, Mineralöle, wie Paraffinöle oder Vaselineöle, Siliconöle, Pflanzenöle wie Kokosöl, Süßmandelö1, Aprikosenöl, Maisöl, Jojobaöl, Olivenöl, Avocadoöl, Sesamöl, Palmöl, Eukalyptusöl, Rosmarinöl, Lavendelöl, Kiefernöl, Thymianöl, Minzöl, Kardamonöl, Orangenblütenöl, Sojaöl, Kleieöl, Reisöl, Rapsöl und Rizinusöl, Weizenkeimöl und daraus isoliertes Vitamin E, Nachtkerzenöl, Pflanzenlecithine (z.B. Sojalecithin), aus Pflanzen isolierte Sphingolipide/Ceramide, tierische Öle oder Fette, wie Talg, Lanolin, Butteröl, Fettsäureester, Ester von Fettalkoholen und Wachse mit einem der Hauttemperatur entsprechenden Schmelzpunkt (tierische Wachse, wie Bienenwachs, Carnaubawachs und Candelillawachs, mineralische Wachse, wie mikrokristalline Wachse, und synthetische Wachse, wie Polyethylen- oder Silikonwachse), sowie sämtliche für kosmetische Zwecke geeigneten Öle, wie beispielsweise in der CTFA-Abhandlung, Cosmetic Ingredient Handbook, 1. Aufig., 1988, The Cosmetic, Toiletry and Fragrance Association, Inc., Washington, erwähnt, mehrfach ungesättigte Fettsäuren, essentielle Fettsäuren (z.B. gamma-Linolensäure), Enzyme, Coenzyme, Enzymhemmstoffe, hydratisierende Mittel, hautberuhigende Mittel, Detergentien oder schaumbildende Mittel, und anorganische oder synthetische mattierende Füllstoffe, mit Ausnahme der nicht vorteilhaften oben genannten nicht-verfilmenden Füllstoffe, abrasive Mittel.
Weiterhin können die erfindungsgemäßen kosmetischen Zusammensetzungen Pflanzenwirkstoffextrakte bzw. daraus gewonnene Auszüge oder Einzelstoffe erwähnt werden enthalten sein, wie solche, die ausgewählt werden aus der Gruppe bestehend aus festen Pflanzenextrakten, flüssigen Pflanzenextrakten, hydrophilen Pflanzenextrakten, lipophilen Pflanzenextrakten, einzelnen Pflanzeninhaltsstoffen; sowie deren Mischungen, wie Flavonoide und ihre Aglyka: Rutin, Quercetin, Diosmin, Hyperosid, (Neo)hesperidin, Hesperitin, Ginkgo Biloba (z.B. Ginkoflavonglykoside), Crataegus-Extrakt (z.B. oligomere Procyanidine), Buchweizen (z.B. Rutin), Sophora japonica (z.B. Rutin), Birkenblätter (z.B. Quercetinglykoside, Hyperosid und Rutin), Holunderblüten (z.B. Rutin), Lindenblüten (z.B. ätherisches Öl mit Quercetin und Farnesol), Johanniskrautöl, (z.B. Olivenölauszug), Calendula, Arnika (z.B. ölige Auszüge der Blüten mit ätherischem Öl, polare Auszüge mit Flavonoiden), Melisse (z.B. Flavone, ätherisches Öl); Immunstimulantien: Echinacea purpurea (z.B. alkoholische Auszüge, Frischpflanzensaft, Preßsaft), Eleutherokokkus senticosus; Alkaloide: Rauwolfia (z.B. Prajmalin), Immergrün (z.B.Vincamin); weitere Phytopharmaka: Aloe, Roßkastanie (z.B. Aescin), Knoblauch (z.B. Knoblauchöl), Ananas (z.B. Bromelaine), Ginseng (z.B. Ginsenoside), Mariendistelfrüchte (z.B. auf Silymarin standardisierter Extrakt), Mäusedornwurzel (z.B. Ruscogenin), Baldrian (z.B. Valepotriate, Tct. Valerianae), Kava-Kava (z.B. Kavalactone), Hopfenblüten (z.B. Hopfenbitterstoffe), Extr. Passi-florae, Enzian (z.B. ethanol. Extrakt), anthrachinonhaltige Drogenauszüge, z.B. aloinhaltiger Aloe Vera-Saft, Pollenextrakt, Algenextrakte, Süßholzwurzelextrakte, Palmenextrakt, Galphimia (z.B.Urtinktur), Mistel (z.B. wässrig-ethanol. Auszug), Phytosterole (z.B. beta-Sitosterin), Wollblumen (z.B. wäßrig-alkohol. Extrakt), Drosera (z.B. Likörweinextrakt), Sanddornfrüchte (z.B. daraus gewonnener Saft oder Sanddornöl), Eibischwurzel, Primelwurzelextrakt, Frischpflanzenextrakte aus Malve, Beinwell, Efeu, Schachtelhalm, Schafgarbe, Spitzwegerich (z.B. Preßsaft), Brennessel, Schöllkraut, Petersilie; Pflanzenextrakte aus Norolaena lobata, Tagetes lucida, Teeoma siems, Momordica charantia, und Aloe Vera Extrakte.

Bevorzugte kosmetische Wirkstoffe sind natürliche und synthetische Feuchthaltefaktoren bzw. Feuchthaltemittel, wie beispielsweise Glycerin, Polyglycerin, Sorbit, Dimethylisosorbid, Milchsäure und/oder Lactate, insbesondere Natriumlactat, Butylenglykol, Propylenglykol, Biosaccaride Gum-1, Glycine Soja, Hydroxyethylharnstoff, Ethylhexyloxyglycerin, Pyrrolidoncarbonsäure und Harnstoff, polymere Moisturizer aus der Gruppe der wasserlöslichen und/oder in Wasser quellbaren und/oder mit Hilfe von Wasser gelierbaren Polysaccharide, Hyaluronsäure, Chitosan, fucosereiche Polysaccharide, welche unter der Bezeichnung Fucogel™ 1000 von der Gesellschaft SOLABIA S.A. erhältlich sind, des weiteren Ceramide, Hautschutzmittel, Hautaufheller, Vitamine, Antioxidantien, sogenannte Antiagingmittel, antiirritative Mittel etc. Weitere bevorzugte kosmetische Wirkstoffe sind natürliche Fette und Öle, d.h. Triglyceride von natürlichen Fettsäuren, beispielsweise aufgrund ihrer rückfettenden und pflegenden Wirkung auf der Haut.

Besonders vorteilhaft im Sinne der vorliegenden Erfindung können wasserlösliche Antioxidantien eingesetzt werden, wie beispielsweise Vitamine, z.B. Ascorbinsäure und deren Derivate. Ganz besonders vorteilhafte sind Vitamin E und dessen Derivate sowie Vitamin A und dessen Derivate. Weitere vorteilhafte Wirkstoffe in der erfindungsgemäßen Zusammensetzung sind α-Hydroxysäure wie Glykolsäure, Milchsäure, Äpfelsäure, Weinsäure, Citronensäure und Mandelsäure, β-Hydroxysäure wie Salicylsäure sowie deren acylierten Derivate, die 2-Hydroxyalkansäure und ihre Derivate; natürliche Wirkstoffe und/oder deren Derivate, wie z.B. alpha-Liponsäure, Folsäure, Phytoen, D-Biotin, Coenzym Q10, alpha-Glucosylrutin, Carnitin, Carnosin, natürliche und/oder synthetische Isoflavonoide, Kreatin, Kreatinin, Taurin und/oder [beta]-Alanin sowie 8-Hexadecen-1,16-dicarbonsäure (Dioic acid, CAS-Nummer 20701-68-2; vorläufige INCI-Bezeichnung Octadecendioic acid) und/oder Licochalcon A und die Pflanzenextrakte.

Pharmazeutische bzw. therapeutische Wirkstoffe sind solche, die im Sinne des Arzneimittelgesetzes unter anderem dazu bestimmt sind, Krankheiten, Leiden, Körperschäden oder krankhafte Beschwerden zu heilen, zu lindern oder zu verhüten. Die Mittel bzw. Wirkstoffe sind für die äußere Anwendung bestimmt, wobei es sich um hautaktive Wirkstoffe aber auch um transdermale Wirkstoffe handelt kann. Sie schließen beispielsweise ein: Mittel zur Behandlung von Hautkrankheiten, wie antibakterielle Wirkstoffe, Antimykotika, antivirale Wirkstoffe, entzündungshemmende Wirkstoffe, wie Dexpanthenol, juckreizstillende Wirkstoffe, Cortison und Derivate, wie Glukokortikolde, wie Prednison, Prednisolon, Methylprednisolon, Betamethason, Dexamethason, Triamcinolon, Paramethason und Fludrocortison, Mittel zur Behandlung von Hautkrankheiten, wie der Neurodermitis, der atopischen Dermatitis etc., und Anti-Herpesmittel.

### Weitere Hilfemittel

Die erfindungsgemäßen Zusammensetzungen können zusätzlich Zusatzstoffe enthalten, die in der Kosmetik üblich sind, wie Antioxidantien, Lichtschutzmittel und/oder andere Hilfs- und Zusatzmittel wie beispielsweise Emulgatoren, grenzflächenaktive Stoffe, Entschäumer, Verdicker, Tenside, Wirkstoffe, Feuchthaltemittel, Füllstoff, UV-Filter, Filmbildner, Lösemittel, Koaleszenzmittel, Aromastoffe, Geruchsabsorber, Parfüms, Gelbildner und/oder andere Polymerdispersionen wie beispielsweise Dispersionen auf Basis von Polyacrylaten enthalten, sensorische Additive, Weichmacher, Pigmente, Puffer, Treibmittel, Verlaufsmittel und/oder Thixotropiemittel, Geschmeidigkeitsmittel, Weichmacher, Konservierungsmittel. Die Mengen der verschiedenen Zusatzstoffe sind dem Fachmann für den einzusetzenden Bereich bekannt und liegen beispielweise im Bereich von 0,0 bis 25 gew. % bezogen auf das Gesamtgewicht der Zusammensetzung.

Die erfindungsgemäßen kosmetischen Zusammensetzungen können auch sensorische Additiv enthalten. Unter sensorische Additiv sind farblose oder weiße, mineralische oder synthetische, lamellare, sphärische oder längliche inerte Partikel oder ein nicht-partikuläres sensorisches Additiv zu verstehen, welche z.B. die sensorischen Eigenschaften der Formulierungen weiter verbessern und beispielsweise ein samtiges oder seidiges Hautgefühl hinterlassen.

Die sensorischen Additive können in der erfindungsgemäßen Zusammensetzung in einer Menge von 0,1 bis 10 Gew.-%, bezogen auf das Gesamtgewicht der Zusammensetzung, und vorzugsweise 0,1 bis 7 % enthalten sein.

Vorteilhafte partikuläre sensorische additive im Sinne der vorliegenden Erfindung sind Talkum, Glimmer (Mica), Siliciumoxid, Kaolin, Stärke und deren Derivaten (beispielweise Tapiocastärke, Distärkephosphat, Aluminium- bzw. Natrium-Stärke Octenylsuccinat und dergleichen), pyrogene Kieselsäure, Pigmente, die weder hauptsächlich UV-Filter-noch färbende Wirkung haben (wie z.B. Bornitrid etc.), Bornitrid, Calciumcarbonat, Dicalciumphosphat , Magnesiumcarbonat, Magnesiumhydrogencarbonat, Hydroxyapatite, mikrokristalline Cellulose, Pulver von synthetischen Polymeren, wie Polyamide (beispielsweise den unter der Handelsbezeichnung "Nylon^{®}" erhältlichen Polymeren), Polyethylen, Poly-β-alanine, Polytetrafluorethylen ("Teflon^{®}"), Polyacrylat, Polyurethan, Lauroyl-lysine, Silikonharz (beispielsweise den unter der Handelsbezeichnung "Tospearl^{®}" der Firma Kobo Products Inc. erhältlichen Polymeren), Hohlpartikel von Polyvinyliden/Acrylonitrile (Expancel^{®} der Firma Akzo Nobel) oder Hohlpartikel von Siliciumoxid (Silica Beads^{®} der Firma MAPRECOS) Vorteilhafte nicht-partikuläre sensorische Additive können aus der Gruppe der Dimethiconole (z. B. Dow Corning 1503 Fluid der Fa. Dow Corning Ltd.), der Siliconcopolymere (z. B. Divinyldimethicone/Dimethicone Copolymer, Dow Corning HMW 2220 der Fa. Dow Corning Ltd.) oder der Siliconelastomere (z. B. Dimethicone Crosspolymer, Dow Corning 9040 Silicone Elastomer Blend der Fa. Dow Corning Ltd.) ausgewählt werden.

Gegebenfalls enthalten die erfindungsgemäßen kosmetischen Zusammensetzungen ein Konservierungsmittel. Zusammensetzungen mit hohen Wassergehalten müssen zuverlässig vor Verkeimung geschützt sein. Die wichtigsten dazu eingesetzten Konservierungsmittel sind Harnstoff-Kondensate, p-Hydroxybenzoesäureester, die Kombination von Phenoxyethanol mit Methyldibromoglutaronitril und Säurekonservierungen mit Benzoesäure, Salicylsäure und Sorbinsäure.
Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant^{®} (Lonza) kommerziell erhältlich ist), lodopropylbutylcarbamate (z.B. Glycacil-L^{®} Glycacil-S^{®} (Lonza), Dekaben^{®} LMB (Jan Dekker)), Parabene (p-Hydroxybenzoesäurealkylester, wie z.B. Methyl-, Ethyl-, Propylund/oder Butylparaben), Dehydroacetic Acid (Euxyl^{®} K 702 der Firma Schülke&Mayr), Phenoxyethanol, Ethanol, Benzoesäure. Vorteilhaft werden auch sogenannte Konservierungshelfer, wie beispielsweise Octoxyglycerin, Glycine, Soja, diol etc. eingesetzt.
Besondere vorteilhaft sind in der Kosmetik gebräuchliche Konservierungsmittel oder Konservierungshilfsstoffe wie Dibromdicyanobutan (2-Brom-2-brommethylglutarodinitril), Phenoxyethanol, 3- lod-2-propinylbutylcarbamat, 2-Brom-2-nitropropan-1 ,3-diol, Imidazolidinylhamstoff, 5-Chlor-2-methyl-4-isothiazolin-3-on, 2-Chloracetamid, Benzalkoniumchlorid, Benzylalkohol, Salicylsäure und Salicylate.

Ganz besondere vorteilhafte besonders werden die Konservierungsmittel aus der Gruppe von lodopropylbutylcarbamate, Parabene (Methyl-, Ethyl-, Propylund/oder Butylparaben) und/oder Phenoxyethanol ausgewählt.

Die vorliegende Erfindung wird an Hand von Beispielen erläutert, wobei sie nicht als einschränkend zu verstehen sind. Alle Mengenangaben, Anteile und Prozentanteile sind, soweit nicht anders angegeben, auf das Gewicht und die Gesamtmenge bzw. auf das Gesamtgewicht der Zusammensetzungen bezogen.

### Beispiele:

### Chemikalie

### Bayhydur^{®} VP LS 2336 (Bayer MateriaiScience AG, Lev., DE):

Hydrophiliertes Polyisocyanat auf Basis von Hexamethylendiisocyanat, lösemittelfrei, Viskosität ca. 6800 mPa s, Isocyanat-Gehalt ca. 16,2%, Bayer MaterialScience AG, Leverkusen, DE.

### Impranil^{®} DLN (Bayer MaterialScience AG, Lev., DE):

Anionisch hydrophilierte, nicht querverzweigte, aliphatische Polyesterpolyurethan-polyharnstoff - Dispersion in Wasser mit einem Feststoffgehalt von ca. 40 %) Bayer MaterialScience AG, Leverkusen, DE.

### Bayhydur^{®} VP LS 2240 (Bayer MaterialScience AG, Lev., DE):

Nicht-ionisch hydrophilierte, wässrige unverzweigte Polyisocyanat-Dispersion enthaltend blockierte Isocyanatgruppen, Feststoffgehalt ca. 35%ig in Wasser/MPA/Xylol (56 : 4,5 : 4,5).

### Dispercoll S 5005 (Bayer MaterialScience AG, Lev., DE):

Wässrige anionische kolloiddisperse Lösung von amorphem Siliciumdioxid, Feststoffgehalt ca. 50%ig in Wasser, pH-Wert ca. 9, mittlere Teilchengröße ca. 55 nm.

### Isofoam^{®} 16 (Petrofer-Chemie, Hildesheim, DE):

Entschäumer

Die anderen Chemikalien wurden im Feinchemikalienhandel bei Sigma-Aldrich GmbH, Taufkirchen, DE bezogen.

Sofern nicht abweichend vermerkt, beziehen sich alle Prozentangaben auf Gewichtsprozent.

Sofern nicht abweichend vermerkt, beziehen alle analytischen Messungen auf Temperaturen von 23°C.

Die angegebenen Viskositäten wurden mittels Rotationsviskosimetrie nach DIN 53019 bei 23°C mit einem Rotationsviskosimeter der Firma Anton Paar Germany GmbH, Ostfildern, DE bestimmt. NCO-Gehalte wurden, wenn nicht ausdrücklich anders erwähnt, volumetrisch gemäß DIN-EN ISO 11909 bestimmt.

Die angegebenen Partikelgröße der wässrigen Dispersionen wurden mittels Laserkorrellationsspektroskopie (Gerät: Malvern Zetasizer 1000, Malver Inst. Limited) bestimmt.

Die Partikelgrößen der erfindungsgemäßen Partikel hergestellt durch Trocknung wässriger Dispersionen wurde mittels optischer Spektroskopie bei 100-facher Vergrößerung durchgeführt. Für die Bildanalyse wurde dabei eine Bildverarbeitungssoftware (SIS GmbH, Deutschland) eingesetzt, die Analyse wurde an 20 verschiedenen Objektstellen durchgeführt.

Die Festkörpergehalte der Dispersionen wurden ermittelt durch Erhitzen einer ausgewogenen Probe auf 120°C. Bei Gewichtskonstanz wurde durch erneutes Auswiegen der Probe Festkörpergehalt berechnet. Mit der gleichen Methode wurde der Wassergehalt der erfindungsgemäßen Pulver ermittelt.

Die Kontrolle auf freie NCO-Gruppen wurde mittels IR-Spektroskopie (Bande bei 2260 cm⁻¹) durchgeführt.

### 1) Herstellung einer wässrigen Nanoharnstoff-Dispersion (A)

Zu einer Lösung von 4,1 g Triethylamin in 4952 g entionisiertem Wasser wurden bei 30°C unter heftigem Rühren 820,20 g Bayhydur^{®} VP LS 2336 und anschließend 0,32 g Isofoam^{®} 16 gegeben und weiter gerührt. Nach 3, 6 und 9 Stunden wurden jeweils weitere 820,20 g Bayhydur^{®} VP LS 2336 und je anschließend 0,32 g Isofoam^{®} 16 zugesetzt und anschließend bei 30°C weitere 4 Stunden nachgerührt. Danach wurde bei 200 mbar Vakuum und 30°C weiter 3 Stunden gerührt und die entstandene Dispersion abgefüllt.

Die erhaltene weiße wässrige Dispersion hatte nachfolgenden Eigenschaften:

| | |
|---|---|
| Partikelgröße (LKS): | 93 nm |
| Viskosität (Viskosimeter, 23°C): | < 50 mPas |
| pH (23°C): | 7,8 |

### 2) Vergleichsbeispiel:

### Trocknung einer wässrigen anionisch hydrophilierten, nicht querverzweigten, aliphatischen Polyesterpolyurethan-polyharnstoff-Dispersion

In einer Rührapparatur mit Destillationsaufsatz wurden 500 g der Dispersion Impranil^{®} DLN evakuiert auf ca. 100 mbar bei 70°C und das Wasser wird über den Destillationsaufsatz während ca. 3 Stunden abdestilliert. Es entstand eine gelartige, verfilmte Masse, die aus der Apparatur geschnitten werden musste.

### 3) Vergleichsbeispiel:

### Trocknung einer wässrigen, nicht-ionisch hydrophilierten, nicht querverzweigten, aliphatischen Polyesterpolyurethan-Dispersion

Es wurde analog Beispiel 2 vorgegangen, jedoch wurde die Dispersion Bayhydur^{®} VP LS 2240 getrocknet. Es bildete sich ein klebriger Film, kein verarbeitbares Pulver.

### 4) Erfindungsgemäßes Beispiel

### Direkte Trocknung einer wässrigen Nanoharnstoff-Dispersion durch destillatives Entfernen des Wassers

Es wurde analog Beispiel 2 vorgegangen, jedoch wurde die Nanoharnstoff-Dispersion aus Beispiel 1 getrocknet. Es bildete sich ein weißes, klumpiges Pulver.

| | |
|---|---|
| Festgehalt: | 99 % |

Durch Verreiben der getrockneten Probe in einem Möser wurde ein feines, rieselfähiges Pulver erhalten.

| | |
|---|---|
| Festgehalt: | 99 % |

### 5) Erfindungsgemäßes Beispiel

### Trocknung einer wässrigen Nanoharnstoff-Dispersion durch Gefriertrocknung

500 g der Nanoharnstoff-Dispersion aus Beispiel 1 wurden in einem 2 Liter-Rundkolben in einem Kältebad (ca. -78°C, Mischung aus Trockeneis und Isopropanol) eingefroren und an eine Gefriertrocknungsanlage angebracht. Beim anschließenden Evakuieren wurde das Wasser entfernt bis die Probe trocken war.

Es bildete sich ein nichtrieselfähige agglomerisierte Substanz.

Festgehaft: 99 %

### 6) Erfindungsgemäßes Beispiel

### Trocknung einer wässrigen Nanoharnstoff-Dispersion durch Sprühtrocknung

2000 g der Nanoharnstoff-Dispersion aus Beispiel 1 wurden über sprühgetrocknet über eine Sprütrocknungsanlage B-290 der Fa. Büchi (Flawli, Schweiz). Die Dispersion wurde über eine Düse mit einem Innendurchmesser von 0,7 mm zugeführt, als Sprühgas wurde Stickstoff im äußeren Ring der Zweistoffdüse eingesetzt. Die Zuführungstemperatur lag bei 60°C. Die Abscheidung der Partikel erfolgte über einen Zyklon und einen nachgeschalteten Feinpartikelfilter.

Es bildete sich ein weißes Pulver.

Festgehalt: 99 %

Mittlere Teilchengröße: 10 µm

Schüttdichte: 0,36 g/ml

### 7) Erfindungsgemäßes Beispiel

### Trocknung einer verdünnten, wässrigen Nanoharnstoff-Dispersion durch Sprühtrocknung

Es wurde vorgegangen wie in Beispiel 6 beschrieben, jedoch wurden vor der Trocknung zu 1000 g der Nanoharnstoff-Dispersion noch 1000 g entionisiertes Wasser gemischt.

Es bildete sich ein weißes Pulver.

Festgehalt: 99 %

Mittlere Teilchengröße: 10 µm

### 8) Erfindungsgemäßes Beispiel

### Trocknung einer wässrigen Nanoharnstoff-Dispersion nach Abmischung mit einer Nanosilica-Dispersion durch Sprühtrocknung

Es wurde vorgegangen wie in Beispiel 6 beschrieben, jedoch wurden vor der Trocknung zu 1000 g der Nanoharnstoff-Dispersion 24 g Dispercoll S 5005 gemischt.

Es bildete sich ein weißes Pulver.
Festgehalt: 99 %

### Beispielformulierungen :

### Polyharnstoff-Pulver bzw. wässrige Nanoharnstoff-Dispersionen in Kosmetik-Formulierungen

### 1. Skin Care Formulierungen

### 1.1 O/W-Emulsion

| | 1 | 2 | 3 | 4 | 5 | 6 | 7 | 8 | 9 | 10 |
|---|---|---|---|---|---|---|---|---|---|---|
| Erfindungsgemäßes Polyharnstoffpulver | 5,0 | | 8,0 | 10 | 15,0 | 2,0 | 10,0 | | 5,0 | |
| Erfindungsgemäße wässrige Nanoharnstoff-Dispersion | | 10 | | | | 2,0 | | 20 | | 5,0 |
| Glyceryl Stearate Citrate | 2,0 | | | | | | | | | |
| Glyceryl Stearate (Selbtsemulgierend) | | 2,0 | | | | | | | | |
| Polyglyceryl-3 Methylglucose Distearate | | | 2,5 | 2,0 | | | | | | |
| Sorbitan Stearate | | | | 1,0 | | | | | | |
| PEG-40 Stearate | | | | | 0,5 | | | | | |
| Glyceryl Stearate | | | | | 2,5 | 0,5 | 2,0 | | 2,0 | |
| PEG-100 Stearate | | | | | | | 2,0 | | | |
| Sodium Stearoyl Glutamate | | | | | | | | 0,5 | 0,2 | |
| Distearyldiammonium chloride | | | | | | | | | | 1,0 |
| Stearic acid | | 1,0 | | | | | | | | |
| Behenyl alcohol | | | | | | 1,0 | | | | |
| Cetyl alcohol | | | | | | | | | | 2,5 |
| Cetearyl alcohol | | 2,0 | | | 5,0 | 10,0 | 2,0 | | | |
| Myristyl alcohol | | 2,0 | | | | | | | | |
| Stearyl alcohol | 1,0 | | 1,0 | | | | | | 3,0 | 1,0 |
| Acrylates/C10-30 Alkyl Acrylate crosspolymer¹ | 0,1 | | | | | | 0,8 | | 0,3 | |
| Ammonium Acryloyldimethyltaurate / VP Copolymer² | | 0,5 | | | | | | | | |
| Acrylic Acid/VP Crosspolymer³ | | | | 0,6 | | | | 0,2 | | |
| Carbomer | | | 0,8 4 | | 0,3⁵ | | | | 0,5 6 | |
| Dimethylpolysiloxane | | | | | | | | | | |
| Dicaprylyl Ether | 1,0 | 3,0 | | | | | | | | 1,0 |
| Myristyl Myristate | 3,0 | | | | | | | 1,0 | | |
| Octyldodecanol | 1,0 | | | 4,0 | 5,0 | 3,0 | | | 4,0 | |
| Butylene Glycol Dicaprylate/Dicaprate | | 2,0 | | | 3,0 | | | | | |
| C12-15 Alkyl Benzoate | | 3,0 | | | 3,0 | | 5,0 | 1,0 | | |
| Isohexadecane | | | 2,0 | | | | 3,0 | | | |
| Caprylic/Capric Triglycerid | | | | 2,0 | | | | 2,0 | | |
| Cyclomethicone | 4,0 | | 2,0 | | | 2,0 | | | 1,0 | |
| Dimethicone | 2,0 | | 1,0 | | | 2,0 | 5,0 | | 1,0 | |
| Mineral Oil | | | | | | 5,0 | | | | 2,5 |
| Hydrietes Polyisobutene | | | | | | 2,0 | | | | |
| Phenethylbenzoate | | | | | | | | 5,0 | | |
| Isodecyl Neopentanoate | | | | | | | | | 2,0 | |
| Oenothera Biennis | | | | | | | | | | 2,0 |
| Shea Butter | | | | | | | | | | 2,0 |
| Butylenglycol | | | | 5,0 | | | | | | |
| Glycerin | 10 | 7,5 | 5,0 | | 3,0 | | 10 | 8,0 | 5,0 | 5,0 |
| Ethanol | 3,0 | | | | | | | | 4,0 | |
| Tapioca starch⁷ | 1,0 | | 1,0 | | | | | | | |
| Distarch Phosphate | | 2,0 | | | | | | | | |
| Aluminium Starch Octenylsuccinate | | | | | | | | 2,0 | | |
| Natrium starch Octenylsucccinate⁹ | | | | | 2,0 | | | | | |
| Butyl Methoxydibenzoylmethane | | | | | | | | 4 | | |
| Octocrylene | | | | | | | | 5 | | |
| Phenylbenzimidazole sulfonic acid | | | 2 | | | | | 2 | | |
| Ethylhexyl Methoxycinnamate | | | 5 | | | | | | | |
| Trisodium EDTA | | | 1 | | | | | 1 | | |
| Wirkstoffe | q.s. | q.s. | q.s. | q.s. | q.s. | q.s. | q.s. | q.s. | q.s. | q.s. |
| Neutralisationsmittel | q.s. | q.s. | q.s. | q.s. | q.s. | q.s. | q.s. | q.s. | q.s. | q.s. |
| Farbstoffe | q.s. | q.s. | q.s. | q.s. | q.s. | q.s. | q.s. | q.s. | q.s. | q.s. |
| Parfüm | q.s. | q.s. | q.s. | q.s. | q.s. | q.s. | q.s. | q.s. | q.s. | q.s. |
| Konservierungsmittel | q.s. | q.s. | q.s. | q.s. | q.s. | q.s. | q.s. | q.s. | q.s. | q.s. |
| Aqua | Ad. 100 | Ad. 100 | Ad. 100 | Ad. 100 | Ad. 100 | Ad. 100 | Ad. 100 | Ad. 100 | Ad. 100 | Ad. 100 |

| | | | | | | | | | | |
|---|---|---|---|---|---|---|---|---|---|---|
| ¹ Pemulen TR-1, Lubrizol ² Aristoflex AVC, Clariant ³ UltraThix P-100, ISP ⁴ Carbopol 980, Lubrizol ⁵ Carbopol Ultrez 10, Lubrizol ⁶ Carbopol 981, Lubrizol ⁷ Tapioca Pure, National Starch ⁸ Dry Flo-PC, National Starch ⁹ Cleargum CO 01, Roquette | | | | | | | | | | |

### 1.2 W/O-Emulsion

| | 1 | 2 | 3 | 4 | 5 |
|---|---|---|---|---|---|
| Erfindungsgemäßes Polyharnstoffpulver | 6,0 | | 8,0 | 5,0 | 10,0 |
| Erfindungsgemäße wässrige Nanoharnstoff-Dispersion | | 10,0 | | 5,0 | |
| Polygylceryl-3 Diisostearate | 1,0 | | | | |
| Polyglyceryl-2 Dipolyhydroxystearate | 3,0 | 3,0 | | | |
| PEG-40 Sorbitan Perisostearate | | 3,0 | | | |
| Triglycerindiisostearate | | | 0,5 | | |
| Diglycerindipolyhydroxytsearate | | | 1,5 | | |
| PEG-30 Dipolyhydroxystearate | | | | 0,25 | |
| PEG-22 Dodecyl Glycol Copolymer | | | | | 5,0 |
| PEG-45 Dodecyl Glycol Polymer | | | | | 1,0 |
| Lanolin Alcohol | 1,0 | | | 0,3 | 0,5 |
| Behenyl Alcohol | | | 0,5 | | |
| Caprylic/Capric Triglyceride | 15,0 | 15,0 | | | |
| Mineral oil | 10,0 | 8,0 | 10,0 | 8,0 | 10,0 |
| Cera Microcrystallina | 5,0 | | 1,0 | | |
| Dicaprylylcarbonate | 1,0 | | | | |
| Isopropylstearate | | | | | 8,0 |
| Isopropylpalmitate | 1,0 | | | | |
| Rizinus oil | 1,0 | | | | |
| Vaseline | | | 6,0 | 5,0 | |
| Octyldodecanol | | | 1,0 | 3,0 | |
| Hydrogenated Cocoglyceride | | | | 2,0 | |
| Oenothera Biennis | | | | | 0,5 |
| Aluminium stearate | | | 0,3 | 0,6 | 0,5 |
| Magnesiumsulfate | 0,5 | 1,0 | 0,5 | 0,5 | 0,5 |
| Sodium citrate | 0,5 | 0,3 | 0,05 | | 0,2 |
| Sodium chloride | | | | 10,0 | |
| Citric acid | | | 0,1 | 0,2 | 0,2 |
| Potassium sorbate | | | 0,15 | | 0,4 |
| Glycerin | 3,0 | 8,0 | 5,0 | 3,0 | |
| Talc | | | | 0,5 | |
| Ethanol | | | 2,0 | | |
| Wirkstoffe | q.s. | q.s. | q.s. | q.s. | q.s. |
| Neutralisationsmittel | q.s. | q.s. | q.s. | q.s. | q.s. |
| Farbstoffe | q.s. | q.s. | q.s. | q.s. | q.s. |
| Parfüm | q.s. | q.s. | q.s. | q.s. | q.s. |
| Konservierungsmittel | q.s. | q.s. | q.s. | q.s. | q.s. |
| Aqua | Ad. 100 | Ad. 100 | Ad. 100 | Ad. 100 | Ad. 100 |

### 1.3 W/Si-Emulsion

| | 1 | 2 | 3 |
|---|---|---|---|
| Erfindungsgemäßes Polyharn- | 5,0 | 10,0 | |
| stoffpulver | | | |
| Erfindungsgemäße wässrige Nanoharnstoff-Dispersion | | | 30,0 |
| Cetyl dimethicone Copolyol | 2,0 | | |
| Cetyl PEG/PPG-10/1 Dimethicone | | 3,0 | 3,0 |
| Cyclomethicone | 15,0 | 25,0 | 25,0 |
| Dimethicone | 15,0 | 5,0 | 5,0 |
| Phenyltrimethicon | 1,0 | | |
| Hydrogenated Polyisobutene | | 2,0 | 2,0 |
| Dimethiconol | | 1,0 | 1,0 |
| Xanthan gum¹⁰ | 0,1 | | |
| Glycerin | 5,0 | 2,0 | 2,0 |
| Magnesium sulfate | 1,0 | | |
| Sodium chloride | | 0,7 | 0,7 |
| Citric acid | | 0,3 | 0,3 |
| Sodium citrate | | 0,9 | 0,9 |
| Potassium sorbate | | 0,3 | 0,3 |
| Wirkstoffe | q.s. | q.s. | q.s. |
| Neutralisationsmittel | q.s. | q.s. | q.s. |
| Farbstoffe | q.s. | q.s. | q.s. |
| Parfüm | q.s. | q.s. | q.s. |
| Konservierungsmittel | q.s. | q.s. | q.s. |
| Aqua | Ad. 100 | Ad. 100 | Ad. 100 |

| | | | |
|---|---|---|---|
| ¹⁰ Keltrol CG-T, CP Kelco | | | |

### 1.4 Hydrodispersion

| | 1 | 2 | 3 | 4 | 5 |
|---|---|---|---|---|---|
| Erfindungsgemäßes Polyharnstoffpulver | 5,0 | 10,0 | | 5,0 | 8,0 |
| Erfindungsgemäße wässrige Nanoharnstoff-Dispersion | | | 30,0 | | |
| Cetearyl Alkohol + PEG-40 Rizinusöl - Natrium Cetearyl Sulfat | 2,5 | | | | |
| Sorbitan Stearate | 1,0 | | | | |
| Ceteareth-20 | | | | 0,5 | |
| Ammonium Acryloyldimethyltaurate /VP Copolymer¹¹ | | | | | 1,0 |
| Acrylates/C10-30 Alkyl Acrylate crosspolymer¹² | 0,8 | 0,3 | 0,3 | 1,0 | |
| Xanthan gum¹³ | | 0,5 | 0,5 | | |
| Octyldodecanol | 2,0 | 2,0 | 2,0 | | 2,0 |
| Caprylic/Capric Triglycerid | 3,0 | 3,0 | 3,0 | | 2,0 |
| Cyclomethicone | 4,0 | | | | 2,0 |
| Isodecyl Neopentanoate | | | | 3,0 | |
| Dimethicone | | | | 2,0 | |
| Dicaprylylcarbonat | | | | | 2,0 |
| Sodium Starch Octenylsuccinat | 1,5 | | | | |
| Tapioka Starch | 3.0 | | | | 1,0 |
| Alcohol | 3.0 | | | | |
| Glycerin | 5,0 | 2,0 | 2,0 | 5,0 | 2,0 |
| Ethylhexyl Methoxycinnamate | | 8,0 | 8,0 | | |
| Octocrylene | | 5,0 | 5,0 | | |
| Phenylbenzimidazol Sulfonsäure | | 2,0 | 2,0 | | |
| Ethanol | | | | 5,0 | |
| Sodium starch Octenylsucccinate¹⁴ | | | | 0,5 | |
| Wirkstoffe | q.s. | q.s. | q.s. | q.s. | q.s. |
| Neutralisationsmittel | q.s. | q.s. | q.s. | q.s. | q.s. |
| Farbstoffe | q.s. | q.s. | q.s. | q.s. | q.s. |
| Parfüm | q.s. | q.s. | q.s. | q.s. | q.s. |
| Konservierungsmittel | q.s. | q.s. | q.s. | q.s. | q.s. |
| Aqua | Ad. 100 | Ad. 100 | Ad. 100 | Ad. 100 | Ad. 100 |

| | | | | | |
|---|---|---|---|---|---|
| ¹¹ Aristoflex AVC, Cla riant ¹² Pemulen TR-1, Lubrizol ¹³ Keltrol CG-T, CP Kelco ¹⁴ Cleargum CO 01, Roquette | | | | | |

### 2. Sun Care

### 2.1 O/W-Emulsion

| | 1 | 2 | 3 | 4 | 5 | 6 | 7 | 8 | 9 | 10 | 11 | 12 | 13 |
|---|---|---|---|---|---|---|---|---|---|---|---|---|---|
| Erfindungsgemäßes Polyhamstoffpulver | 6,0 | 5,0 | | 2,0 | 15,0 | 3,0 | 10,0 | 4,0 | 5,0 | 2,0 | 5,0 | | 7,50 |
| ErFndungsgemäße wässrige Nanoharnstoff-Dispersion | | | 16,0 | | | | | | 5,0 | | | 20,0 | |
| VP/Eicosene Copolymer | 0,5 | | | | 2,0 | | | | | 1,5 | | | |
| PVP/Hexadecane Copolymer | | 1,0 | | 0,5 | | 1,0 | 0,5 | 1,0 | 1,0 | | 0,5 | 1,0 | |
| Diglycol/CHDMsophtal ates/SIP Copolymer | | | 3,0 | | | | | | | | | | 2,5 |
| Sorbitan laurate (and) Polyglyceryl-10 laurate | | | | | | | | | | | | | 2,5 |
| Glyceryl Stearate Citrate | 2,0 | | | | | | | | | | | | |
| Glyceryl Stearate (Selbtsemulgierend) | | 2,0 | | | | | | | | | 2,0 | | |
| Polygyceryl-2 Dipolyhyhroxystearat | | | | | | | | | | | | 0,25 | |
| Polyglyceryl-3 Methylglucose Distearate | | | 2,5 | 2,0 | | | | | | | | | |
| Sorbitan stearate | | | | 1,0 | | | | | | | | | |
| Glyceryl Isostearate | | | | | | | | | | | | 3,5 | |
| Isoceteth-20 | | | | | | | | | | | 0,5 | 2,0 | |
| Ceteareth-12 | | | | | | | | | | | 5,0 | | |
| PEG-40 Stearate | | | | | 0,5 | | | | | | | | |
| Glyceryl Stearate | | | | | 2,5 | 0,5 | 2,0 | | 2,0 | | | | |
| PEG-100 Stearate | | | | | | | 2,0 | | | | | | |
| Sodium Stearoyl Glutamate | | | | | | | | 0,5 | 0,2 | | | | |
| Distearyldiammonium Chloride | | | | | | | | | | 1,0 | | | |
| Stearic acid | | 1,0 | | | | | | | | | | | |
| Behenyl Alcohol | | | | | | 1,0 | | | | | | | |
| Cetyl Alcohol | | | | | | | | | | 2,5 | 1,0 | | |
| Cetearyl Alcohol | | 2,0 | | | 5,0 | 10,0 | 2,0 | | | | | | |
| Myristyl Alcohol | | 2,0 | | | | | | | | | | | |
| Stearyl Alcohol | 1,0 | | 1,0 | | | | | | 3,0 | 1,0 | | | |
| Ethylhexyl stearate | | | | | | | | | | | | | 7,0 |
| Acrylates/C10-30 Alkyl Acrylate crosspolymer¹⁵ | 0,1 | | | | | | 0,8 | | 0,3 | | | | 0,2 |
| Ammonium Acryloyldimethyltaurate | | 0,5 | | | | | | | | | | | |
| NP Copolymer¹⁶ | | | | | | | | | | | | | |
| Acrylic AcidNP Crosspolymer¹⁷ | | | | 0,6 | | | | 0,2 | | | | | |
| Carbomer | | | 0,8 18 | | 0,3¹⁹ | | | | 0,5 20 | | | | |
| Xanthan gum | | | | | | | | | | | | | 0,15 |
| Dimethylpolysiloxan | | | | 5 | | | | | | | | | |
| Dicaprylyl Ether | 1,0 | 3,0 | | | | | | | | 1,0 | | | |
| Myristyl Myristate | 3,0 | | | | | | | 1,0 | | | | | |
| Octyldodecanol | 1,0 | | | 4,0 | 5,0 | 3,0 | | | 4,0 | | 3,0 | | |
| Butylene Glycol Dicaprylate/Dicaprate | | 2 | | 2,0 | 3,0 | | | 2 | | 7,0 | | | |
| C12-15 Alkyl Benzoate | | 2 | | | 3,0 | 1 | 5,0 | 2 | 5,0 | | | 5,0 | 6,0 |
| Isohexadecan | | | 2,0 | | | | 3,0 | | | | | | 1,5 |
| Caprylic/Capric Triglycerid | | | | 2,0 | | | | 2,0 | | | | | |
| Cyclomethicone | 4,0 | | 2,0 | | | 2,0 | | | 1,0 | | | | |
| Dimethicone | 2,0 | | 1,0 | | | 2,0 | 5,0 | | 1,0 | | | | |
| Mineral Oil | | | | | | 5,0 | | | | 2,5 | | | |
| Hydrietes Polyisobutene | | | | | | 2,0 | | | | | | | |
| Phenethylbenzoate | | | | | | | | 5,0 | | | | | |
| Isodecyl Neopentanoate | | | | | | | | | 2,0 | | | | |
| Oenothera Biennis | | | | | | | | | | 2,0 | | | |
| Shea Butter | | | | | | | | | | 2,0 | | | |
| Butylenglycol | | | | 5,0 | | | | | | | | | |
| Glycerin | 10 | 7,5 | 5,0 | | 3,0 | | 10 | 8,0 | 5,0 | 5,0 | 5,0 | 7,0 | 5,0 |
| Ethylhexylglycerin | 0,5 | | | | | 0,5 | | | | | | | |
| Ethanol | 3,0 | | | | | | | | 4,0 | | | | |
| Tapioca starch²¹ | 1,0 | | 1,0 | | | | | | | | | | |
| Distarch Phosphate | | 2,0 | | | | | | | | | | | |
| Aluminium starch ²²Octenylsuccinat | | | | | | | | 2,0 | | | | | |
| Natrium starch Octenylsucccinate²³ | | | | | 2,0 | | | | | | | | |
| Bisethylhexyloxy phenol Methoxyphenyl Triazine | | | 1 | | | | | | 2,0 | 3,0 | | | |
| Ethylhexyl Triazone | | | | 2,0 | | | | | 2,0 | | | | |
| Butyl Methoxydibenzoylmethane | 3 | 3 | | | 2,5 | 4 | | 2 | 3,0 | 1,0 | 2,0 | 3,0 | 2,0 |
| Ethylhexyl Methoxycinnamate | 4 | 4 | 3 | | 5,0 | | 5,0 | 5 | 5,0 | | 5,0 | | 7,0 |
| Octocrylene | | | | | 4,0 | 5 | | | | | | 4,0 | |
| Phenylbenzimidazole Sulfonic Acid | | | | 1,0 | 2,0 | 2 | 2,0 | | | | | | 4,0 |
| Titanium Dioxide + Trimethoxycaprylylsilane | | | | | | 1 | | | | | | | |
| Terephthalidene dicamphor sulfonic acid | | | | | | | | | | | | 3,0 | |
| Diethylamino Hydrobenzoyl Hexylbenzoat | | | | | | | 1,0 | | | | | | |
| Phenylen-1,4-bis-(2-benzimidazol)-3,3,5,5'-tetrasulfonic acid | | | | 1,0 | | | 2,0 | | | | | | |
| Polysilicon-15 | | | | | | | | | | 3,0 | | | |
| Benzophenone-3 | | | | | | | | | | | 2,0 | | |
| Titanium Dioxide | | | | 2,0 | | | | | | | | | |
| Trisodium EDTA | 1 | 1 | 1 | | | | 1 | 1 | 1 | 1 | 1 | | 1 |
| Wirkstoffe | q.s. | q.s. | q.s. | q.s. | q.s. | q.s. | q.s. | q.s. | q.s. | q,s. | q.s. | q.s. | q.s. |
| Neutralisationsmittel | q.s. | q.s. | q.s. | q.s. | q.s. | q.s. | q.s. | q.s. | q.s. | q.s. | q.s. | q.s. | q.s. |
| Farbstoffe | q.s. | q.s. | q.s. | q.s. | q.s. | q.s. | q.s. | q.s. | q.s. | q.s. | q.s. | q.s. | q.s. |
| Parfüm | q.s. | q.s. | q.s. | q.s. | q.s. | q.s. | q.s.: | q.s. | q.s. | q.s. | q.s. | q.s. | q.s. |
| Konservierungsmittel | q.s. | q.s. | q.s. | q.s. | q.s. | q.s. | q.s. | q.s. | q.s. | q.s. | q.s. | q.s. | q.s. |
| Aqua | Ad. 100 | Ad. 100 | Ad. 100 | Ad. 100 | Ad. 100 | Ad. 100 | Ad. 100 | Ad. 100 | Ad. 100 | Ad. 100 | Ad. 100 | Ad. 100 | Ad. 100 |

| | | | | | | | | | | | | | |
|---|---|---|---|---|---|---|---|---|---|---|---|---|---|
| ¹⁵ Pemulen TR-1, Lubrizol ¹⁶ Aristoflex AVC, Clariant ¹⁷ UltraThix P-100, ISP ¹⁸ Carbopol 980, Lubrizol ¹⁹ Carbopol Ultrez 10, Lubrizol ²⁰ Carbopol 981, Lubrizol ²¹ Tapioca Pure, National Starch ²² Dry Flo-PC, National Starch ²³ Cleargum CO 01, Roquette | | | | | | | | | | | | | |

### 2.2 W/O-Emulsion

| | 1 | 2 | 3 | 4 | 5 | 6 | 7 |
|---|---|---|---|---|---|---|---|
| Erfindungsgemäßes Polyharnstoffpulver | 2,0 | 5,0 | 8,0 | | | 2,0 | 10,0 |
| Erfindungsgemäße wässrige Nanoharnstoff-Dispersion | | | | 5,0 | 30,0 | | |
| VP/Eicosene Copolymer | 1,0 | | | 1,0 | | 0,5 | |
| PVP/Hexadecane Copolymer | | 0,5 | 1,0 | | 1,0 | | 2,0 |
| Polygylceryl-3 Diisostearate | 1,0 | | | 1,0 | | | |
| Polyglyceryl-2 Dipolyhydroxystearate | 3,0 | 3,0 | | 3,0 | | | |
| PEG-4.0 Sorbitan Perisostearate | | 3,0 | | | | | |
| Triglycerindiisostearate | | | 0,5 | | 0,5 | | |
| Diglycerindipolyhydroxytsearate | | | 1,5 | | 1,5 | | |
| PEG-30 Dipolyhydroxystearate | | | | | | 0,25 | |
| PEG-22 Dodecyl Glycol Copolymer | | | | | | | 5,0 |
| PEG-45 Dodecyl Glycol Polymer | | | | | | | 1,0 |
| Lanolin Alcohol | 1,0 | | | 1,0 | | 0,3 | 0,5 |
| Behenyl Alcohol | | | 0,5 | | 0,5 | | |
| Caprylic/Capric Triglyceride | 15,0 | 15,0 | | 15,0 | | | |
| Mineral oil | 10,0 | 8,0 | 10,0 | 10,0 | 10,0 | 8,0 | 10,0 |
| Cera Microcrystallina | 5,0 | | 1,0 | 5,0 | 1,0 | | |
| Dicaprylylcarbonate | 1,0 | | | 1,0 | | | |
| Isopropylstearate | | | | | | | 8,0 |
| Isopropylpalmitate | 1,0 | | | 1,0 | | | |
| Rizinus oil | 1,0 | | | 1,0 | | | |
| Vaseline | | | 6,0 | | 6,0 | 5,0 | |
| Octyldodecanol | | | 1,0 | | 1,0 | 3,0 | |
| Hydrierte Kokoglyceride | | | | | | 2,0 | |
| Oenothera Biennis | | | | | | | 0,5 |
| Butylene Glycol Dicaprylate/Dicaprate | | | 2 | | 2 | | |
| C12-15 Alkyl Benzoate | | 4 | 2 | | 2 | 1 | 4 |
| Aluminiumstearate | | | 0,3 | | 0,3 | 0,6 | 0,5 |
| Magnesiumsulfate | 0,5 | 1,0 | 0,5 | 0,5 | 0,5 | 0,5 | 0,5 |
| Sodium citrate | 0,5 | 0,3 | 0,05 | 0,5 | 0,05 | | 0,2 |
| Sodium chloride | | | | | | 10,0 | |
| Citric acid | | | 0,1 | | 0,1 | 0,2 | 0,2 |
| Potassium sorbate | | | 0,15 | | 0,15 | | 0,4 |
| Ethylhexylglycerin | 0,5 | | | 0,5 | | | |
| Glycerin | 3,0 | 8,0 | 5,0 | 3,0 | 5,0 | 3,0 | |
| Trisodium EDTA | | 1 | 1 | | 1 | | |
| Ethylhexyl Triazone | | | | | | | |
| Butyl Methoxydibenzoylmethane | | 2,5 | | | | 4 | 2 |
| Ethylhexyl Methoxycinnamate | | | 3 | | 3 | | 5 |
| Octocrylene | 3 | | | 3 | | 5 | |
| Phenylbenzimidazole Sulfonic Acid | | 4 | | | | 2 | |
| Titanium Dioxide | 1 | | | 1 | | 1 | |
| Talc | | | | | | 0,5 | |
| Ethanol | | | 2,0 | | 2,0 | | |
| Trisodium EDTA | | 1 | 1 | | 1 | | |
| Wirkstoffe | q.s. | q.s. | q.s. | q.s. | q.s. | q.s. | q.s. |
| Neutralisationsmittel | q.s. | q.s. | q.s. | q.s. | q.s. | q.s. | q.s. |
| Farbstoffe | q.s. | q.s. | q.s. | q.s. | q.s. | q.s. | q.s. |
| Parfüm | q.s. | q.s. | q.s. | q.s. | q.s. | q.s. | q.s. |
| Konservierungsmittel | q.s. | q.s. | q.s. | q.s. | q.s. | q.s. | q.s. |
| Aqua | Ad. 100 | Ad. 100 | Ad. 100 | Ad. 100 | Ad. 100 | Ad. 100 | Ad. 100 |

### 2.3 W/Si-Emulsion

| | 1 | 2 | 3 |
|---|---|---|---|
| Erfindungsgemäßes Polyharnstoffpulver | 5,0 | | 10,0 |
| Erfindungsgemäße wässrige Nanoharnstoff-Dispersion | | 10,0 | |
| VP/Eicosene Copolymer | 1,0 | 1,0 | |
| PVP/Hexadecane Copolymer | | | 1,0 |
| Cetyl dimethicone Copolyol | 2,0 | 2,0 | |
| Cetyl PEG/PPG-10/1 Dimethicone | | | 3,0 |
| Cyclomethicon | 15,0 | 15,0 | 25,0 |
| Dimethicone | 15,0 | 15,0 | 5,0 |
| Phenyltrimethicon | 1,0 | 1,0 | |
| Hydrierte Polyisobuten | | | 2,0 |
| Dimethiconol | | | 1,0 |
| Xanthan gum²⁴ | 0,1 | 0,1 | |
| Ethylhexylglycerin | | | 0,5 |
| Glycerin | 5,0 | 5,0 | 2,0 |
| Magnesium sulfate | 1,0 | 1,0 | |
| Natrium chloride | | | 0,7 |
| Citric acid | | | 0,3 |
| Sodium citrat | | | 0,9 |
| Potassium sorbate | | | 0,3 |
| Trisodium EDTA | | | 1 |
| Ethylhexyl Triazone | 2 | 2 | |
| Butyl Methoxydibenzoylmethane | 3 | 3 | |
| Ethylhexyl Methoxycinnamate | | | 2 |
| Titanium Dioxide | 0,5 | 0,5 | 2 |
| Wirkstoffe | q.s. | q.s. | q.s. |
| Neutralisationsmittel | q.s. | q.s. | q.s. |
| Farbstoffe | q.s. | q.s. | q.s. |
| Parfüm | q.s. | q.s. | q.s. |
| Konservierungsmittel | q.s. | q.s. | q.s. |
| Aqua | Ad. 100 | Ad. 100 | Ad. 100 |

| | | | |
|---|---|---|---|
| ²⁴ Keltrol CG-T, CP Kelco | | | |

### 2.4 Hydrodispersion

| | 1 | 2 | 3 | 4 | 5 | 6 |
|---|---|---|---|---|---|---|
| Erfindungsgemäßes Polyharnstoffpulver | 2,5 | | 10,0 | 5,0 | 5,0 | 8,0 |
| Erfindungsgemäße wässrige Nanoharnstoff-Dispersion | | 10,0 | | | 20,0 | |
| VP/Eicosene Copolymer | 1,0 | 1,0 | | | | |
| PVP/Hexadecane Copolymer | | | 0,5 | | | 1,0 |
| Diglycol/CHDM/Isophtalates/SIP Copolymer | | | | 2,0 | 2,0 | |
| Cetearyl Alkohol + PEG-40 Rizinusöl - Natrium Cetearyl Sulfat | 2,5 | 2,5 | | | | |
| Sorbitan Stearat | 1,0 | 1,0 | | | | |
| Ceteareth-20 | | | | 0,5 | 0,5 | |
| Ammonium Acryloyldimethyltaurate /VP Copolymer²⁵ | | | | | | 1,0 |
| Acrylates/C10-30 Alkyl Acrylate crosspolymr²⁶ | 0,8 | 0,8 | 0,3 | 1,0 | 1,0 | |
| Xanthan gum²⁷ | | | 0,5 | | | |
| Octyldodecanol | 2,0 | 2,0 | 2,0 | | | 2,0 |
| Caprylic/Capric Triglycerid | 3,0 | 3,0 | 3,0 | | | 2,0 |
| Cyclomethicon | 4,0 | 4,0 | | | | 2,0 |
| Isodecyl Neopentanoate | | | | 3,0 | 3,0 | |
| Dimethicone | | | | 2,0 | 2,0 | |
| Dicaprylylcarbonat | | | | | | 2,0 |
| Butylene Glycol Dicaprylate/Dicaprate | 2,0 | 2,0 | | 5,0 | 5,0 | |
| C12-15 Alkyl Benzoate | 2,0 | 2,0 | | | | 5,0 |
| Natrium Starch Octenylsuccinat | 1,5 | 1,5 | | | | |
| Tapioka Starch | 3.0 | 3.0 | | | | 1,0 |
| Ethanol | 3.0 | 3.0 | | | | |
| Glycerin | 5,0 | 5,0 | 2,0 | 5,0 | 5,0 | 2,0 |
| Ethylhexyl Methoxycinnamat | 3,0 | 3,0 | 8,0 | | | |
| Octocrylen | | | 5,0 | | | |
| Phenylbenzimidazol Sulfonsäure | | | 2,0 | | | |
| Butyl Methoxydibenzoylmethane | 2,0 | 2,0 | | 3,0 | 3,0 | 3,0 |
| Ethylhexyl Triazone | | | | 2,0 | 2,0 | 2,0 |
| Bis-Ethylhexyloxyphenol Methoxyphenyl Triazine | | | | | | 2,0 |
| Titanium Dioxide | | | | 0,5 | 0,5 | |
| Trisodium EDTA | 1,0 | 1,0 | | 1,0 | 1,0 | 1,0 |
| Ethanol | | | | 5,0 | 5,0 | |
| Natrium starch Octenylsucccinate²⁸ | | | | 0,5 | 0,5 | |
| Wirkstoffe | q.s. | q.s. | q.s. | q.s. | q.s. | q.s. |
| Neutralisationsmittel | q.s. | q.s. | q.s. | q.s. | q.s. | q.s. |
| Farbstoffe | q.s. | q.s. | q.s. | q.s. | q.s. | q.s. |
| Parfüm | q.s. | q.s. | q.s. | q.s. | q.s. | q.s. |
| Konservierungsmittel | q.s. | q.s. | q.s. | q.s. | q.s. | q.s. |
| Aqua | Ad. 100 | Ad. 100 | Ad. 100 | Ad. 100 | Ad. 100 | Ad. 100 |

| | | | | | | |
|---|---|---|---|---|---|---|
| ²⁵ Aristoflex AVC, Clariant ²⁶ Pemulen TR-1, Lubrizol ²⁷ Keltrol CG-T, CP Kelco ²⁸ Cleargum CO 01, Roquette | | | | | | |

### 3. Dekorative Kosmetik

### 3.1 Mascara

| Rohstoffe | Gew.% (bezogen auf die kosmetische Zusammensetzung) | Gew.% (bezogen auf die kosmetische Zusammensetzung) | Gew.% (bezogen auf die kosmetische Zusammensetzung) |
|---|---|---|---|
| lsododecane | 20,00 | 20,00 | 20,00 |
| D5 Cyclomethicone | 5,00 | 5,00 | 5,00 |
| Carnauba Wax | 6,00 | 6,00 | 6,00 |
| Trimethyl Siloxysilicate | 0,75 | 0,75 | 0,75 |
| Dimethicone 200/200 | 10,00 | 10,00 | 10,00 |
| PVP/Eicosene Copolymer | 7,5 | 7,5 | 7,5 |
| Erfindungsgemäßes Polyharnstoffpulver | 5,0 | 5,0 | 0 |
| Erfindungsgemäße wässrige Nanoharnstoff-Dispersion | 0 | 5,0 | 10,0 |
| Ceresin Wax SP252 | 3,00 | 3,00 | 3,00 |
| Paraffin Wax 130/135 | 3,50 | 3,50 | 3,50 |
| Polyethylene | 2,50 | 2,50 | 2,50 |
| Nylon-12 | 2,00 | 2,00 | 2,00 |
| Silica | 2,00 | 2,00 | 2,00 |
| Stearic Acid | 1,00 | 1,00 | 1,00 |
| Bentone Gel in Isododecane | 15,00 | 15,00 | 15,00 |
| Phenoxyethanol | 1,00 | 1,00 | 1,00 |
| Black Iron Oxide LC989 EM | 10,00 | 10,00 | 10,00 |
| White Beeswax | 1,75 | 1,75 | 1,75 |
| Deionized Water | Ad. 100 | Ad. 100 | Ad. 100 |
| Magnesium Aluminium Silicate | 0,50 | 0,50 | 0,50 |
| Triethanolamine 99 % | 0,90 | 0,90 | 0,90 |
| Net-DTB (10% in Butylene Glycol) | 1,00 | 1,00 | 1,00 |

### 3.2 Foundation

| Rohstoffe | Gew.% (bezogen auf die kosmetische Zusammensetzung) | Gew.% (bezogen auf die kosmetische Zusammensetzung) | Gew.% (bezogen auf die kosmetische Zusammensetzung) |
|---|---|---|---|
| Deionized Water | ad. 100 | ad. 100 | ad. 100 |
| Cellulose Gum | 0,30 | 0,30 | 0,30 |
| Magnesium Aluminum Silicate | 0,35 | 0,35 | 0,35 |
| Lecithin | 0,40 | 0,40 | 0,40 |
| Triethanolamine 99% | 1,25 | 1,25 | 1,25 |
| Butylene Glycol | 6,00 | 6,00 | 6,00 |
| Titanium Dioxide (Water Dispersible) | 8,00 | 8,00 | 8,00 |
| Red Iron Oxide | 0,40 | 0,40 | 0,40 |
| Yellow lron Oxide | 0,80 | 0,80 | 0,80 |
| Black Iron Oxide | 0,10 | 0,10 | 0,10 |
| Colloidal Kaolin | 2,00 | 2,00 | 2,00 |
| Methyl Paraben | 0,20 | 0,20 | 0,20 |
| lsoeicosane | 10,00 | 10,00 | 10,00 |
| Isostearic Acid | 1,00 | 1,00 | 1,00 |
| Stearic Acid | 2,50 | 2,50 | 2,50 |
| Glyceryl Stearate | 1,50 | 1,50 | 1,50 |
| Tridecyl Trimellitate | 1,00 | 1,00 | 1,00 |
| Glyceryl Stearate SE | 1,00 | 1,00 | 1,00 |
| Propyl Paraben | 0,20 | 0,20 | 0,20 |
| Acrylates Copolymer²⁹ | 5,0 | 5,0 | 5,0 |
| Erfindungsgemäßes Polyharnstoffpulver | 5,0 | 2,5 | 0 |
| Wässrige Nanoharnstoff-Dispersion | 0 | 2,5 | 20,0 |
| Wirkstoffe | q.s. | q.s. | q.s. |
| Farbstoffe | q.s. | q.s. | q.s. |
| Parfüm | q.s. | q.s. | q.s. |
| Konservierungsmittel | q.s. | q.s. | q.s. |
| Aqua | Ad.100 | Ad.100 | Ad.100 |

| | | | |
|---|---|---|---|
| ²⁹ Covacryl E 14, Sensient | | | |

### 3.3 Eyeliner

| Rohstoffe | Gew.% (bezogen auf die kosmetische Zusammensetzung) | Gew.% (bezogen auf die kosmetische Zusammensetzung) | Gew.% (bezogen auf die kosmetische Zusammensetzung) |
|---|---|---|---|
| Oleyl Alcohol | 0,5 | 0,5 | 0,5 |
| Propylene Glycol | 7,5 | 7,5 | 7,5 |
| Xanthan Gum | 0,1 | 0,1 | 0,1 |
| Silica | 0,1 | 0,1 | 0,1 |
| Acrylates Copolymer³⁰ | 0,5 | 0,5 | 0,5 |
| Erfindungsgemäßes Polyharnstoffpulver | 2,0 | 1,0 | 0 |
| Wässrige Nanoharnstoff-Dispersion | 0 | 1,0 | 2,0 |
| Wirkstoffe | q.s. | q.s. | q.s. |
| Farbstoffe | q.s. | q.s. | q.s. |
| Parfüm | q.s. | q.s. | q.s. |
| Konservierungsmittel | q.s. | q.s. | q.s. |
| Aqua | Ad.100 | Ad.100 | Ad.100 |

| | | | |
|---|---|---|---|
| ³⁰ Covacryl E 14, Sensient | | | |

### 3.4 Bräunungsmittel

| Rohstoffe | Gew.% (bezogen auf die kosmetische Zusammensetzung) | Gew.% (bezogen auf die kosmetische Zusammensetzung) |
|---|---|---|
| Dihydroxyaceton | 3,0 | 3,0 |
| Glycerin | 8,0 | 8,0 |
| Cetyl Alcohol | 0,5 | 0,5 |
| Silica | 3,0 | 3,0 |
| Methylglucose Sesquistearate | 2,0 | 2,0 |
| PEG-100 stearate | 1,0 | 1,0 |
| Cyclomethicone | 4,0 | 4,0 |
| Erfindungsgemäßes Polyharnstoffpulver | 10,0 | |
| Erfindungsgemäße wässrige Nanoharnstoff-Dispersion | | 20,0 |
| Octyldodecanol | 3,0 | 3,0 |
| Dicaprylylcarbonate | 2,0 | 2,0 |
| EDTA | 1,0 | 1,0 |
| Xanthan Gum | 0,3 | 0,3 |
| Sodium Citrate | 0,4 | 0,4 |
| Citric acid | 0,3 | 0,3 |
| Vitamin E- Acetat | 0,5 | 0,5 |
| Wirkstoffe | q.s. | q.s. |
| Farbstoffe | q.s. | q.s. |
| Parfüm | q.s. | q.s. |
| Konservierungsmittel | q.s. | q.s. |
| Aqua | Ad.100 | Ad.100 |

### 3.5 Getönte Tagescreme

| Rohstoffe | Gew.% (bezogen auf die kosmetische Zusammensetzung) | Gew.% (bezogen auf die kosmetische Zusammensetzung) |
|---|---|---|
| Glyceryl Stearate Citrate | 3,5 | 3,5 |
| Octyldodecanol | 3,0 | 3,0 |
| Cyclomethicone | 3,0 | 3,0 |
| Cetearyl Alcohol | 1,5 | 1,5 |
| Squalane | 2,0 | 2,0 |
| Shea butter | 5,0 | 5,0 |
| Carbomer | 0,5 | 0,5 |
| Glycerin | 10,0 | 10,0 |
| 4-Methylbenzyliden Campher | 5,0 | 5,0 |
| Octyl Methoxycinnamat | 2,5 | 2,5 |
| Octocrylene | 6,0 | 6,0 |
| Butyl Methoxydibenzoylmethane | 2,5 | 2,5 |
| Erfindungsgemäßes Polyharnstoffpulver | 5,0 | |
| Erfindungsgemäße wässrige Nanoharnstoff-Dispersion | | 20,0 |
| EDTA | 1,0 | 1,0 |
| Wirkstoffe | q.s. | q.s. |
| Farbstoffe | q.s. | q.s. |
| Parfüm | q.s. | q.s. |
| Konservierungsmittel | q.s. | q.s. |
| Aqua | Ad.100 | Ad.100 |

### 3.6 Lippenstift

| Rohstoffe | Gew.% (bezogen auf die kosmetische Zusammensetzung) | Gew.% (bezogen auf die kosmetische Zusammensetzung) | Gew.% (bezogen auf die kosmetische Zusammensetzung) |
|---|---|---|---|
| Rizinus oil | 3,0 | 3,0 | 3,0 |
| Caprylic/Capric Triglycerides | 3,0 | 3,0 | 3,0 |
| Octyldodecanol | 5,0 | 5,0 | 5,0 |
| Hydrogenated polyisobutene | 3,0 | 3,0 | 3,0 |
| Jojaba oil | 1,0 | 1,0 | 1,0 |
| Lanolin oil | 1,0 | 1,0 | 1,0 |
| PEG 45 / Dodecyl Glycol copolymer | 2,0 | 2,0 | 2,0 |
| Polyglyceryl-3 Diisostearat | 2,4 | 2,4 | 2,4 |
| Cetyl palmitate | 1,0 | 1,0 | 1,0 |
| C20-40 Alkyl Stearate | 8,0 | 8,0 | 8,0 |
| Carnauba wax | 2,0 | 2,0 | 2,0 |
| Microcristalline wax | 8,0 | 8,0 | 8,0 |
| Glycerin | 10,0 | 10,0 | 10,0 |
| Erfindungsgemäßes Polyharnstoffpulver | 15,0 | 8,0 | |
| Erfindungsgemäße wässrige Nanoharnstoff-Dispersion | | 8,0 | 30,0 |
| Wirkstoffe | q.s. | q.s. | q.s. |
| Farbstoffe | q.s. | q.s. | q.s. |
| Parfüm | q.s. | q.s. | q.s. |
| Konservierungsmittel | q.s. | q.s. | q.s. |
| Aqua | Ad.100 | Ad.100 | Ad.100 |

| Rohstoffe | Gew.% (bezogen auf die kosmetische Zusammensetzung) |
|---|---|
| Microcristalline wax | 3,0 |
| Candelilla wax | 12,0 |
| Cera alba | 2,5 |
| Polyglyceryl-3 Diisostearate | 10,0 |
| Cyclomethicone | 25,0 |
| Octyldodecanol | Ad. 100 |
| Polyisobutene | 5,0 |
| Caprylic/capric Triglecerides | 12,0 |
| Pentaerythrityl tetraisostearate | 10,0 |
| PVP/Hexadecene Copolymer | 0,5 |
| Erfindungsgemäßes Polyharnstoffpulver | 5,0 |
| Wirkstoffe | q.s. |
| Farbstoffe | q.s. |
| Parfüm | q.s. |
| Konservierungsmittel | q.s. |

### 3.7 Gesichtspuder (Lose)

| Rohstoffe | Gew.% (bezogen auf die kosmetische Zusammensetzung) |
|---|---|
| Talc | 59,000 |
| Mica | 10,000 |
| Zinc Stearate | 6,000 |
| Lauroyl Lysine | 0,450 |
| Bismuth oxychloride | 5,000 |
| Nylon-12 | 5,000 |
| Silica | 5,000 |
| Yellow Iron Oxide | 0,750 |
| Red Iron Oxide | 0,375 |
| Black Iron Oxide | 0,075 |
| Potassium Sorbate | 0,200 |
| BHT | 0,050 |
| Erfindungsgemäßes Polyharnstoffpulver | 5,000 |
| Ethylhexyl Palmitat | 1,000 |
| Phenoxyethanol | 1,000 |
| Dimethicone | 1,000 |
| Tocopheryl Acetate | 0,100 |

### 3.8 Gesichtspuder (gepresst)

| Rohstoffe | Gew.% (bezogen auf die kosmetische Zusammensetzung) |
|---|---|
| Titanium dioxide | 5,0 |
| Magnesium stearate | 3,0 |
| Magnesium silica | 33,0 |
| Glimmer | 7,0 |
| Eisenoxide | 2,0 |
| Talc | Ad 100 |
| Isopropyl isostearate | 2,0 |
| Octyldodecyl Stearoyl Stearate | 2,0 |
| Erfindungsgemäßes Polyharnstoffpulver | 10,0 |
| Wirkstoffe | q.s |
| Farbstoffe | q.s |
| Parfüm | q.s |
| Konservierungsmittel | q.s |

### 3.9 Gegossenes Blush

| Rohstoffe | Gew.% (bezogen auf die kosmetische Zusammensetzung) |
|---|---|
| Caprylic/capric triglyceride | Ad 100 |
| Microcrystalline wax | 3,5 |
| Cera alba | 4,0 |
| C20-40 alkyl stearate | 6,0 |
| Cetyl palmitate | 2,5 |
| Isononyl isononanoate | 6,0 |
| Lauroyl Lysin | 1,0 |
| Nylon | 4,0 |
| PVP/Eicosene Copolymer | 2,0 |
| Tocopheryl acetate | 1,0 |
| Erfindungsgemäßes Polyharnstoffpulver | 10,0 |
| Wirkstoffe | q.s |
| Farbstoffe | q.s |
| Parfüm | q.s |
| Konservierungsmittel | q.s |

### 4. Deodorantien and Antitranspirantien

### 4.1 Deospray

| Rohstoffe | Gew.% (bezogen auf die kosmetische Zusammensetzung) | Gew.% (bezogen auf die kosmetische Zusammensetzung) |
|---|---|---|
| Farnesol | 1,0 | 1,0 |
| Octyldodecanol | 0,5 | 0,5 |
| Ethanol | Ad.100 | Ad.100 |
| Treibgas | 60,0 | 60,0 |
| Erfindungsgemäße wässrige Nanoharnstoff-Dispersion | | 10,0 |
| Erfindungsgemäßes Polyharnstoffpulver | 5,0 | |

### 4.2 Deopumpspray

| Rohstoffe | Gew.% (bezogen auf die kosmetische Zusammensetzung) |
|---|---|
| Deoparfümöl | 2,5 |
| Lösungsvermittler | 0,5 |
| Ethanol | 60,0 |
| Water | 30 |
| PEG-100 | 2,0 |
| Erfindungsgemäßes Polyharnstoffpulver | 5,0 |

### 4.3 Antitranspirant-Rollergel

| Rohstoffe | Gew.% (bezogen auf die kosmetische Zusammensetzung) | Gew.% (bezogen auf die kosmetische Zusammensetzung) | Gew.% (bezogen auf die kosmetische Zusammensetzung) |
|---|---|---|---|
| ACH (50 wt.% aq. Lsg.) | 20,0 | 20,0 | 20,0 |
| Hydroxyethylcellulose | 0,5 | 0,5 | 0,5 |
| Etahnol | 40 | 40 | 40 |
| Water | Ad. 100 | Ad. 100 | Ad. 100 |
| Farbstoffe | q.s | q.s | q.s |
| Parfüm | q.s | q.s | q.s |
| Lösungsvermittel | q.s | q.s | q.s |
| Erfindungsgemäße wässrige Nanoharnstoff-Dispersion | | 7,5 | 20,0 |
| Erfindungsgemäßes Polyharnstoffpulver | 7,5 | 7,5 | |

### 4.4 Antitranspirant-Stift

| Rohstoffe | Gew.% (bezogen auf die kosmetische Zusammensetzung) |
|---|---|
| Al-Zr-Tetrachlorohydrex Glycerin (35 wt.% aq. Lsg) | 40,0 |
| Stearyl alcohol | 20,0 |
| Glycerylstearate + PEG-100 stearate | 1,0 |
| Talc | 1,5 |
| PEG-20 | 5,0 |
| Aerosil | 1,5 |
| Cyclopentasiloxane | Ad. 100 |
| Farbstoffe | q.s |
| Parfüm | q.s |
| Lösungsvermittel | q.s |
| Erfindungsgemäßes Polyharn- | 7,5 |
| stoffpulver | |

### 4.5 Deo Creme

| Rohstoffe | Gew.% (bezogen auf die kosmetische Zusammensetzung) | Gew.% (bezogen auf die kosmetische Zusammensetzung) |
|---|---|---|
| Glyceryl sterarate | 4,0 | 4,0 |
| PEG-2000 stearate | 4,5 | 4,5 |
| Cetyl alcohol | 5,0 | 5,0 |
| Cyclopentasiloxane | 6,0 | 6,0 |
| Mineral oil | 4,5 | 4,5 |
| Avocado oil | 0,1 | 0,1 |
| Water | Ad.100 | Ad.100 |
| Konservierungsmittel | q.s. | q.s. |
| Farbstoffe | q.s | q.s |
| Deoparfümöl | q.s | q:s |
| Lösungsvermittel | q.s | q.s |
| Erfindungsgemäße wässrige Nanoharnstoff-Dispersion | | 15 |
| Erfindungsgemäßes Polyharnstoffpulver | 10 | |

### 4.6 Roller Deo O/W Emulsion

| Rohstoffe | Gew.% (bezogen auf die kosmetische Zusammensetzung) | Gew.% (bezogen auf die kosmetische Zusammensetzung) |
|---|---|---|
| Glyceryl laurate | 1,0 | 1,0 |
| Glyceryl stearate | 4,0 | 4,0 |
| Cetyl alcohol | 3,0 | 3,0 |
| Octyldodecanol | 5,0 | 5,0 |
| Ethanol | 10,0 | 10,0 |
| Glycerin | 5,0 | 5,0 |
| Carbomer³¹ | 0,6 | 0,6 |
| Water | Ad.100 | Ad.100 |
| Konservierungsmittel | q.s. | q.s. |
| Farbstoffe | q.s | q.s |
| Deoparfümöl | q.s | q.s |
| Neutralisierungsmittel | q.s | q.s |
| Lösungsvermittel | q.s | q.s |
| Erfindungsgemäße wässrige. Nanoharnstoff-Dispersion | | 10,0 |
| Erfindungsgemäßes Polyharnstoffpulver | 5,0 | |

| | | |
|---|---|---|
| ³¹ Carbopol 980, Lubrizol | | |

### 4.7 Mikroemulsion

| Rohstoffe | Gew.% (bezogen auf die kosmetische Zusammensetzung) | Gew.% (bezogen auf die kosmetische Zusammensetzung) | Gew.% (bezogen auf die kosmetische Zusammensetzung) |
|---|---|---|---|
| Glyceryl stearate | 2,0 | 2,0 | 2,0 |
| Isosteareth-20 | 4,0 | 4,0 | 4,0 |
| Octyldodecanol | 2,0 | 2,0 | 2,0 |
| Dicaprylyl carbonate | 2,0 | 2,0 | 2,0 |
| Glycerin | 3,0 | 3,0 | 3,0 |
| ACH (50 wt.% Aq. Lsg.) | 20,0 | 20,0 | 20,0 |
| Avocado oil | 0,10 | 0,10 | 0,10 |
| Water | Ad.100 | Ad.100 | Ad.100 |
| Konservierungsmittel | q.s. | q.s. | q.s. |
| Farbstoffe | q.s | q.s | q.s |
| Deoparfümöl | q.s | q.s | q.s |
| Neutralisierungsmittel | q.s | q.s | q.s |
| Lösungsvermittel | q.s | q.s | q.s |
| Erfindungsgemäße wässrige Nanoharnstoff-Dispersion | | 5,0 | 20,0 |
| Erfindungsgemäßes Polyharnstoffpulver | 5,0 | 5,0 | |

### 5. Hair Styling

### 5.1 "Pump-setting-Spray"

| Rohstoffe | Gew.% (bezogen auf die kosmetische Zusammensetzung) | Gew.% (bezogen auf die kosmetische Zusammensetzung) | Gew.% (bezogen auf die kosmetische Zusammensetzung) |
|---|---|---|---|
| Acrylates copolymer³² | 2 | 2 | 2 |
| Ethanol | 55 | 55 | 55 |
| Erfindungsgemäße wässrige Nanoharnstoff-Dispersion | | 5,0 | 15,0 |
| Erfindungsgemäßes Polyharnstoffpulver | 5,0 | 5,0 | |
| Parfüm | q.s. | q.s. | q.s. |
| Wasser | ad 100 | ad 100 | ad 100 |

| | | | |
|---|---|---|---|
| ³² Luvimer 100 P, BASF AG | | | |

### 5.2 Aerosol-Hair-Sprays

| | 1 | 2 | 3 | 4 | 5 |
|---|---|---|---|---|---|
| Rohstoffe | Gew.% (bezogen auf die kosmetische Zusammensetzung) | | | | |
| Erfindungsgemäße wässrige Nanoharnstoff-Dispersion | | | 10 | 5,0 | |
| Erfindungsgemäßes Polyharnstoffpulver | 5 | 10 | | 5 | 8 |
| Octylacrylamid/acrylat / Butylaminoethyl methacrylat³³ (bezogen auf Feststoff) | | 2,0 | | 5,0 | |
| Acrylates Copolymer³⁴ | | | 2,0 | | 5,0 |
| Acrylates/ T-Butylacrylamide Copolymer³⁵ | 3,0 | | | | |
| Aminomethylpropanol | | | q.s | q.s | |
| Glycerin | | 0,5 | | | |
| Panthenol | | | 0,5 | | 0,5 |
| PEG/PPG-18/18 Dimethicone | | | | 0,5 | |
| PEG-12 dimethicone | | 0,05 | | | |
| Propylene Glycol | | | | 0,5 | |
| Cyclomethicone | | | 1,0 | | 1,0 |
| Benzophenone-3 | | 0,1 | 0,1 | | 0,1 |
| Parfüm | q.s | q.s | q.s | q.s | q.s |
| Ethanol | 14,5 | 20 | 60 | 30 | 20 |
| Wasser | ad 100 | ad 100 | ad 100 | ad 100 | ad 100 |
| Propan/butan 3,5 Bar. (20°C) | | | 20 | 10 | |
| Dimethylether | 40 | 30 | | 30 | 20 |
| Fluorkohlenwasserstoff 152 A | | | | | 20 |

| | | | | | |
|---|---|---|---|---|---|
| ³³ Amphomer, National starch ³⁴ Luvimer P-100, BASF AG ³⁵ Ultrahold Strong, BASF AG | | | | | |

### 5.3 Haarschaum

| | | 1 | 2 |
|---|---|---|---|
| Rohstoffe | Gew.% (bezogen auf die kosmetische Zusammensetzung) | | |
| Erfindungsgemäße wässrige Nanoharnstoff-Dispersion | | | 10,0 |
| Erfindungsgemäßes Polyharnstoffpulver | 4,0 | 2,0 | |
| Octylacrylamid/acrylat/Butylaminoethyl methacrylat³⁶ (bezogen auf Feststoff) | | 5,0 | |
| Acrylates Copolymer³⁷ | 3,0 | | 3,0 |
| Glycerin | | 0,1 | |
| Panthenol | 0,5 | 0,05 | 0,5 |
| Polyquatemium-4 | | 2 | |
| Cetyltrimethylammoniumchlorid | 0,5 | 0,2 | 0,5 |
| PEG-12 dimethicone | 0,5 | | 0,5 |
| Cyclomethicone | 0,5 | | 0,5 |
| Benzophenone-3 | | 0,1 | |
| Parfüm | q.s. | q.s. | q.s. |
| Ethanol | 10 | 15 | 10 |
| Wasser | ad 100 | ad 100 | ad 100 |
| Konservierungsmittel | q.s. | q.s. | q.s. |
| Dimethylether | 7 | 10 | 7 |
| Fluorkohlenwasserstoff 152 A | 3 | | 3 |

| | | | |
|---|---|---|---|
| ³⁶ Amphomer, National starch ³⁷ Luvimer P-100, BASF AG | | | |

### 5.4 Haargel/creme

| | 1 | 2 | 3 | 4 | 5 |
|---|---|---|---|---|---|
| Rohstoffe | Gew.% (bezogen auf die kosmetische Zusammensetzung) | | | | |
| VPNA Copolymer³⁸ | 12,0 | | | 5,0 | 5,0 |
| Acrylates-Copolymer³⁹ | | 4,0 | 4,0 | | |
| Erfindungsgemäße wässrige Nanoharnstoff-Dispersion | | | 10,0 | | 8,0 |
| Erfindungsgemäßes Polyharnstoffpulver | 5 | 2 | | 8 | 8 |
| Carbomer | 0,8 | | | | |
| Acrylsäure/VP Copolymer | | 0,5 | 0,5 | | |
| Ammonium acryloyldimethyltaurat/VP Copolymer | | | | 0,8 | 0,8 |
| Glycerin | 0,5 | | | | |
| Panthenol | | 0,5 | 0,5 | 0,5 | 0,5 |
| Propylenglycol | | | | 0,2 | 0,2 |
| Cyclomethicone | | 0,2 | 0,2 | | |
| Neutralisierungsmittel | q.s. | q.s. | q.s. | q.s. | q.s. |
| Parfüm | q.s. | q.s. | q.s. | q.s. | q.s. |
| Ethanol | 20 | | | | |
| Wasser | ad. 100 | ad. 100 | ad. 100 | ad. 100 | ad. 100 |
| Konservierungsmittel | q.s. | q.s. | q.s. | q.s. | q.s. |

| | | | | | |
|---|---|---|---|---|---|
| ³⁸ Luviskol VA 64 powder, BASF AG ³⁹ Luvimer 100 P, BASF AG | | | | | |

### 5.6 Shampoo

| Rohstoffe | Gew.% (bezogen auf die kosmetische Zusammensetzung) | Gew.% (bezogen auf die kosmetische Zusammensetzung) | Gew.% (bezogen auf die kosmetische Zusammensetzung) |
|---|---|---|---|
| Sodium laureth sulfate | 7,0 | 7,0 | 7,0 |
| Cocamidopropyl betaine | 5,0 | 5,0 | 5,0 |
| Polyquaternium-10 | 0,15 | 0,15 | 0,16 |
| Diammonium lauryl sulfosuccinate | 1,5 | 1,5 | 1,5 |
| Water | Ad.100 | Ad.100 | Ad.100 |
| Konservierungsmittel | q.s. | q.s. | q.s. |
| Farbstoffe | q.s | q.s | q.s |
| Parfüm | q.s | q.s | q.s |
| Neutralisierungsmittel | q.s | q.s | q.s |
| Lösungsvermittel | q.s | q.s | q.s |
| Erfindungsgemäße wässrige Nanoharnstoff-Dispersion | | 5,0 | 30,0 |
| Erfindungsgemäßes Polyharnstoffpulver | 5,0 | 5,0 | |

### 6. Reinigungspräparate

### 6.1 Hautreinungsgel

| Rohstoffe | Gew.% (bezogen auf die kosmetische Zusammensetzung) | Gew.% (bezogen auf die kosmetische Zusammensetzung) | Gew.% (bezogen auf die kosmetische Zusammensetzung) |
|---|---|---|---|
| Sodium laureth sulfate | 13,0 | 13,0 | 13,0 |
| Cocamidopropyl betaine | 1,5 | 1,5 | 1,5 |
| Sodium cocoyl glutamate | 1,5 | 1,5 | 1,5 |
| PEG-40 hydrogenated rizinus oil | 0,5 | 0,5 | 0,5 |
| PEG-100 hydrogenated glyceryl palmitate | 0,5 | 0,5 | 0,5 |
| Polyquaternium-10 | 0,2 | 0,2 | 0,2 |
| Sodium benzoate | 0,5 | 0,5 | 0,5 |
| Sodium salicylate | 0,2 | 0,2 | 0,2 |
| Citric acid | 0,5 | 0,5 | 0,5 |
| Water | Ad.100 | Ad.100 | Ad.100 |
| Konservierungsmittel | q.s. | q.s. | q.s. |
| Farbstoffe | q.s | q.s | q.s |
| Parfüm | q.s | q.s | q.s |
| Lösungsvermittel | q.s | q.s | q.s |
| Erfindungsgemäße wässrige Nanoharnstoff-Dispersion | | 8,0 | 15,0 |
| Erfindungsgemäßes Polyharnstoffpulver | 5,0 | 8,0 | |

### 6.2 Gesichtreiningungsgel

| Rohstoffe | Gew.% (bezogen auf die kosmetische Zusammensetzung) | Gew.% (bezogen auf die kosmetische Zusammensetzung) |
|---|---|---|
| Sodium myreth sulfate | 2,0 | 2,0 |
| Decyl glucoside | 2,0 | 2,0 |
| Acrylates/C10-30 Alkyl Acrylate Crosspolymer⁴⁰ | 0,3 | 0,3 |
| Acrylates Copolymer | 0,3 | 0,3 |
| Water | Ad.100 | Ad.100 |
| Neutralisierungsmittel | q.s. | q.s. |
| Konservierungsmittel | q.s. | q.s. |
| Farbstoffe | q.s | q.s |
| Parfüm | q.s | q.s |
| Lösungsvermittel | q.s | q.s |
| Erfindungsgemäße wässrige Nanoharnstoff-Dispersion | | 20,0 |
| Erfindungsgemäßes Polyharnstoffpulver | 7,0 | |

| | | |
|---|---|---|
| ⁴⁰ Carbopol 1382, Lubrizol | | |

### 6.3 Hautreinigungsemuision

| Rohstoffe | Gew.% (bezogen auf die kosmetische Zusammensetzung) | Gew.% (bezogen auf die kosmetische Zusammensetzung) |
|---|---|---|
| Mineral oil | 10,0 | 10,0 |
| Soja oil | 10,0 | 10,0 |
| Sodium laureth sulfate | 7,5 | 7,5 |
| Sodium benzoate | 0,3 | 0,3 |
| Sodium Salicylate | 0,2 | 0,2 |
| Acrylates/C10-30 Alkyl Acrylate Crosspolymer⁴¹ | 0,8 | 0,8 |
| Acrylates Copolymer | 0,3 | 0,3 |
| Water | Ad.100 | Ad.100 |
| Neutralisierungsmittel | q.s. | q.s. |
| Konservierungsmittel | q.s. | q.s. |
| Farbstoffe | q.s | q.s |
| Parfüm | q.s | q.s |
| Lösungsvermittel | q.s | q.s |
| Erfindungsgemäße wässrige Nanoharnstoff-Dispersion | | 20,0 |
| Erfindungsgemäßes Polyharnstoffpulver | 7,0 | |

| | | |
|---|---|---|
| ⁴¹ Carbopol 1382, Lubrizol | | |

### 6.4 Duschöl

| Rohstoffe | Gew.% (bezogen auf die kosmetische Zusammensetzung) |
|---|---|
| Helianthus Annuus | Ad.100 |
| Rizinus oil | 14,0 |
| MIPA laureth sulfate | 20,0 |
| Laureth-4 | 12,0 |
| Poloxamer 101 | 2,0 |
| Neutralisierungsmittel | q.s. |
| Konservierungsmittel | q.s. |
| Farbstoffe | q.s |
| Parfüm | q.s |
| Lösungsvermittel | q.s |
| Erfindungsgemäßes Polyharnstoffpulver | 10,0 |

### 6.5 Pumpfoamer

| Rohstoffe | Gew.% (bezogen auf die kosmetische Zusammensetzung) | Gew.% (bezogen auf die kosmetische Zusammensetzung) |
|---|---|---|
| Sodium cocoyl palmitate | 2,5 | 2,5 |
| Decyl glucoside | 3,0 | 3,0 |
| Polyquaternium-10 | 0,1 | 0,1 |
| PEG-200 hydrogenated glyceryl palmitate | 0,5 | 0,5 |
| PEG-40 hydrogenated rizinus oil | 0,1 | 0,1 |
| Sodium benzoate | 0,5 | 0,5 |
| Water | Ad.100 | Ad.100 |
| Konservierungsmittel | q.s. | q.s. |
| Farbstoffe | q.s | q.s |
| Parfüm | q.s | q.s |
| Lösungsvermittel | q.s | q.s |
| Erfindungsgemäße wässrige Nanoharnstoff-Dispersion | | 20,0 |
| Erfindungsgemäßes Polyharnstoffpulver | 10,0 | |

### 6.6 Ölbad

| Rohstoffe | Gew.% (bezogen auf die kosmetische Zusammensetzung) |
|---|---|
| Laureth-2 | 10,0 |
| Mineral oil | 20,0 |
| Octyldodecanol | 20,0 |
| Isopropyl palmitate | 25,0 |
| Soja oil | Ad.100 |
| Konservierungsmittel | q.s. |
| Farbstoffe | q.s |
| Parfüm | q.s |
| Erfindungsgemäßes Polyharnstoffpulver | 10,0 |

### 6.7 Schaumbad

| Rohstoffe | Gew.% (bezogen auf die kosmetische Zusammensetzung) | Gew.% (bezogen auf die kosmetische Zusammensetzung) | Gew.% (bezogen auf die kosmetische Zusammensetzung) |
|---|---|---|---|
| Sodium laureth sulfate | 10,0 | 10,0 | 10,0 |
| Cocoamidopropyl betaine | 4,0 | 4,0 | 4,0 |
| Decyl glucoside | 2,5 | 2,5 | 2,5 |
| PEG-7 glyceryl cocoate | 3,0 | 3,0 | 3,0 |
| Glycerin | 5,0 | 5,0 | 5,0 |
| Natrium chloride | q.s. | q.s. | q.s. |
| Water | Ad.100 | Ad.100 | Ad.100 |
| Neutralisierungsmittel | q.s. | q.s. | q.s. |
| Konservierungsmittel | q.s. | q.s. | q.s. |
| Farbstoffe | q.s | q.s | q.s |
| Parfüm | q.s | q.s | q.s |
| Lösungsvermittel | q.s | q.s | q.s |
| Erfindungsgemäße wässrige Nanoharnstoff-Dispersion | | 10,0 | 20,0 |
| Erfindungsgemäßes Polyharnstoffpulver | 20,0 | 10,0 | |

## Patentansprüche

1. Nanoharnstoffpulver, welches ausgehend von einer wässrigen Polyharnststoffdispersion durch Trocknung erhalten wird.

2. Nanoharnstoffpulver nach Anspruch 1, **dadurch gekennzeichnet, dass** das Pulver ausgehend von wässrigen Dispersionen von quervernetzten Nanoharnstoffpartikein erhalten wird.

3. Nanoharnstoffpulver nach Anspruch 1 oder 2, **dadurch gekennzeichnet, dass** die Partikel des Pulvers eine Größe von 0,5 bis 1000 µm aufweisen.

4. Verfahren zur Herstellung von Nanoharnstoffpulvern, **dadurch gekennzeichnet, dass** eine wässrige Dispersionen von Nanoharnstoffen getrocknet wird.

5. Verfahren nach Anspruch 4, **dadurch gekennzeichnet, dass** die Trocknung unter Entfernung von Wasser aus der Dispersion bei Atmosphärendruck, Unterdruck oder Überdruck erfolgt.

6. Verfahren nach Anspruch 4 oder 5, **dadurch gekennzeichnet, dass** eine wässrige Dispersion von quervernetzten Nanoharnstoffpartikeln verwendet wird.

7. Verfahren nach Anspruch 6, **dadurch gekennzeichnet, dass** die Trocknung der wässrigen Dispersion mittels eines Srühtrocknungsverfahrens erfolgt.

8. Nanoharnstoffpulver, erhältlich nach einem Verfahren gemäß einem der Ansprüche 4 bis 7.

9. Verwendung von Nanoharnstoffpulvern gemäß einem der Ansprüche 1 bis 3 oder 8 in Kosmetika, Beschichtungsmittel, Dichtstoffe, Klebstoffe, Füllstoff, Additiv, Hilfs- und/oder Zusatzstoff.

10. Kosmetika, Gegenstände, Beschichtungsmittel, Dichtstoff, Klebstoff, Füllstoff, Additiv, Hilfs- und/oder Zusatzstoff, enthaltend Polyharnstoffpulver gemäß einem der Ansprüche 1 bis 3 oder 8.

11. Verwendung von wässrigen Dispersionen, enthaltend quervernetzte Polyharnstoffe, in Kosmetika.

12. Kosmetika, enthaltend wässrige Dispersionen von quervernetzten Polyharnstoffen.

13. Verfahren zur Herstellung von Kosmetika unter Verwendung von Nanoharnstoffpulvern gemäß einem der Ansprüche 1 bis 3 oder 8 und/oder wässrigen Dispersionen von quervernetzten Polyharnstoffen.
